# EUROPEAN PATENT APPLICATION

(11) **EP 0 878 194 A1**
(43) Date of publication of application: **18.11.1998**
(21) Application number: 97901785.2
(22) Date of filing: 30.01.1997
(51) Int. Cl.: A61K 31/445

(54) **REMEDIES OR PREVENTIVES FOR AIDS**

(30) Priority: 31.01.1996 JP 14825/96
(71) Applicant: SANKYO COMPANY LIMITED, Chuo-ku, Tokyo 103 (JP); Ube Industries, Ltd., Ube-shi, Yamaguchi-ken 755-0052 (JP)
(72) Inventor: KOMAI, Tomoaki, Sankyo Company, Limited, Tokyo 140 (JP); OHMINE, Toshinori, Sankyo Company, Limited, Tokyo 140 (JP); NISHIGAKI, Takashi, Sankyo Company, Limited, Tokyo 140 (JP); KIMURA, Tomio, Niiza-shi, Saitama-ken 352 (JP); KATSUBE, Tetsushi, Ube Industries, Ltd., Ube-shi, Yamaguchi-ken 755 (JP)
(74) Representative: Gibson, Christian John Robert
(86) International application number: JP9700218
(87) International publication number: WO9727856

(57) **Abstract**

The present invention is to provide the combined use of one kind or two or more kinds of a quinolone carboxylic acid having anti-HIV activity and one kind or two or more kinds of a reverse transcriptase inhibitor or HIV protease inhibitor, and an AIDS therapeutic agent or preventive agent containing as its active ingredients one kind or two or more kinds of a quinolone carboxylic acid having anti-HIV activity and one kind or two or more kinds of a reverse transcriptase inhibitor or HIV protease inhibitor.

## Description

### Technical Field

The present invention relates to the combined use of one kind or two or more kinds of a quinolone carboxylic acid having anti-HIV activity and one kind or two or more kinds of a reverse transcriptase inhibitor; the combined use of one kind or two or more kinds of a quinolone carboxylic acid having anti-HIV activity and one kind or two or more kinds of an HIV protease inhibitor; and the combined use of one kind or two or more kinds of a quinolone carboxylic acid having anti-HIV activity, one kind or two or more kinds of a reverse transcriptase inhibitor and one kind or two or more kinds of an HIV protease inhibitor for the treatment and prevention of AIDS; and relates to agents for the treatment and prevention of AIDS containing as its active ingredients one kind or two or more kinds of a quinolone carboxylic acid having anti-HIV activity and one kind or two or more kinds of a reverse transcriptase inhibitor; agents for the treatment and prevention of AIDS containing as its active ingredients one kind or two or more kinds of a quinolone carboxylic acid having anti-HIV activity and one kind or two or more kinds of an HIV protease inhibitor; and agents for the treatment and prevention of AIDS containing as its active ingredients one kind or two or more kinds of a quinolone carboxylic acid having anti-HIV activity, one kind or two or more kinds of a reverse transcriptase inhibitor and one kind or two or more kinds of an HIV protease inhibitor.

### Background Art

Several drugs have been marketed and numerous compounds are being developed or researched for the purpose of treatment or prevention of occurrence of AIDS. For example, compounds that inhibit reverse transcriptase or HIV protease have been known to be effective in the treatment of AIDS and the prevention of its occurrence.

However, since there are limitations on the treatment and prevention with a single drug, using a plurality of agents for the treatment or prevention of AIDS is being attempted.

Meanwhile, the inventors of the present invention have already found that quinolone carboxylic acids have anti-HIV activity and are effective in the treatment of AIDS or in the prevention of its occurrence (EP-A-572,259 and WO 96/02512).

The present inventors made various researches in consideration of the importance of the treatment or prevention of occurrence of AIDS, and consequently it was found that AIDS could be treated or prevented more effectively by using in combination one kind or two or more kinds of a quinolone carboxylic acid having anti-HIV activity and one kind or two or more kinds of a reverse transcriptase inhibitor; by using in combination one kind or two or more kinds of a quinolone carboxylic acid having anti-HIV activity and one kind or two or more kinds of an HIV protease inhibitor; and by using in combination one kind or two or more kinds of a quinolone carboxylic acid having anti-HIV activity, one kind or two or more kinds of a reverse transcriptase inhibitor and one kind or two or more kinds of an HIV protease inhibitor.

The present invention provides the combined use of one kind or two or more kinds of a quinolone carboxylic acid having anti-HIV activity and one kind or two or more kinds of a reverse transcriptase inhibitor; the combined use of one kind or two or more kinds of a quinolone carboxylic acid having anti-HIV activity and one kind or two or more kinds of an HIV protease inhibitor; and the combined use of one kind or two or more kinds of a quinolone carboxylic acid having anti-HIV activity, one kind or two or more kinds of a reverse transcriptase inhibitor and one kind or two or more kinds of an HIV protease inhibitor for the treatment or prevention of AIDS; and the present invention provides agents for the treatment and prevention of AIDS containing as its active ingredients one kind or two or more kinds of a quinolone carboxylic acid having anti-HIV activity and one kind or two or more kinds of a reverse transcriptase inhibitor; agents for the treatment and prevention of AIDS containing as its active ingredients one kind or two or more kinds of a quinolone carboxylic acid having anti-HIV activity and one kind or two or more kinds of an HIV protease inhibitor; and agents for the treatment and prevention of AIDS containing as its active ingredients one kind or two or more kinds of a quinolone carboxylic acid having anti-HIV activity, one kind or two or more kinds of a reverse transcriptase inhibitor and one kind or two or more kinds of an HIV protease inhibitor.

### Disclosure of the Invention

The active ingredients of the combined use for the treatment and prevention of AIDS as well as the active ingredients of the agent for the treatment and prevention of AIDS contain:
1) One kind or two or more kinds of a quinolone carboxylic acid having anti-HIV activity and one kind or two or more kinds of a reverse transcriptase inhibitor;
2) One kind or two or more kinds of a quinolone carboxylic acid having anti-HIV activity and one kind or two or more kinds of an HIV protease inhibitor; and
3) One kind or two or more kinds of a quinolone carboxylic acid having anti-HIV activity, one kind or two or more kinds of a reverse transcriptase inhibitor and one kind or two or more kinds of an HIV protease inhibitor.

Typical examples of the quinolone carboxylic acid having anti-HIV activity that is an active ingredient of the present invention include the quinolone carboxylic acid and its pharmacologically acceptable salts of a formula (Ia), (Ib) or (Ic) shown below. 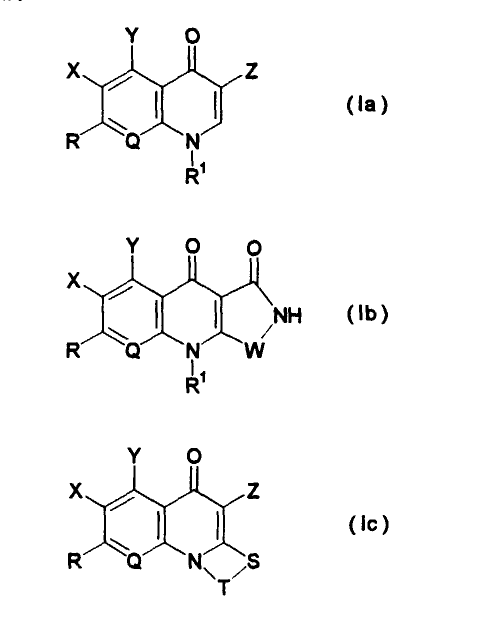 In the above formula (Ia), (Ib) or (Ic),
X represents a hydrogen atom or a halogen atom,
Y represents a hydrogen atom, a halogen atom, an alkyl group having from 1 to 4 carbon atoms, an amino group, a mono- or dialkyl-amino group which is substituted with one or two alkyl groups having from 1 to 4 carbon atoms, or a mono- or diaralkyl-amino group which is substituted with one or two aralkyl groups having from 7 to 14 carbon atoms,
Z represents an optionally protected carboxyl group or a 5-tetrazolyl group,
Q represents a nitrogen atom or a group of formula (d): 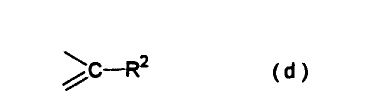 [wherein R² represents a hydrogen atom, a halogen atom, an alkyl group having from 1 to 4 carbon atoms which may be substituted with halogen, or an alkoxy group having from 1 to 4 carbon atoms which may be substituted with halogen],
W represents an omen atom or a sulfur atom,
T represents an alkylene group having from 1 to 4 carbon atoms which may be substituted with alkyl having from 1 to 4 carbon atoms or an alkenylene group having from 2 to 4 carbon atoms which may be substituted with alkyl having from 1 to 4 carbon atoms,
R¹ represents a hydrogen atom; an optionally substituted alkyl group having from 1 to 4 carbon atoms [the substituents are hydroxyl, carboxyl, halogen, alkoxy having from 1 to 4 carbon atoms, cycloalkyl having from 3 to 6 carbon atoms, alkanoyloxy having from 2 to 5 carbon atoms, a group of formula (e): 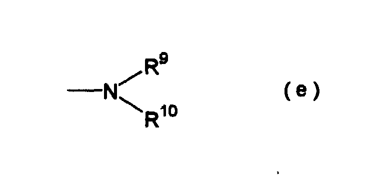 (wherein R⁹ and R¹⁰ each represents a hydrogen atom or an alkyl group having from 1 to 4 carbon atoms, or R⁹ and R¹⁰ may together form a 3- to 7-membered saturated heteromonocyclic group with a nitrogen atom to which they bond, optionally containing a hetero atom selected from N, O and S), an aryl group having from 6 to 10 carbon atoms which may be substituted with R⁰ as defined later, a 5- or 6-membered aromatic heteromonocyclic group containing one or two hetero atoms selected from N, O and S which may be substituted with R⁰ as defined later, or an aromatic heterocyclic fused-ring group in which a benzene ring is fused with the aromatic heteromonocyclic group which may be substituted with R⁰ as defined later], an alkenyl group having from 2 to 5 carbon atoms which may be substituted with halogen; an alkynyl group having from 2 to 4 carbon atoms; an amino group; a mono- or dialkyl-amino group substituted with one or two alkyl groups having from 1 to 4 carbon atoms; a cycloalkyl group having from 3 to 6 carbon atoms which may be substituted with halogen; an alkoxy group having from 1 to 4 carbon atoms; or an aryl group having from 6 to 10 carbon atoms which may be substituted with R⁰ as defined later, a 5- or 6-membered aromatic heteromonocyclic group containing one or two hetero atoms selected from N, O and S which may be substituted with R⁰ as defined later, or an aromatic heterocyclic fused-ring group in which a benzene ring is fused with the aromatic heteromonocyclic group which may be substituted with R⁰ as defined later, or
R¹ and R² in the formula (d) of Q together form a group of formula (f): 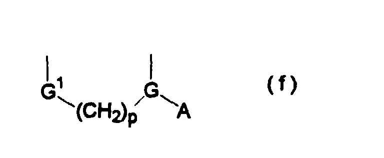 [wherein A represents a hydrogen atom or an alkyl group having from 1 to 4 carbon atoms which may be substituted with halogen, hydroxyl or alkoxy having from 1 to 4 carbon atoms, G represents a nitrogen atom or a group of formula (g): 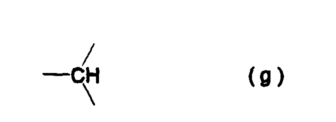 G¹ represents a methylene group, a carbonyl group, an oxygen atom, a sulfur atom or a group of formula -N(R¹¹)- (wherein R¹¹ represents a hydrogen atom or an alkyl group having from 1 to 4 carbon atoms) and p represents 0 or 1],
R represents a group of formula (h) or (i): 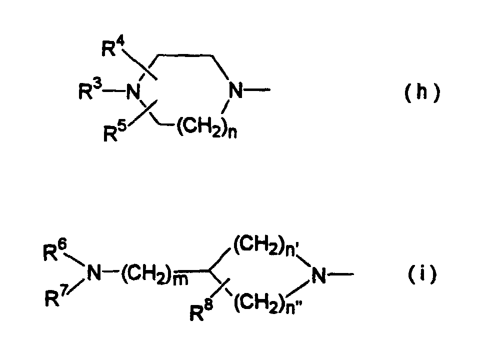 [wherein R³ and R⁶ each represents an aryl group having from 6 to 10 carbon atoms which may be substituted with R⁰ (R⁰ represents a group selected from halogen, nitro, hydroxyl, alkyl having from 1 to 4 carbon atoms, alkyl having from 1 to 4 carbon atoms substituted with fluorine, alkoxy having from 1 to 4 carbon atoms, alkylthio having from 1 to 4 carbon atoms, amino and mono- or dialkyl-amino substituted with one or two alkyl groups having from 1 to 4 carbon atoms), a 5- or 6-membered aromatic heteromonocyclic group containing one or two hetero atoms selected from N, O and S which may be substituted with R⁰ as defined above, or an aromatic heterocyclic fused-ring group in which a benzene ring is fused with the aromatic heteromonocyclic group which may be substituted with R⁰ as defined above; R⁴, R⁵ and R⁷ each represents a hydrogen atom or an alkyl group having from 1 to 4 carbon atoms; R⁸ represents a hydrogen atom, a hydroxyl group, an alkyl group having from 1 to 4 carbon atoms or an alkoxy group having from 1 to 4 carbon atoms; n represents 1 or 2; m represents 0 or 1; n' represents 1 or 2; and n'' represents 1, 2, 3 or 4].

Namely, the compounds of the formulae (Ia), (Ib) and (Ic) include the following compounds of formulae (Ia-1), (Ib-1), (Ic-1), (Ia-2), (Ib-2) and (Ic-2): 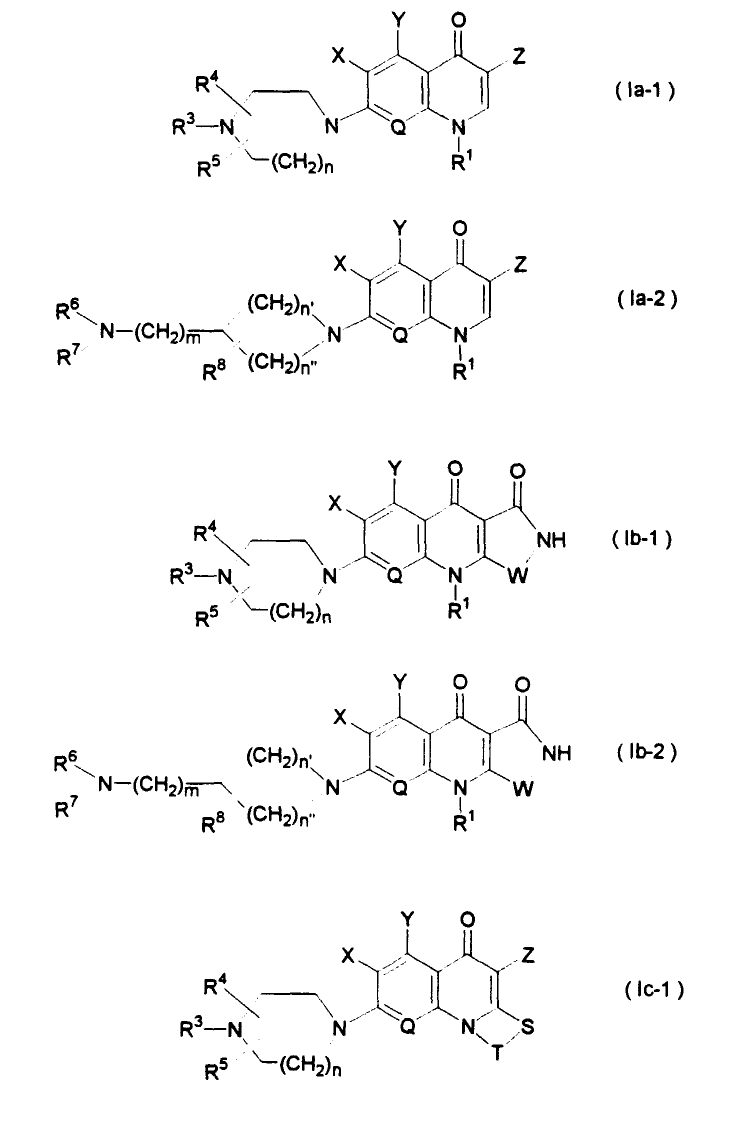 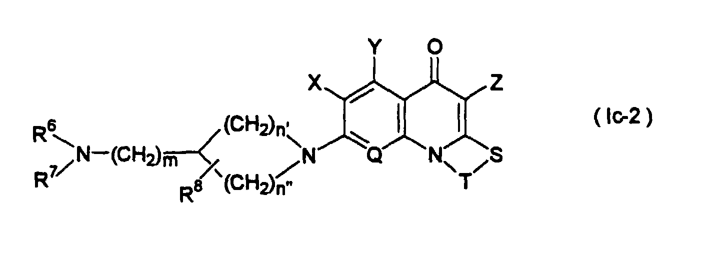 (wherein, X, Y, Z, W, Q, T, R¹, R³ to R⁸ and m, n, n' and n'' have the same meanings as defined above).

Further, of the compounds represented by the formulae (Ia), (Ib) and (Ic), preferable compounds include those of formulae (Ia-3), (Ib-3), (Ic-3), (Ia-4), (Ib-4), (Ic-4), (Ia-5), (Ib-5), (Ic-5), (Ia-6) and (Ia-7): 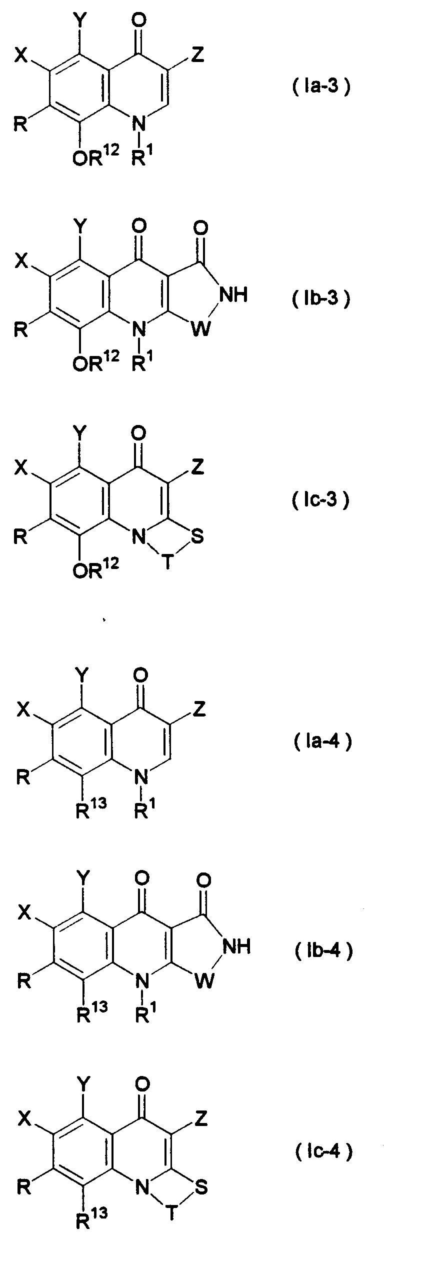 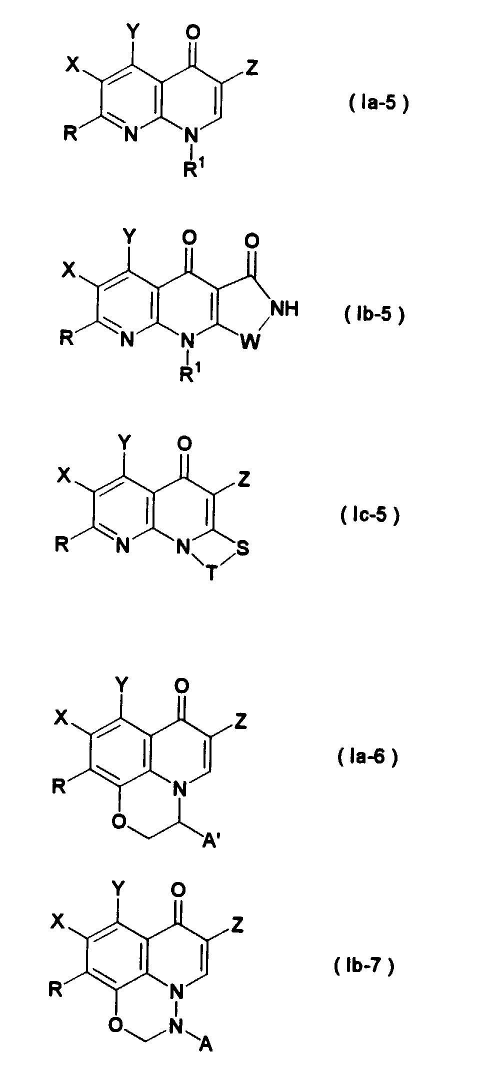 (wherein A, X, Y, Z, W, R, T and R¹ have the same meanings as defined above, -OR¹² represents an alkoxy group having from 1 to 4 carbon atoms which may be substituted with halogen (particularly fluorine), particularly a difluoromethoxy group, R¹³ represents a hydrogen atom, a halogen atom or an alkyl group having from 1 to 4 carbon atoms which may be substituted with halogen (particularly fluorine), particularly a trifluoromethyl group, further, and A' represents a hydrogen atom or an alkyl group having from 1 to 4 carbon atoms which may be substituted with halogen (particularly fluorine)).

In the present invention, the compounds of the formulae (Ia-3), (Ib-3), (Ic-3), (Ia-4) and (Ia-6) are preferable, and the compounds of the formulae (Ia-3), (Ia-4) and (Ia-6) in which A' is an alkyl group having from 1 to 4 carbon atoms substituted with fluorine are more preferable.

Examples of the halogen atom of X in the formulae (Ia), (Ib) and (Ic), include fluorine, chlorine, bromine and iodine atoms, preferably fluorine and chlorine atoms, more preferably a fluorine atom.

X in the formulae (Ia), (Ib) and (Ic) preferably includes hydrogen, fluorine and chlorine atoms, more preferably fluorine and chlorine atoms, most preferably a fluorine atom.

Y in the formulae (Ia), (Ib) and (Ic) includes hydrogen atoms; halogen atoms such as fluorine, chlorine, bromine and iodine; alkyl groups having from 1 to 4 carbon atoms such as methyl, ethyl, propyl, isopropyl, butyl and isobutyl; amino groups; monoalkyl-amino groups substituted with alkyl having from 1 to 4 carbon atoms such as methylamino, ethylamino, propylamino, isopropylamino and butylamino; dialkyl-amino groups substituted with alkyl having from 1 to 4 carbon atoms such as dimethylamino, diethylamino, dipropylamino, diisopropylamino and dibutylamino; monoaralkyl-amino groups substituted with aralkyl having from 7 to 14 carbon atoms such as benzylamino and phenylethylamino; and diaralkyl-amino groups substituted with aralkyl having from 7 to 14 carbon atoms such as dibenzylamino and di(phenylethyl)amino, preferably hydrogen and fluorine atoms, and amino, methyl and ethyl groups, more preferably hydrogen atoms.

The protecting group of the carboxyl group of 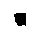 the carboxyl group which may be protected*"* of Z in the formulae (Ia) and (Ic) includes a group which is easily removed in vivo to be converted to a carboxyl group such as an alkyl group having from 1 to 4 carbon atoms such as methyl, ethyl, propyl, isopropyl, butyl and isobutyl; an aralkyl group having from 7 to 14 carbon atoms such as benzyl, 1-phenylethyl and 1-naphthylmethyl; an alkanoyloxyalkyl group having from 1 to 4 carbon atoms such as acetoxymethyl and pivaloyloxymethyl; an alkoxycarbonyloxyalkyl group having from 1 to 4 carbon atoms such as 1-(ethoxycarbonyloxy)ethyl and 1-(isopropoxycarbonyloxy)ethyl; an N,N-dialkyl-aminocarbonylalkyl group such as N,N-dimethylaminocarbonylmethyl; an N,N-dialkyl-aminoalkyl group such as 2-(N,N-dimethylamino)ethyl group; an alkyl group substituted with a 5- or 6-membered saturated heteromonocyclic group containing one or two hetero atoms selected from N, O and S such as 2-morpholinoethyl, 2-piperidinoethyl and 2-(4-methylpiperidino)ethyl; and (5-methyl-2-oxo-1,3-dioxolen-4-yl)methyl and (5-phenyl-2-oxo-1,3-dioxolen-4-yl)methyl.

Z in the formulae (Ia) and (Ic) may include preferably an optionally protected carboxyl group, more preferably a carboxyl group.

In the case where Q in the formulae (Ia), (Ib) and (Ic) is a group of formula (d), the halogen atom includes fluorine, chlorine, bromine and iodine atoms, preferably fluorine and chlorine atoms.

In the case where Q in the formulae (Ia), (Ib) and (Ic) is a group of formula (d), the alkyl group having from 1 to 4 carbon atoms which may be substituted with halogen of R² includes alkyl groups such as methyl, ethyl, propyl, isopropyl and butyl; fluoroalkyl groups such as fluoromethyl, difluoromethyl, trifluoromethyl, 2-fluoroethyl, 3-fluoropropyl and 4-fluorobutyl; and chloroalkyl groups such as chloromethyl, dichloromethyl, trichloromethyl, 2-chloroethyl, 3-chloropropyl and 4-chlorobutyl, preferably alkyl and fluoroalkyl groups, more preferably methyl, fluoromethyl, difluoromethyl and trifluoromethyl groups, most preferably a trifluoromethyl group.

In the case where Q in the formulae (Ia), (Ib) and (Ic) is a group of the formula (d), the alkoxy group having from 1 to 4 carbon atoms which may be substituted with halogen includes alkoxy groups such as methoxy, ethoxy, propoxy, isopropoxy and butoxy; fluoroalkoxy groups such as fluoromethoxy, difluoromethoxy, trifluoromethoxy, 2-fluoroethoxy, 2-fluoropropoxy, 3-fluoropropoxy and 4-fluorobutoxy; and chloroalkoxy groups such as 2-chloroethoxy, 2-chloropropoxy, 3-chloropropoxy and 4-chlorobutoxy, preferably alkoxy and fluoroalkoxy groups, (paticularly methoxy, ethoxy, fluoromethoxy, difluoromethoxy, trifluoromethoxy and 2-fluoroethoxy groups), more preferably methoxy, fluoromethoxy, difluoromethoxy and trifluoromethoxy groups, still more preferably methoxy and difluoromethoxy groups, most preferably a difluoromethoxy group.

In the case where Q in the formulae (Ia), (Ib) and (Ic) is a group of the formula (d), R² preferably includes hydrogen atoms; halogen atoms; alkyl groups having from 1 to 4 carbon atoms which may be substituted with a fluorine atom; and alkoxy groups having from 1 to 4 carbon atoms which may be substituted with fluorine, more preferably fluoromethyl, difluoromethyl, trifluoromethyl, methoxy, fluoromethoxy, difluoromethoxy and trifluoromethoxy groups, still more preferably methoxy, difluoromethoxy and trifluoromethyl groups, most preferably difluoromethoxy and trifluoromethyl groups.

Q in the formulae (Ia), (Ib) and (Ic) preferably includes the formula (d) in which R² is a methoxy, difluoromethoxy or trifluoromethyl group.

W in the formula (Ib) preferably includes a sulfur atom.

T in the formula (Ic) includes an alkylene group having from 1 to 4 carbon atoms which may be substituted with an alkyl group having from 1 to 4 carbon atoms such as methylene, ethylidene [-CH(CH₃)-], ethylene, trimethylene, propylene and tetramethylene; and an alkenylene group from having 2 to 4 carbon atoms which may be substituted with an alkyl group having from 1 to 4 carbon atoms such as -CH=CH- and -C(CH₃)=CH-, preferably an ethylidene, -CH=CH- and -C(CH₃)=CH- groups, more preferably an ethylidene group.

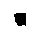 The alkyl group having from 1 to 4 carbon atoms*"* of R ¹ in the formulae (Ia) and (Ib), in which the alkyl group may be substituted, includes alkyl groups such as methyl, ethyl, propyl, isopropyl, butyl, isobutyl, s-butyl and t-butyl, preferably methyl, ethyl, propyl and isopropyl groups, more preferably methyl and ethyl groups.

The halogen atom, which is a substituent of the alkyl group having from 1 to 4 carbon atoms of R¹ in the formulae (Ia) and (Ib), includes fluorine, chlorine, bromine and iodine atoms, preferably fluorine and chlorine atoms, more preferably a fluorine atom.

The alkoxy group having from 1 to 4 carbon atoms, which is a substituent of the alkyl group having from 1 to 4 carbon atoms of R¹ in the formulae (Ia) and (Ib), includes methoxy, ethoxy, propoxy, isopropoxy and butoxy groups, preferably methoxy and ethoxy groups, more preferably a methoxy group.

The cycloalkyl group having from 3 to 6 carbon atoms, which is a substituent of the alkyl group having from 1 to 4 carbon atoms of R¹ in the formulae (Ia) and (Ib), includes cyclopropyl, cyclobutyl, cyclopentyl and cyclohexyl groups, preferably a cyclopropyl group.

The alkanoyloxy group having from 2 to 5 carbon atoms, which is a substituent of the alkyl group having from 1 to 4 carbon atoms of R¹ in the formulae (Ia) and (Ib), includes acetoxy, propionyloxy and butyryloxy groups, preferably an acetoxy group.

The alkyl group having from 1 to 4 carbon atoms of R⁹ and R¹⁰ in the formula (e), which is a substituent of the alkyl group having 1 to 4 carbon atoms of R¹ in the formulae (Ia) and (Ib), includes the same groups as mentioned above for the alkyl group having from 1 to 4 carbon atoms of the R¹, preferably methyl and ethyl groups.

The 3- to 7-membered saturated heteromonocyclic group formed of R⁹ and R¹⁰ together with a nitrogen atom to which they bond, optionally further containing a hetero atom selected from N, O and S, in the definition of formula (e), which is a substituent of the alkyl group having from 1 to 4 carbon atoms of R¹ in the formulae (Ia) and (Ib), includes aziridino, azetidino, pyrrolidino, piperidino, morpholino, thiomorpholino and piperazino, preferably piperidino and morpholino groups.

The group of formula (e), which is a substituent of the alkyl group having from 1 to 4 carbon atoms of R¹ in the formulae (Ia) and (Ib), preferably includes amino, methylamino, dimethylamino, piperidino and morpholino groups, more preferably a dimethylamino group.

The 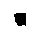 aryl group having from 6 to 10 carbon atoms*"* of the aryl group having from 6 to 10 carbon atoms which may be substituted with R⁰ as defined later, which is a substituent of the alkyl group having from 1 to 4 carbon atoms of R¹ in the formulae (Ia) and (Ib), includes phenyl, 1-naphthyl and 2-naphthyl groups, preferably a phenyl group.

The "5- or 6-membered aromatic heteromonocyclic group containing one or two hetero atoms selected from N, O and S or an aromatic heterocyclic fused-ring group in which a benzene ring is fused with the aromatic heteromonocyclic group*"* which may be substituted with R⁰ as defined later (hereinafter the aromatic heteromonocyclic group and the aromatic heterocyclic fused-ring group are also referred to as an aromatic heterocyclic group), which is a substituent of the alkyl group having from 1 to 4 carbon atoms of R¹ in the formulae (Ia) and (Ib), includes 2-thienyl, 2-furyl, 2-oxazolyl, 2-thiazolyl, 2-imidazolyl, 2-pyridyl, 3-pyridyl, 4-pyridyl, 2-pyrimidinyl, 4-pyrimidinyl, 5-pyrimidinyl, 2-pyrazinyl, 3-pyridazinyl, 2-benzoxazolyl, 2-benzothiazolyl, 2-benzimidazolyl, 3-benzisoxazolyl and 3-benzisothiazolyl groups, preferably 2-thienyl, 2-furyl, 2-pyridyl and 2-pyrimidinyl groups, more preferably a 2-pyridyl group.

The substituent R⁰ in the definition of the aryl group having from 6 to 10 carbon atoms and the aromatic heterocyclic group which may be substituted with R⁰ as defined later, in which the aryl and the heterocyclic group is a substituent of the alkyl group having from 1 to 4 carbon atoms of R¹ in the formulae (Ia) and (Ib), includes a halogen atom such as fluorine, chlorine, bromine and iodine atoms; a nitro group; a hydroxyl group; alkyl groups having from 1 to 4 carbon atoms such as methyl, ethyl, propyl, isopropyl, butyl, s-butyl, isobutyl and t-butyl groups; alkyl groups having from 1 to 4 carbon atoms which are substituted with a fluorine atom such as fluoromethyl, difluoromethyl, trifluoromethyl, 2-fluoroethyl, 2-fluoropropyl, 3-fluoropropyl, 2-fluorobutyl, 3-fluorobutyl and 4-fluorobutyl groups; alkoxy groups having from 1 to 4 carbon atoms such as methoxy, ethoxy, propoxy, isopropoxy and butoxy groups; alkylthio groups having from 1 to 4 carbon atoms such as methylthio, ethylthio, propylthio, isopropylthio and butylthio groups; an amino group; monoalkyl-amino groups which are substituted with an alkyl group having from 1 to 4 carbon atoms such as methylamino, ethylamino, propylamino, isopropylamino, butylamino, isobutylamino, s-butylamino and t-butylamino groups; and dialkyl-amino groups which are substituted with an alkyl group having from 1 to 4 carbon atoms such as dimethylamino, diethylamino, dipropylamino, diisopropylamino, dibutylamino, diisobutylamino and ethyl(methyl)amino; preferably fluorine and chlorine atoms; and methyl and methoxy groups.

Examples of the whole alkyl group, having 1 to 4 carbon atoms which may be substituted, of R¹ in the formulae (Ia) and (Ib) include methyl, ethyl, propyl, isopropyl, butyl, s-butyl, t-butyl; 2-hydroxyethyl, 2-hydroxypropyl, 3-hydroxypropyl; carboxymethyl, 1-carboxymethyl, 2-carboxymethyl; fluoromethyl, chloromethyl, 2-fluoroethyl, 2-chloroethyl, 2-bromoethyl, 2-iodoethyl, 3-fluoropropyl, 3-chloropropyl, 3-bromopropyl, 4-fluorobutyl, difluoromethyl, trifluoromethyl, 2,2,2-trifluoroethyl; cyclopropylmethyl; 2-acetoxymethyl, 2-acetoxypropyl, 3-acetoxypropyl; 2-aminoethyl, 2-methylaminoethyl, 2-dimethylaminoethyl, 2-morpholinoethyl, 2-piperidinoethyl; 2-methoxymethyl; phenylmethyl, 1-phenylethyl, 2-phenylethyl, naphthylmethyl, 2-fluorophenylmethyl, 3-fluorophenylmethyl, 4-fluorophenylmethyl, 2,4-difluorophenylmethyl, 3,4-difluorophenylmethyl, 2,6-difluorophenylmethyl, 2-methylphenylmethyl, 3-methylphenylmethyl, 4-methylphenylmethyl, 2-chlorophenylmethyl, 3-chlorophenylmethyl, 4-chlorophenylmethyl, 2-methoxyphenylmethyl, 3-methoxyphenylmethyl, 4-methoxyphenylmethyl, 2-thienylmethyl, 2-furylmethyl, 2-pyridylmethyl and 2-pyrimidinylmethyl groups,
preferably methyl, ethyl, propyl, isopropyl, 2-hydroxyethyl, carboxymethyl, 2-fluoroethyl, 2-chloroethyl, 2,2,2-trifluoroethyl, 2-acetoxyethyl, phenylmethyl, 2-phenylethyl, 2-pyridylmethyl, 2-dimethylaminoethyl and 2-morpholinoethyl groups,
more preferably methyl, ethyl, 2-hydroxyethyl and 2-fluoroethyl groups,
most preferably methyl, ethyl and 2-hydroxyethyl groups.

Examples of the alkenyl group having from 2 to 5 carbon atoms which may be substituted with halogen of R¹ in the formulae (Ia) and (Ib) include vinyl, 2-propenyl, 1-butenyl, 2-butenyl, 3-butenyl, 3,3-dimethyl-2-propenyl, 2-fluorovinyl, 2-(2-fluoro)propenyl, 3,3-difluoro-2-propenyl, 3,3-dichloro-2-propenyl groups, preferably vinyl, 2-propenyl, 3,3-dimethyl-2-propenyl and 3,3-dichloro-2-propenyl groups, more preferably vinyl and 2-propenyl groups.

Examples of the alkynyl group having from 2 to 4 carbon atoms of R¹ in the formulae (Ia) and (Ib) include ethynyl, 1-propynyl, 2-propynyl and 2-butynyl groups, preferably ethynyl and 2-propynyl groups, more preferably a 2-propynyl group.

The mono- or dialkylamino group which is substituted with one or two alkyl groups having from 1 to 4 carbon atoms, in the definition of R¹ in the formulae (Ia) and (Ib), include mono alkyl-amino groups such as methylamino, ethylamino, propylamino, isopropylamino and butylamino groups; and dialkyl-amino groups such as dimethylamino, diethylamino, dipropylamino, diisopropylamino and dibutylamino groups, preferably methylamino, ethylamino and dimethylamino groups, more preferably a methylamino group.

Examples of the cycloalkyl group having from 3 to 6 carbon atoms which may be substituted with halogen in the definition of R¹ in the formulae (Ia) and (Ib), include cyclopropyl, cyclobutyl, cyclopentyl, cyclohexyl, 2-fluorocyclopropyl, 2-chlorocyclopropyl, 2-bromocyclopropyl, 2,2-difluorocyclopropyl, 2-chloro-2-fluorocyclopropyl, 2-fluorocyclobutyl, 2-fluorocyclopentyl and 2-fluorocyclohexyl groups, preferably cyclopropyl, cyclobutyl, cyclopentyl and 2-fluorocyclopropyl groups, more preferably a cyclopropyl group.

The alkoxy group having 1 to 4 carbon atoms in the definition of R¹ in the formulae (Ia) and (Ib) includes methoxy, ethoxy, propoxy, isopropoxy and butoxy groups, preferably methoxy, ethoxy and propoxy groups, more preferably methoxy and ethoxy groups.

The 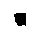 aryl group having from 6 to 10 carbon atoms*"* which may be substituted with R⁰ as defined later and the 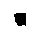 5- or 6-membered aromatic heteromonocyclic group containing one or two hetero atoms selected from N, O and S or the aromatic heterocyclic fused-ring group in which a benzene ring is fused with the aromatic heteromonocyclic group*"* which may be substituted with R⁰ as defined later, in the definition of R¹ in the formulae (Ia) and (Ib), include the same groups as the above-mentioned substituent of the alkyl group having from 1 to 4 carbon atoms of R¹, preferably phenyl, naphthyl, 2-thiazolyl, 2-oxazolyl, 2-pyridyl, 3-pyridyl, 4-pyridyl, 2-pyrimidinyl, 4-pyrimidinyl, 5-pyrimidinyl, 2-benzoxazolyl and 2-benzothiazolyl groups, more preferably phenyl and 2-pyridyl groups.

The substituent R⁰ of the aryl group having from 6 to 10 carbon atoms which may be substituted with R⁰, the 5- or 6-membered aromatic heteromonocyclic group containing one or two hetero atoms selected from N, O and S which may be substituted with R⁰ or the aromatic heterocyclic fused-ring group in which a benzene ring is fused with the aromatic heteromonocyclic group which may be substituted with R⁰, in the definition of R¹ in the formulae (Ia) and (Ib), include the same groups as the above-mentioned substituent R⁰ of the aromatic heterocyclic group which may be substituted with R⁰ as defined later, in which the aromatic heterocyclic group is a substituent of the alkyl group having from 1 to 4 carbon atoms of R¹ in the formulae (Ia) and (Ib), preferably fluorine and chlorine atoms; and a methyl group.

R¹ in the formulae (Ia) and (Ib) includes preferably a hydrogen atom; methyl, ethyl, propyl, isopropyl, butyl, s-butyl, t-butyl; 2-hydroxyethyl, 2-hydroxypropyl, 3-hydroxypropyl; carboxymethyl, 1-carboxyethyl, 2-carboxyethyl; fluoromethyl, 2-fluoroethyl, 2-chloroethyl, 3-fluoropropyl, 3-chloropropyl, difluoromethyl, trifluoromethyl, 2,2,2-trifluoroethyl; cyclopropylmethyl; 2-acetoxyethyl, 2-acetoxypropyl, 3-acetoxypropyl; 2-aminoethyl, 2-methylaminoethyl, 2-dimethylaminoethyl, 2-morpholinoethyl, 2-piperidinoethyl; 2-methoxyethyl; phenylmethyl, 1-phenylethyl, 2-phenylethyl, naphthylmethyl, 2-fluorophenylmethyl, 3-fluorophenylmethyl, 4-fluorophenylmethyl, 2,4-difluorophenylmethyl, 3,4-difluorophenylmethyl, 2,6-difluorophenylmethyl, 2-methylphenylmethyl, 3-methylphenylmethyl, 4-methylphenylmethyl, 2-chlorophenylmethyl, 3-chlorophenylmethyl, 4-chlorophenylmethyl, 2-methoxyphenylmethyl, 3-methoxyphenylmethyl, 4-methoxyphenylmethyl; 2-thienylmethyl, 2-furylmethyl, 2-pyridylmethyl, 2-pyrimidinylmethyl; amino; methylamino, ethylamino, dimethylamino; methoxy, ethoxy, propoxy; cyclopropyl, cyclobutyl, cyclopentyl, 2-fluorocyclopropyl; phenyl, naphthyl, 2-fluorophenyl, 3-fluorophenyl, 4-fluorophenyl, 2-chlorophenyl, 3-chlorophenyl, 4-chlorophenyl, 2,4-difluorophenyl, 3,4-difluorphenyl, 2,6-difluorophenyl, 2-methylphenyl, 3-methylphenyl, 4-methylphenyl; 2-thiazolyl, 2-oxazolyl, 2-pyridyl, 3-pyridyl, 4-pyridyl, 2-pyrimidinyl, 4-pyrimidinyl, 5-pyrimidinyl, 2-benzoxazolyl, 2-benzothiazolyl; vinyl, 2-propenyl, 3,3-dimethyl-2-propenyl, 3,3-dichloro-2-propenyl; ethynyl and 2-propynyl groups,
more preferably a hydrogen atom; methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, 2-phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; and 2-propynyl groups,
still more preferably methyl, ethyl, 2-hydroxyethyl, 2-fluoroethyl, cyclopropyl and methylamino groups,
most preferably methyl, ethyl, 2-hydroxyethyl and methylamino groups.

The 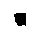 alkyl group having from 1 to 4 carbon atoms which may be substituted with a halogen atom, a hydroxyl group or an alkoxy group having from 1 to 4 carbon atoms*"* in the definition of A in the formula (f), in the case where R¹ and R² together form a group of the formula (f) in the formulae (Ia) and (Ib), includes alkyl groups such as methyl, ethyl, propyl, isopropyl and butyl; halogenoalkyl groups such as fluoromethyl, chloromethyl, difluoromethyl, trifluoromethyl, 2-fluoroethyl, 2-chloroethyl, 2-bromoethyl, 2-fluoropropyl, 3-fluoropropyl, 2-fluorobutyl, 3-fluorobutyl and 4-fluorobutyl; hydroxyalkyl groups such as hydroxymethyl, 2-hydroxyethyl, 3-hydroxypropyl and 4-hydroxybutyl; and alkoxyalkyl groups such as methoxymethyl, ethoxymethyl, propoxymethyl, butoxymethyl, methoxyethyl, methoxypropyl and methoxybutyl, preferably methyl, fluoromethyl and hydroxymethyl groups.

The 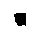 A*"* in the formula (f), in the case where R¹ and R² together form a group of the formula (f) in the formulae (Ia) and (Ib), includes preferably hydrogen atoms, and methyl, fluoromethyl and hydroxymethyl groups.

The 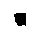 G*"* in the formula (f), in the case where R¹ and R² together form a group of the formula (f) in the formulae (Ia) and (Ib), includes preferably the group of the formula (g). Further, the alkyl group having from 1 to 4 carbon atoms of R¹¹ in the formula -N (R¹¹)- of G¹ of the formula (f) includes methyl, ethyl, propyl, isopropyl and butyl, preferably methyl and ethyl groups. G¹ of the formula (f) includes preferably oxygen and sulfur atoms, more preferably an omen atom.

The 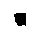 p*"* in the formula (f), in the case where R¹ and R² together form a group in the formula (f), in the formulae (Ia) and (Ib) is preferably 1.

The substituent R⁰ of the aryl group and the aromatic heterocyclic group of R³ and R⁶ in the formulae (h) and (i) of R in the formulae (Ia), (Ib) and (Ic) includes the same groups as the substituent R⁰ of the aromatic heterocyclic group which may be substituted with R⁰, in which the aromatic heterocyclic group is a substituent of the alkyl group having from 1 to 4 carbon atoms of R¹ in the above-mentioned general formulae (Ia) and (Ib), preferably fluorine and chlorine atoms; methyl, ethyl, trifluoromethyl, methoxy, ethoxy, methylthio and ethylthio groups, more preferably fluorine and chlorine atoms; and methyl, trifluoromethyl, methoxy, and methylthio groups.

The aryl group having from 6 to 10 carbon atoms of R³ and R⁶ in the formulae (h) and (I) of R in the formulae (Ia), (Ib) and (Ic) includes phenyl, 1-naphthyl and 2-naphthyl, preferably a phenyl group.

Examples of the aromatic heterocyclic group of R³ and R⁶ in the formulae (h) and (i) of R in the formulae (Ia), (Ib) and (Ic) include 2-thienyl, 2-furyl, 2-oxazolyl, 2-thiazolyl, 2-imidazolyl, 2-pyridyl, 3-pyridyl, 4-pyridyl, 2-pyrimidinyl, 4-pyrimidinyl, 5-pyrimidinyl, 2-pyrazinyl, 3-pyridazinyl, 2-benzoxazolyl, 2-benzothiazolyl, 2-benzimidazolyl, 3-benzisoxazolyl and 3-benzisothiazolyl, preferably 2-thiazolyl, 4-pyrimidinyl, 2-pyrazinyl, 3-benzisothiazolyl, 2-pyridyl, 2-pyrimidinyl, 2-benzothiazolyl and 2-benzoxazolyl groups, more preferably 2-pyrimidinyl, 2-pyridyl, 2-benzothiazolyl and 2-benzoxazolyl groups.

R³ and R⁶ in the formulae (h) and (i) of R in the formulae (Ia), (Ib) and (Ic) include preferably phenyl groups which may be substituted with R⁰ and aromatic heterocyclic groups which may be substituted with R⁰, more preferably the group in which R is the group of the formula (h) and R³ in the formula (h) is an aromatic heterocyclic group which may be substituted with R⁰, particularly preferably the group in which R is the group of the formula (h) and R³ in the formula (h) is a 5- or 6-membered aromatic heterocyclic group containing one or two nitrogen atoms which may be substituted with R⁰, most preferably the group in which R is the group of the formula (h) and R³ in the formula (h) is a pyridyl, pyrazinyl, pyrimidinyl, thiazolyl, benzothiazolyl, benzisothiazolyl or benzoxazolyl group which may be substituted with R⁰.

Further, more specifically, R³ and R⁶ in the formulae (h) and (i) of R in the formulae (Ia), (Ib) and (Ic) include preferably phenyl, 4-fluorophenyl; 2-pyridyl; 2-pyrazinyl; 2-pyrimidinyl, 4-pyrimidinyl; dimethoxy-2-pyrimidinyl; 2-thiazolyl; 2-benzoxazolyl; 2-benzothiazolyl; 3-benzisothiazolyl; phenyl which is substituted with fluorine, chlorine, methoxy, nitro, trifluoromethyl, amino or dimethylamino at the 2-, 3- or 4-position; 2-pyridyl which is substituted with methoxy, amino or nitro; 2-pyrimidinyl which is substituted with chlorine, methyl or ethyl; 4-pyrimidinyl which is substituted with chlorine, methyl or ethyl; 2-benzothiazolyl which is substituted with chlorine, methyl or methoxy; and 2-benzoxazolyl which is substituted with chlorine, methyl or methoxy groups.
more preferably phenyl, 4-fluorophenyl; 2-pyridyl; 2-pyrazinyl; 2-pyrimidinyl, 4-pyrimidinyl; 2-thiazolyl; 2-benzoxazolyl; 2-benzothiazolyl; phenyl which is substituted with fluorine, chlorine, methoxy, nitro, trifluoromethyl, amino or dimethylamino at the 2-, 3- or 4-position; 2-pyridyl which is substituted with methoxy or nitro; 2-pyrimidinyl which is substituted with chlorine, methyl or ethyl; 4-pyrimidinyl which is substituted with chlorine, methyl or ethyl; 2-benzothiazolyl which is substituted with methoxy; and 2-benzoxazolyl which is substituted with a methoxy group;
still more preferably phenyl; 2-pyridyl; 2-pyrimidinyl; 2-thiazolyl; 2-benzothiazolyl; 2-benzoxazolyl; 6-methoxybenzothiazolyl; and 6-methoxybenzoxazolyl groups,
most preferably 2-pyrimidinyl; 2-benzothiazolyl; 2-benzoxazolyl; and 6-methoxybenzothiazolyl groups.

Examples of the alkyl group having from 1 to 4 carbon atoms of R⁴, R⁵ and R⁷ in the formulae (h) and (i) of R in the (Ia), (Ib) and (Ic) include alkyl groups having from 1 to 4 carbon atoms such as methyl, ethyl, propyl, isopropyl, butyl, isobutyl, s-butyl and t-butyl, preferably methyl, ethyl, propyl and isopropyl groups, more preferably methyl and ethyl groups.

R⁴, R⁵ and R⁷ in the formulae (h) and (i) of R in the formulae (Ia), (Ib) and (Ic) include preferably hydrogen atoms, and methyl, ethyl, propyl and isopropyl groups, more preferably hydrogen atoms, and methyl and ethyl groups.

The alkyl group having from 1 to 4 carbon atoms of R⁸ in the formula (i) of R in the formulae (Ia), (Ib) and (Ic) includes alkyl groups such as methyl, ethyl, propyl, isopropyl, butyl, isobutyl and t-butyl, preferably methyl, ethyl, propyl and isopropyl groups, more preferably methyl and ethyl groups.

The alkoxy group having from 1 to 4 carbon atoms of R⁸ in the formula (i) of R in the formulae (Ia), (Ib) and (Ic) includes alkoxy groups such as methoxy, ethoxy, propoxy, isopropoxy and butoxy, preferably methoxy, ethoxy and propoxy groups.

R⁸ in the formula (i) of R in the formulae (Ia), (Ib) and (Ic) preferably includes hydrogen atoms, and hydroxyl, methyl, ethyl, propyl, isopropyl, methoxy, ethoxy and propoxy groups, more preferably hydrogen atoms, and hydroxyl, methyl, ethyl, methoxy and ethoxy groups.

In 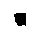 R*"* in the formulae (Ia), (Ib) and (Ic), and in the formula (h), n is preferably 1, and in the formula (i), the sum of n' and n'' is preferably 3, 4 or 5, more preferably 3 or 4, and m is preferably 0.

R in the formulae (Ia), (Ib) and (Ic) preferably includes 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl and 4-(2-pyridyl)piperazin-1-yl groups, more preferably 4-(2-pyrimidinyl)piperazin-1-yl, 4-(2-benzothiazolyl)piperazin-1-yl, 4-(2-benzoxazolyl)piperazin-1-yl, 4-(2-pyridyl)piperazin-1-yl and 4-(6-methoxy-2-benzothiazolyl)piperazin-1-yl groups.

In the case where the above-mentioned general formula (Ia), (Ib) or (Ic) has carboxyl groups in the molecule, these carboxyl groups may be protected with a protecting group to be an ester, and such a protecting group includes the same groups as those described on 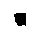 the optionally protected carboxyl group*"* of the above-mentioned Z.

The compounds of the formulae (Ia), (Ib) and (Ic) can be, if necessary, a pharmacologically acceptable salt.

Such a salt includes a mineral acid salt such as hydrochloric acid salt, hydrobromic acid salt, hydroiodic acid salt, sulfuric acid salt and phosphoric acid salt; an organic acid salt such as methanesulfonic acid salt, ethanesulfonic acid salt, benzenesulfonic acid salt, p-toluenesulfonic acid salt, oxalic acid salt, maleic acid salt, fumaric acid salt, tartaric acid salt and citric acid salt; and a metal salt of carboxylic acid such as sodium salt, potassium salt, calcium salt, magnesium salt, manganese salt, iron salt and aluminum salt.

Further, the compounds of the formulae (Ia), (Ib) and (Ic) of the present invention can also exist as a hydrate.

Of the compounds of the formulae (Ia), (Ib) and (Ic) which are the active ingredients of the present invention, preferable compounds include :
1) the compound in which X is a fluorine atom,
2) the compound in which Y is a hydrogen or fluorine atom, or an amino, methyl or ethyl group,
3) the compound in which Y is a hydrogen atom,
4) the compound in which Z is an optionally protected carboxyl group,
5) the compound in which Q is a group of the formula (d) and R² of the formula (d) is a difluoromethoxy or trifluoromethyl group,
6) the compound in which W is a sulfur atom,
7) the compound in which T is an ethylidene, -CH=CH- or -C(CH₃)=CH- group,
8) the compound in which T is an ethylidene group,
9) the compound in which R¹ is a hydrogen atom; a methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group,
10) the compound in which R¹ is a methyl, ethyl, 2-hydroxyethyl, cyclopropyl or methylamino group,
11) the compound in which R is a 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group, and
12) the compound in which R is a 4-(2-pyrimidinyl)piperazin-1-yl, 4-(2-benzothiazolyl)piperazin-1-yl, 4-(2-benzoxazolyl)piperazin-1-yl, 4-(2-pyridyl)piperazin-1-yl or 4-(6-methoxy-2-benzothiazolyl)piperazin-1-yl group.

The compound of the formula (Ia), (Ib) or (Ic) which is the active ingredient of the present invention can be exemplified in Table 1 to Table 31 and Table A to Table G.

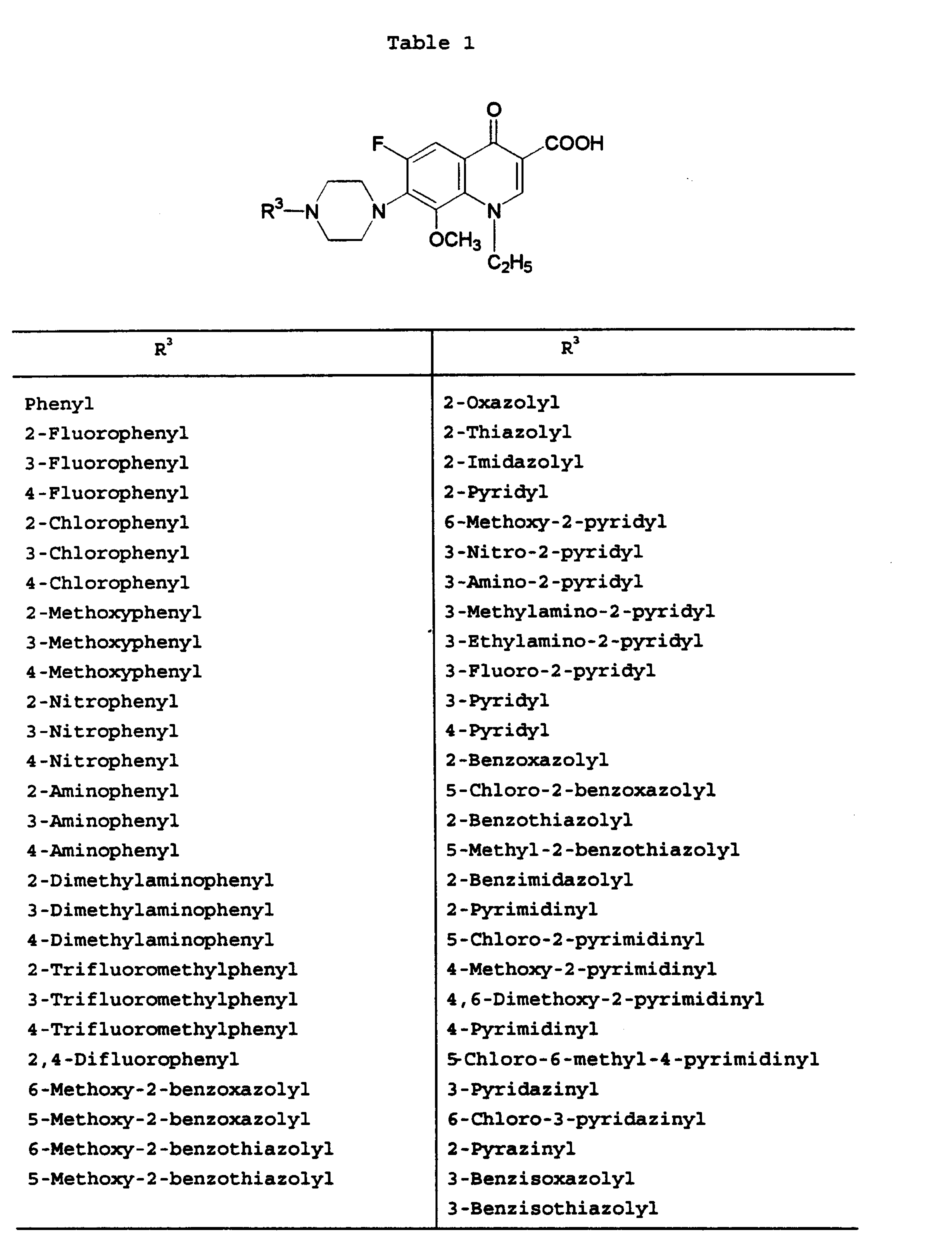

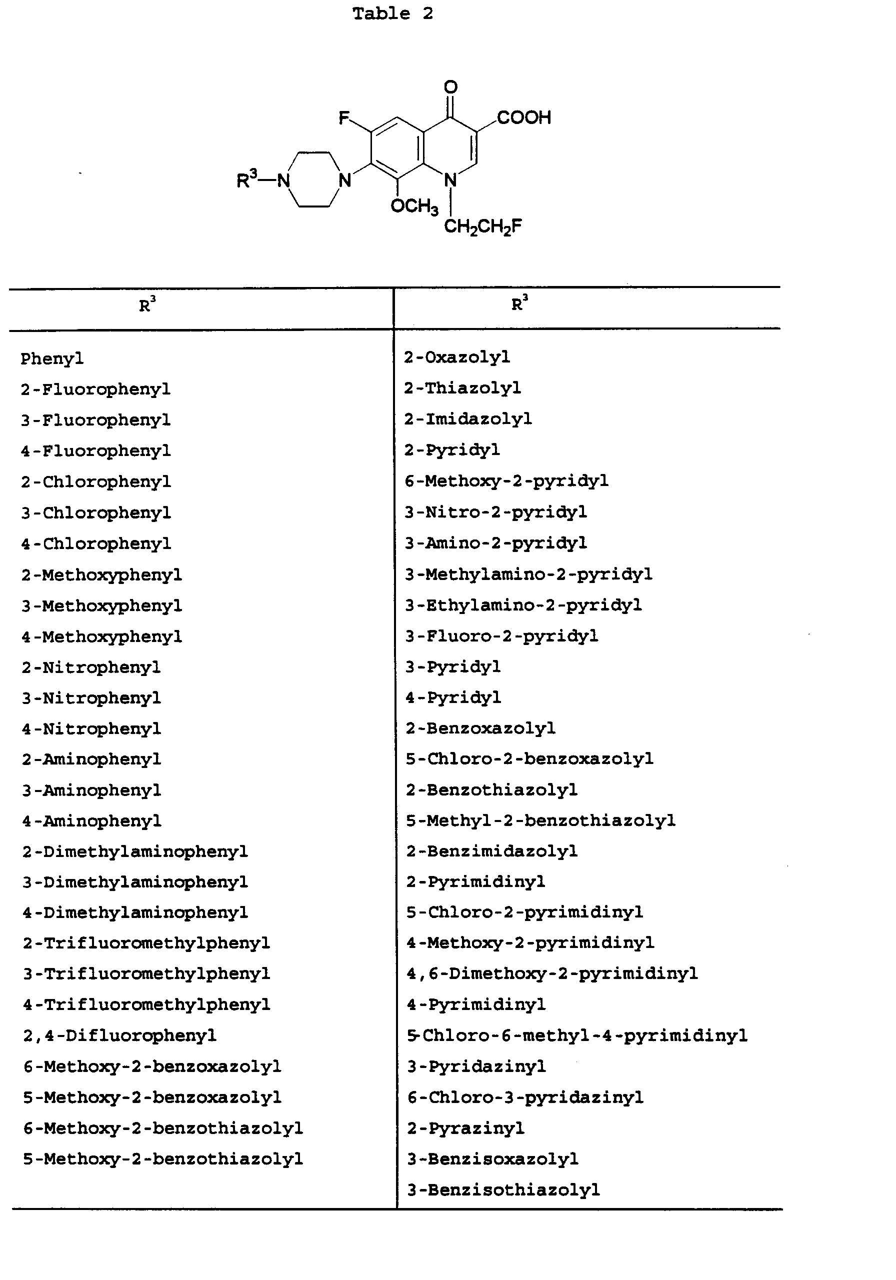

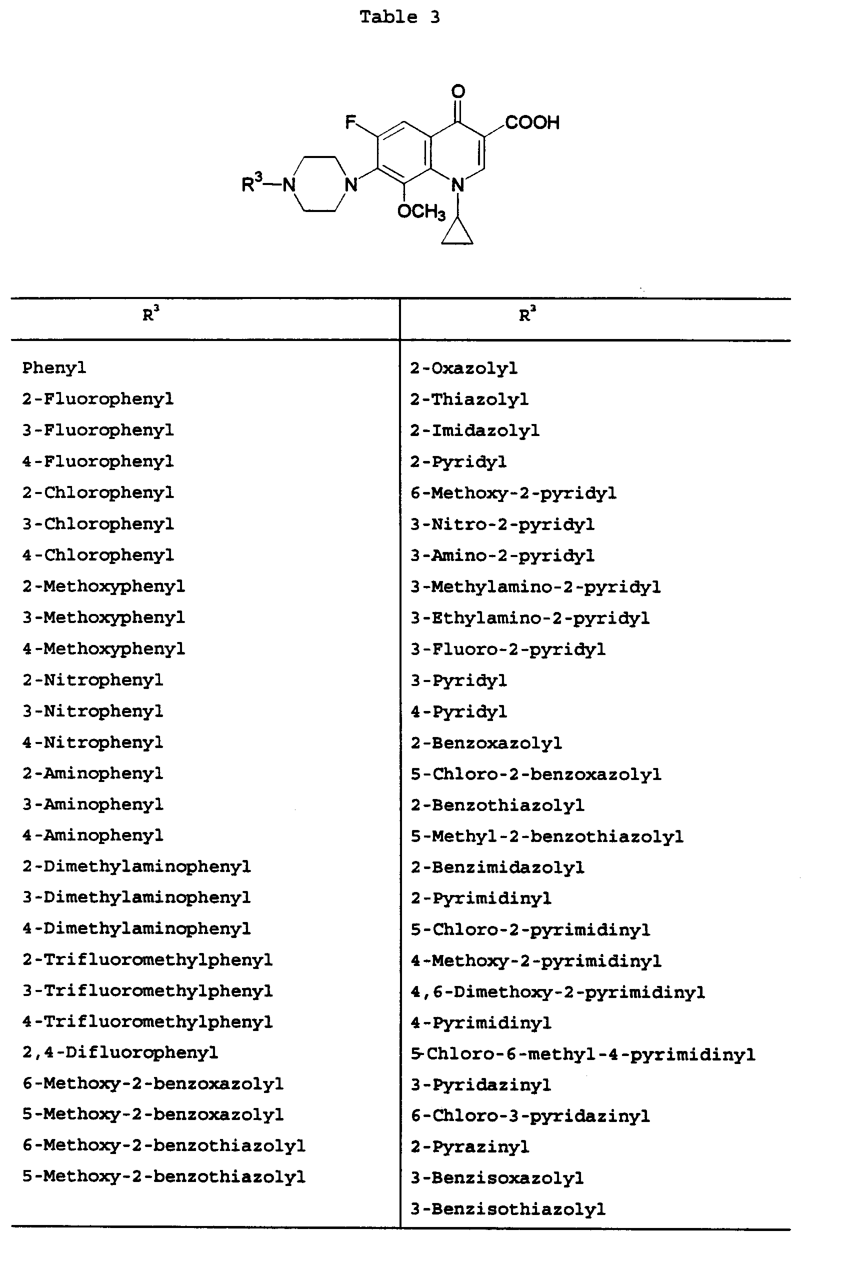

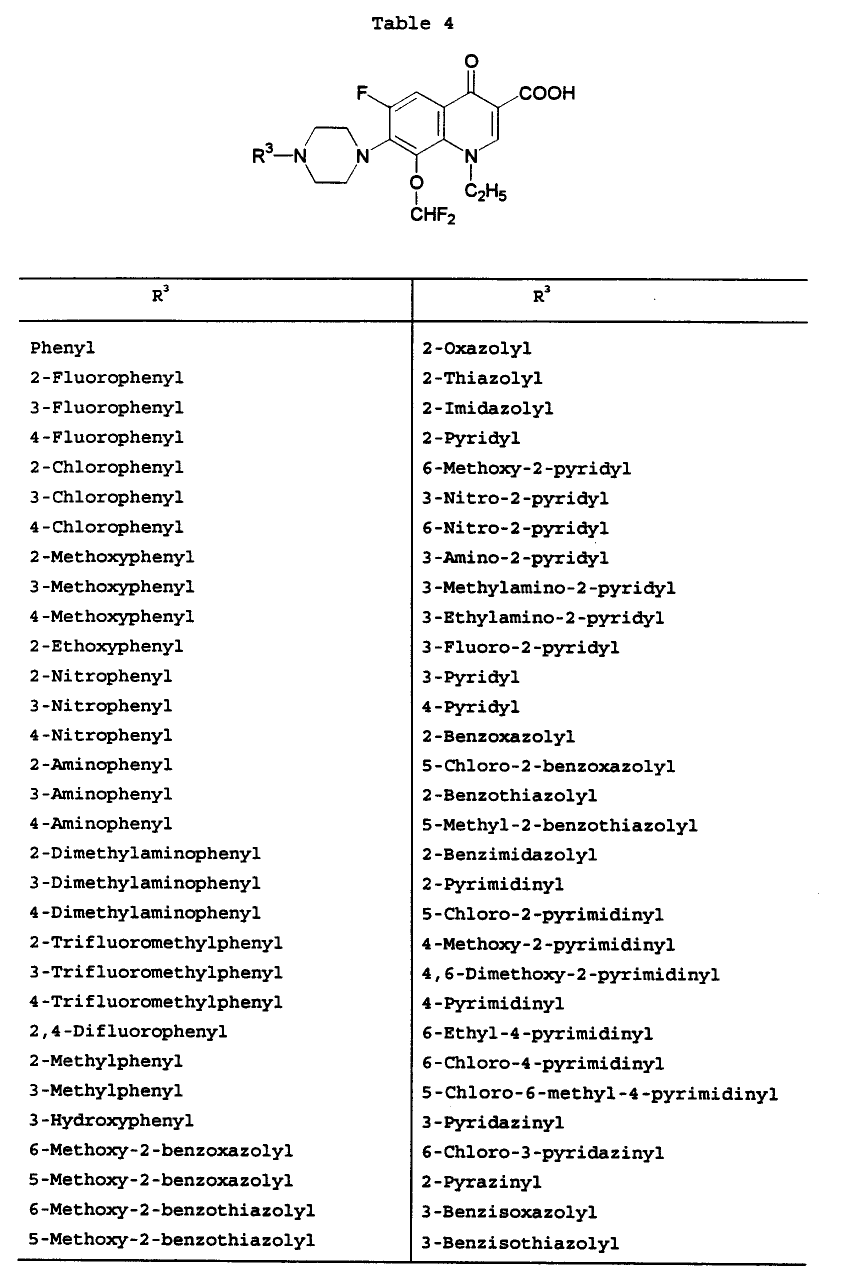

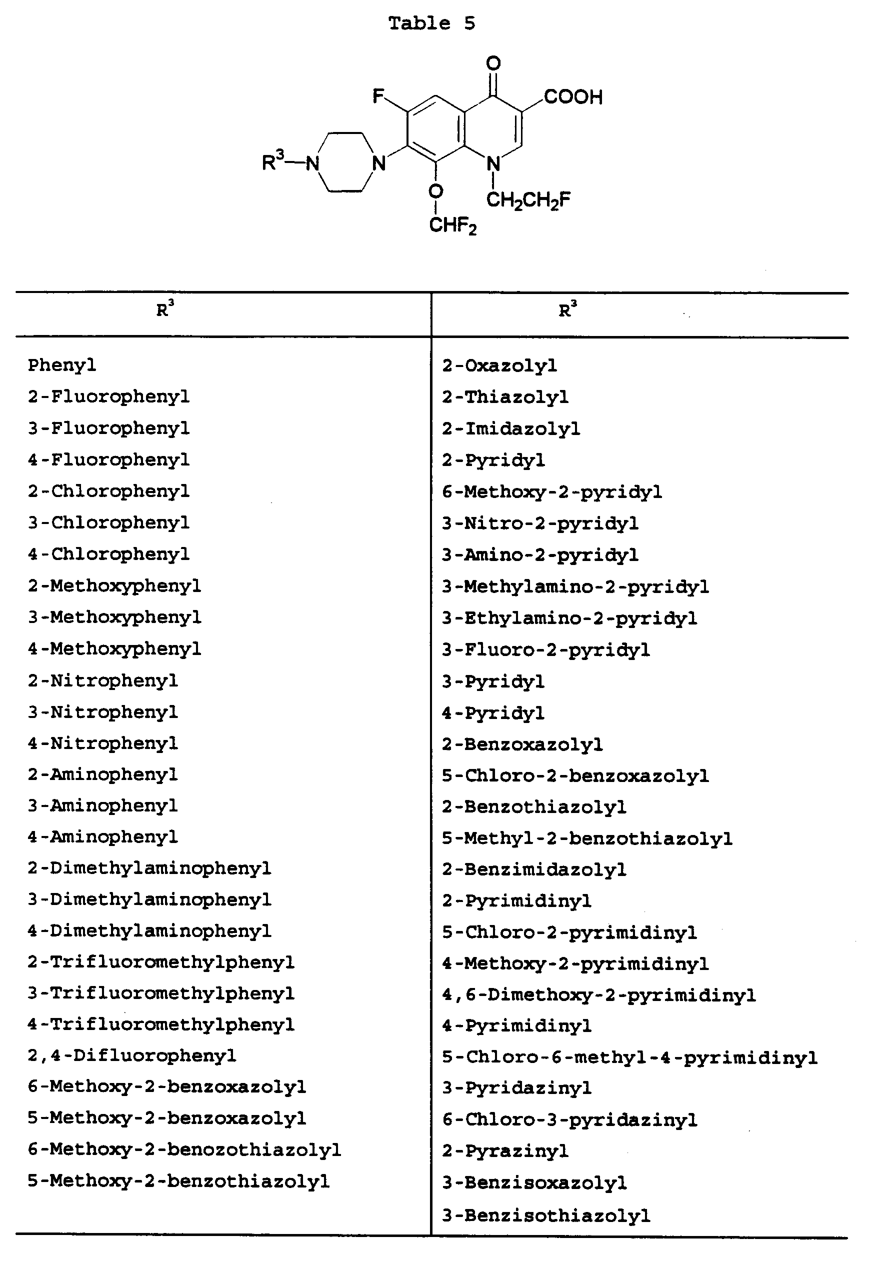

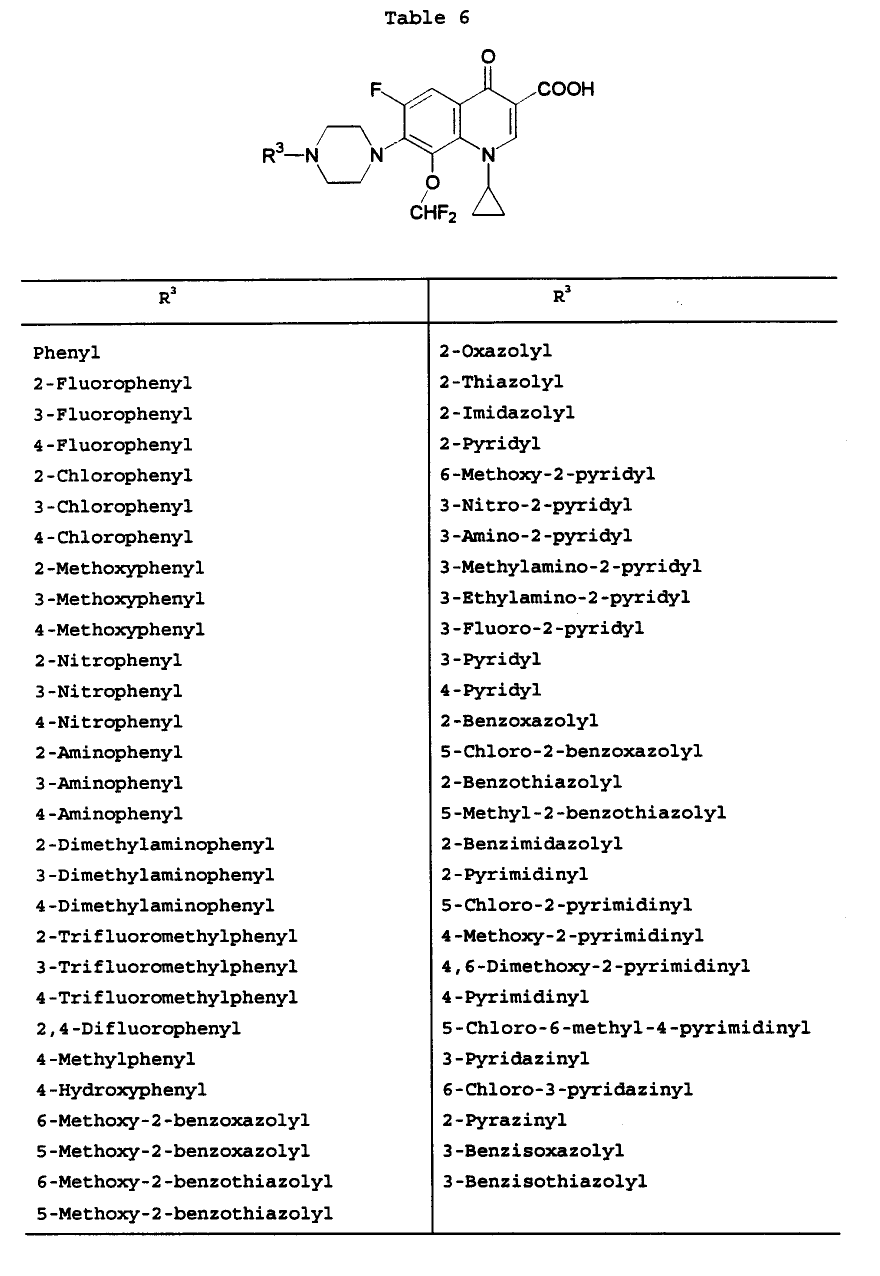

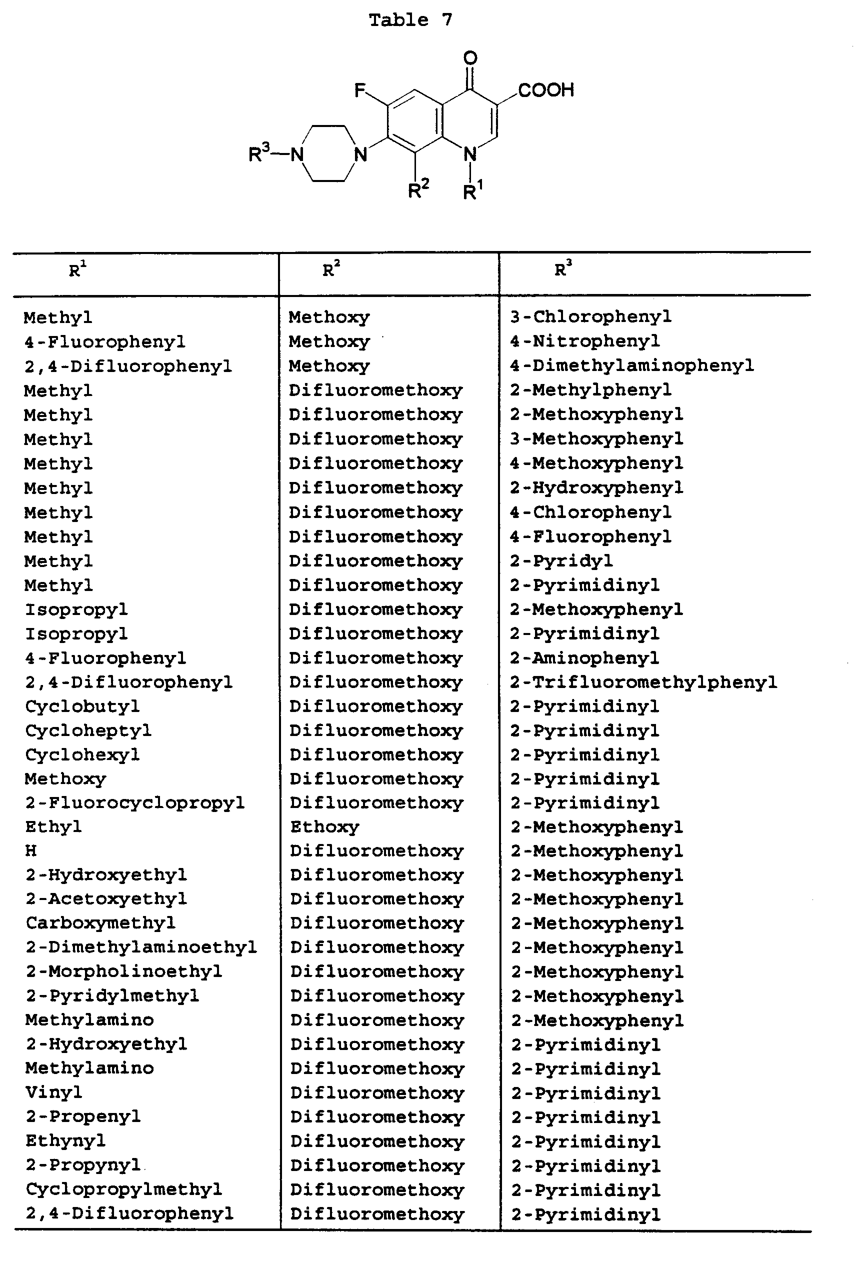

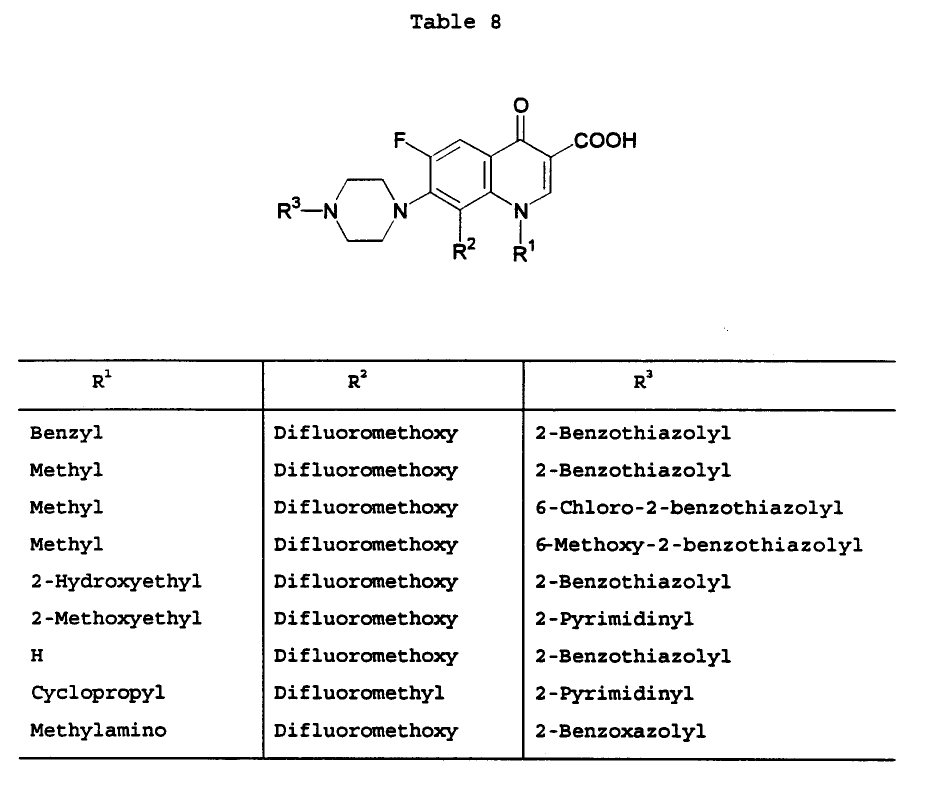

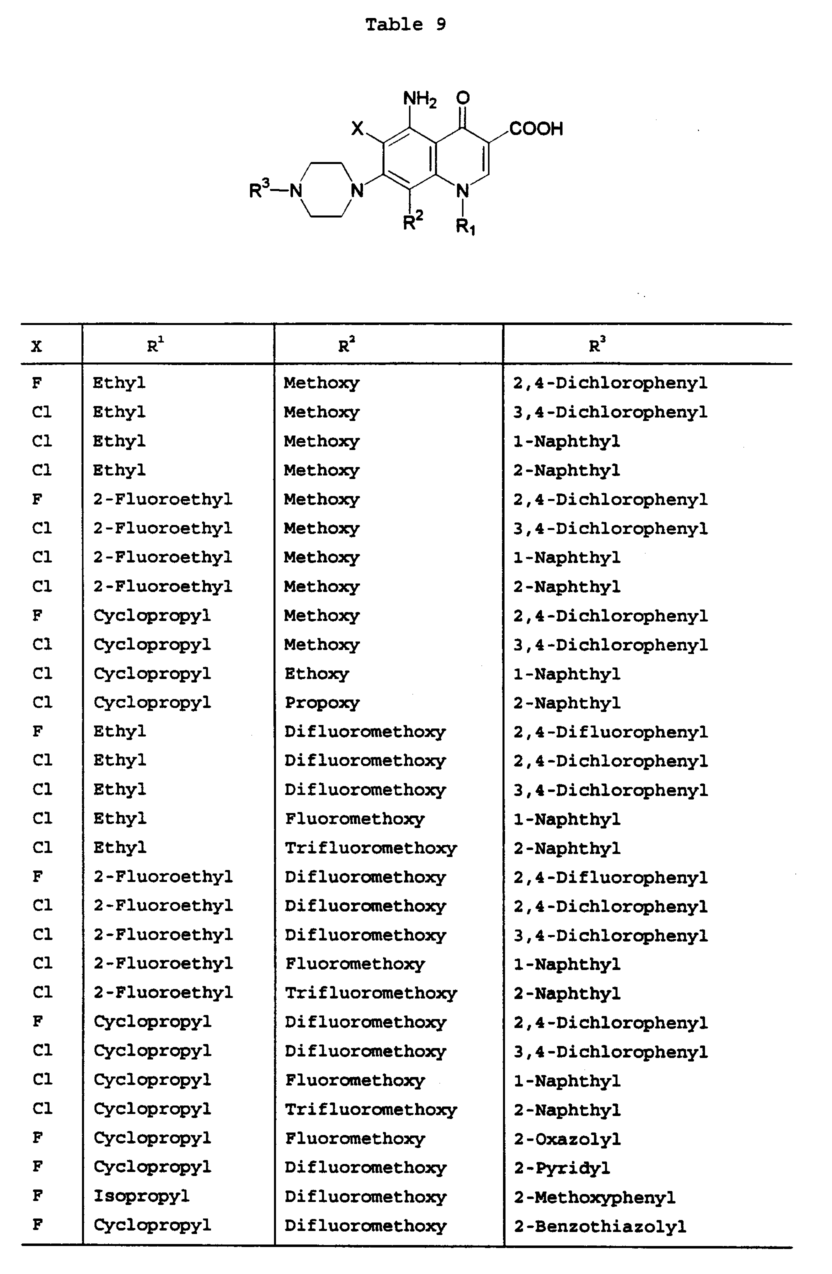

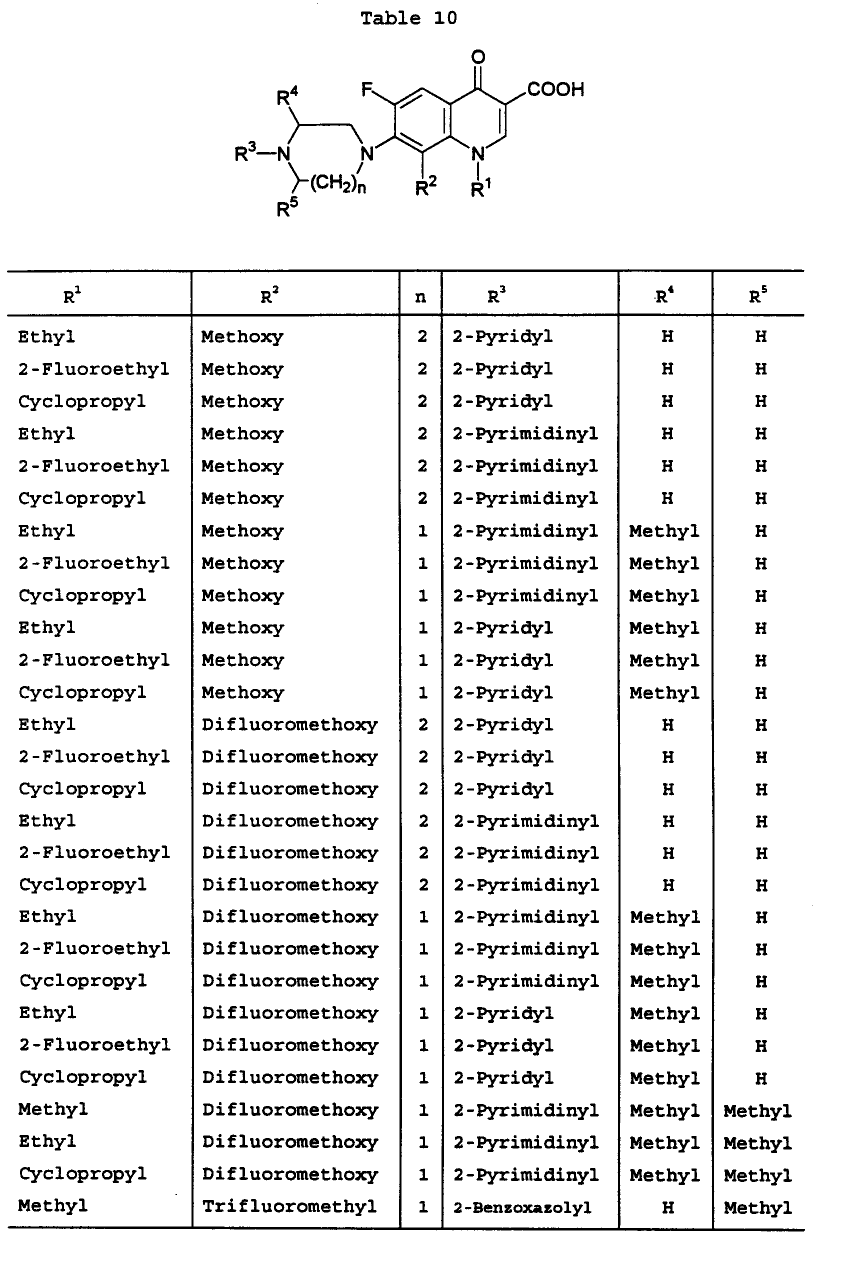

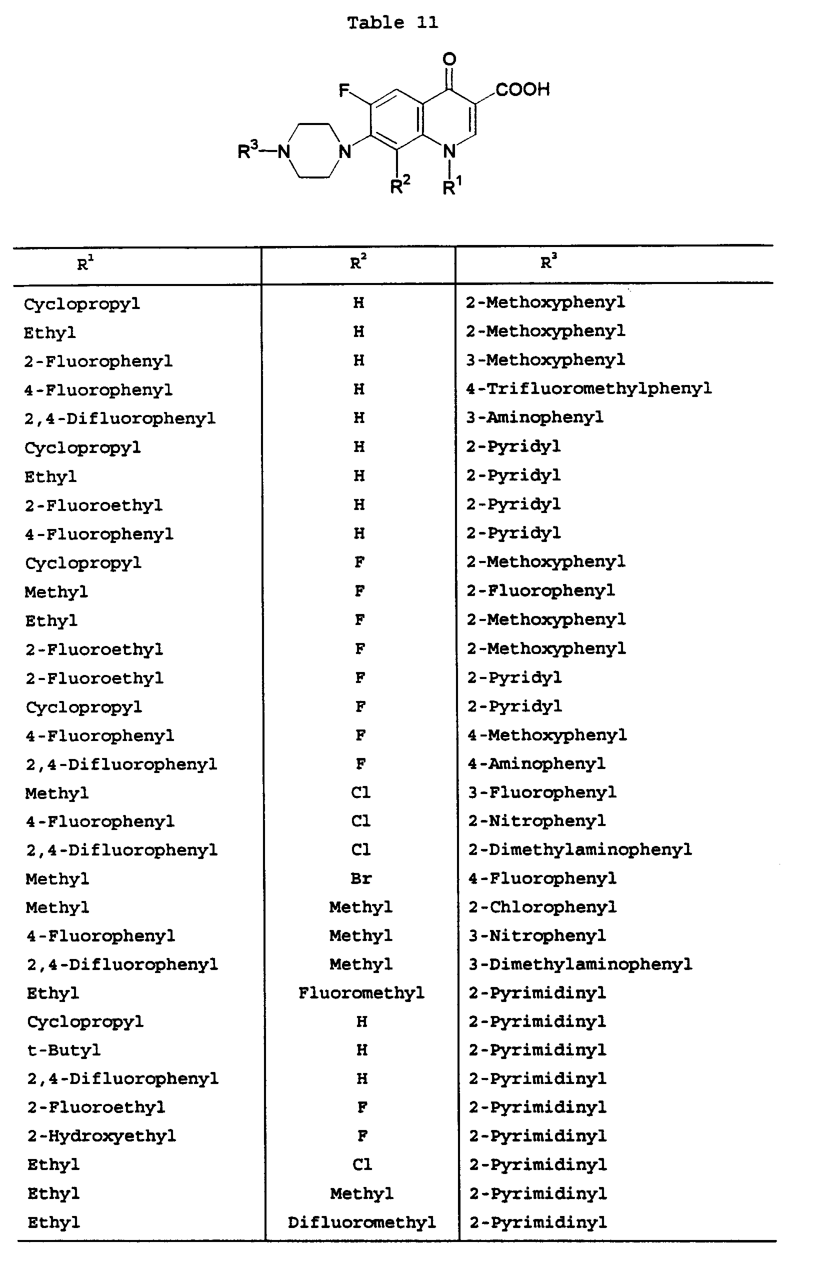

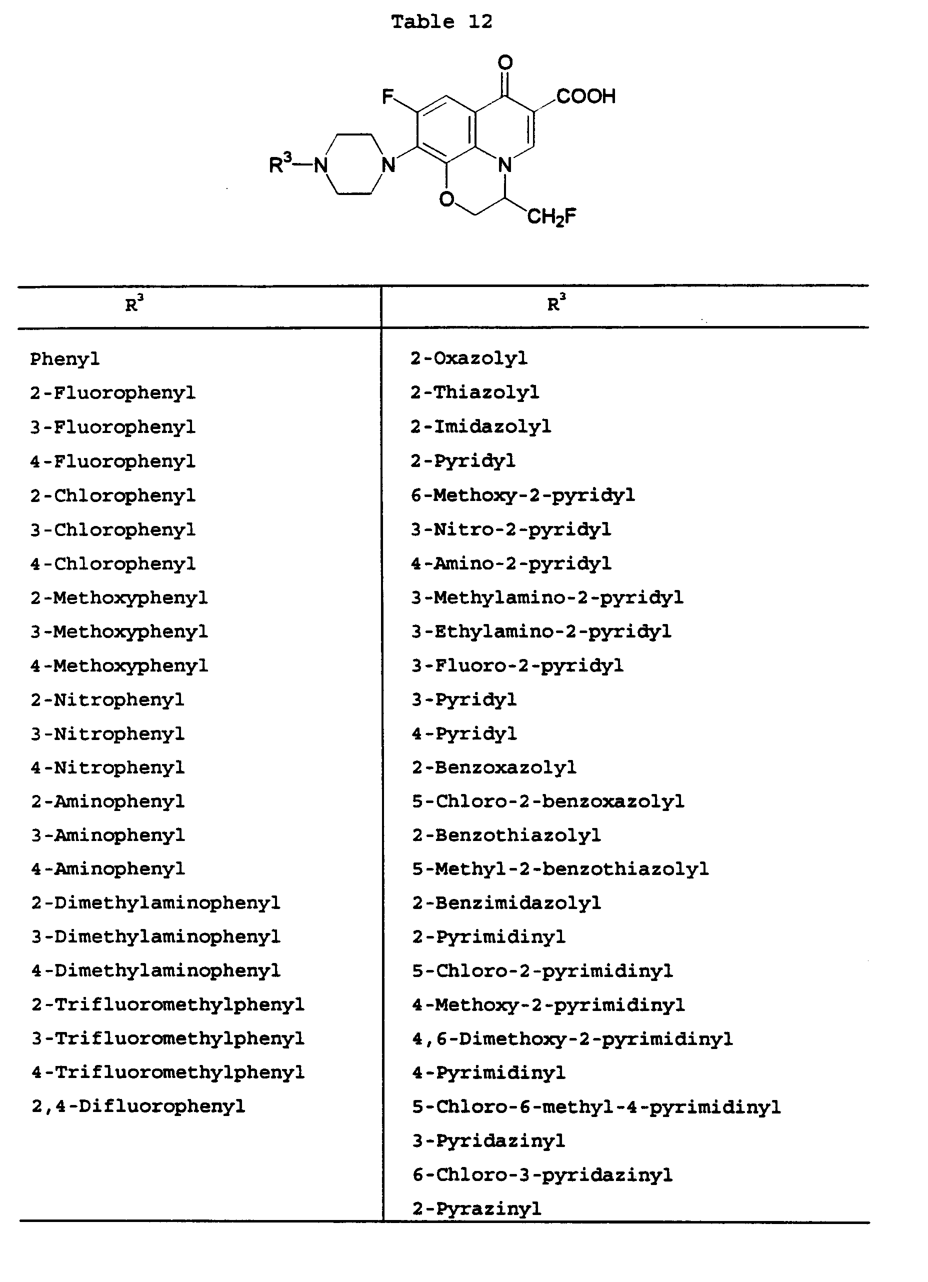

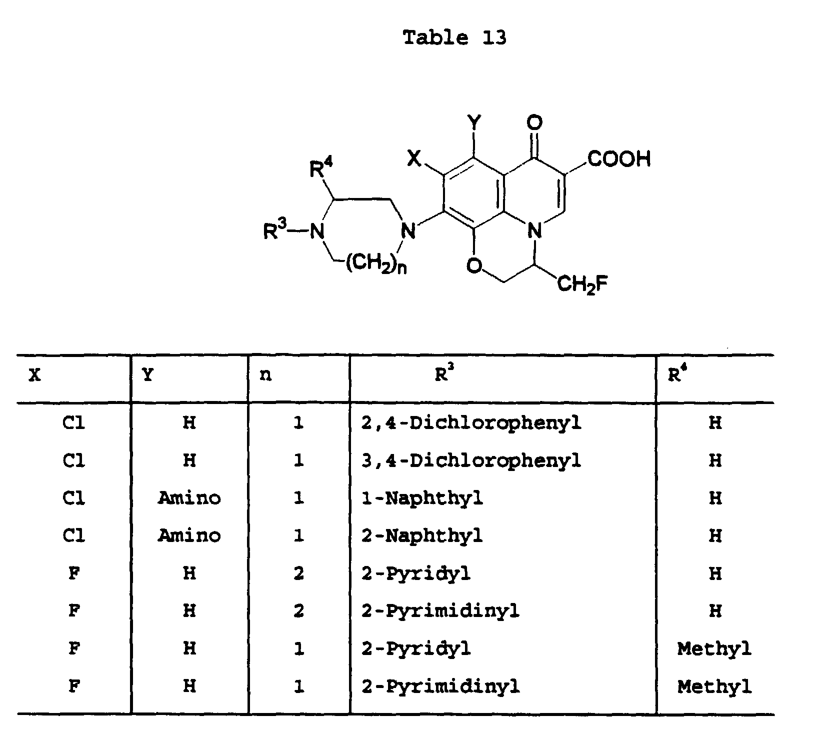

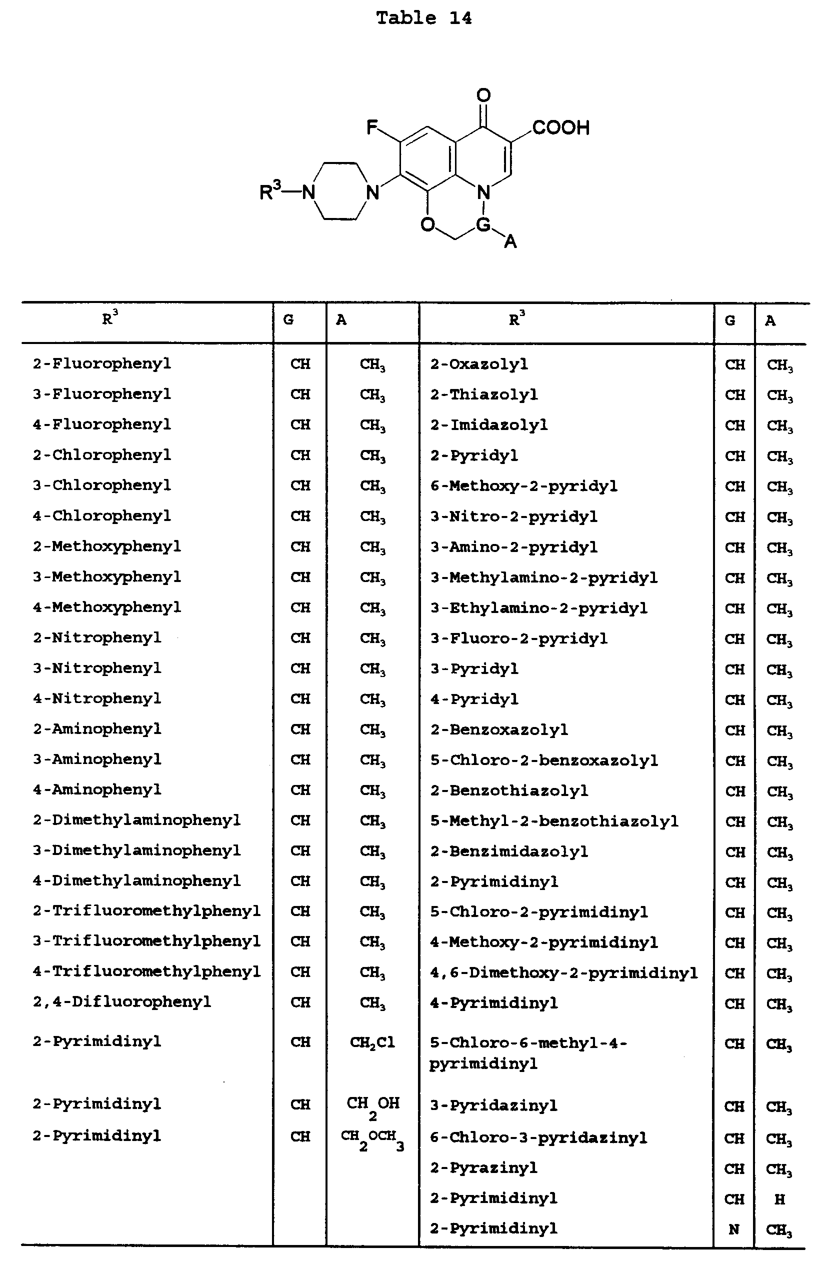

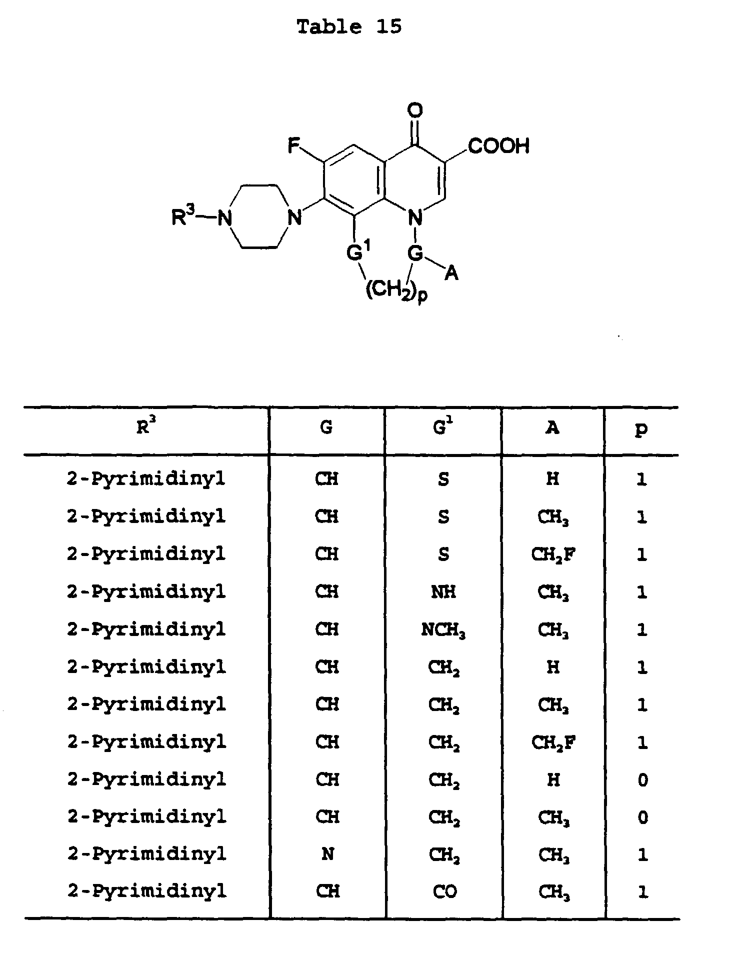

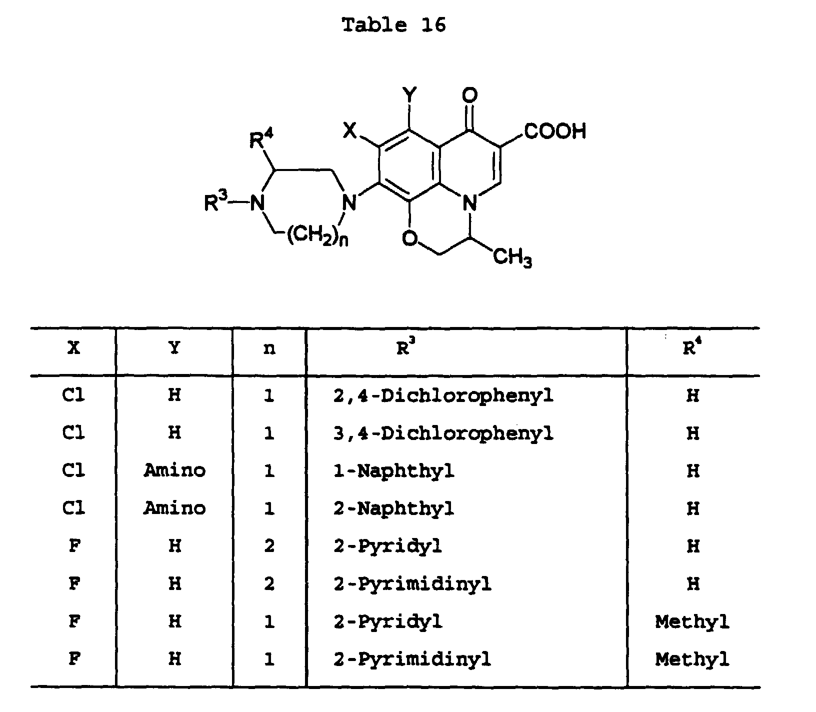

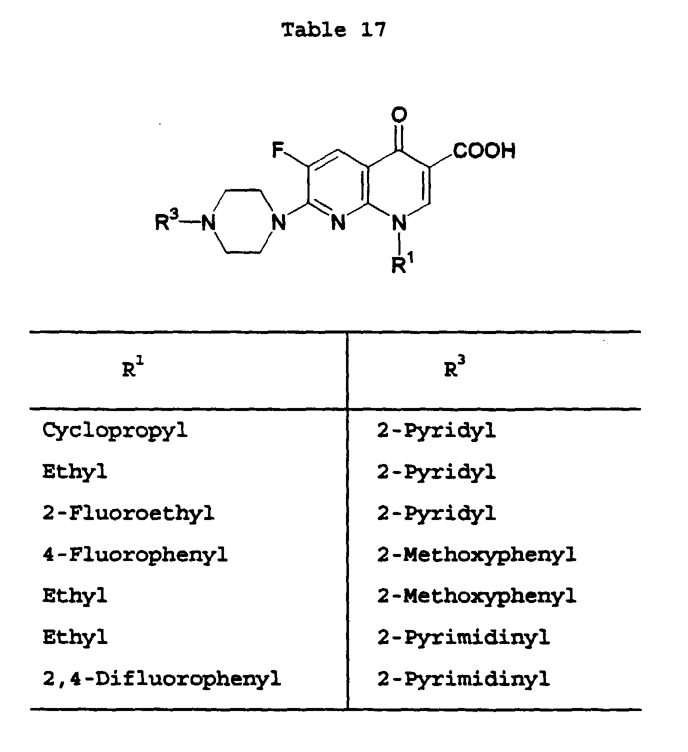

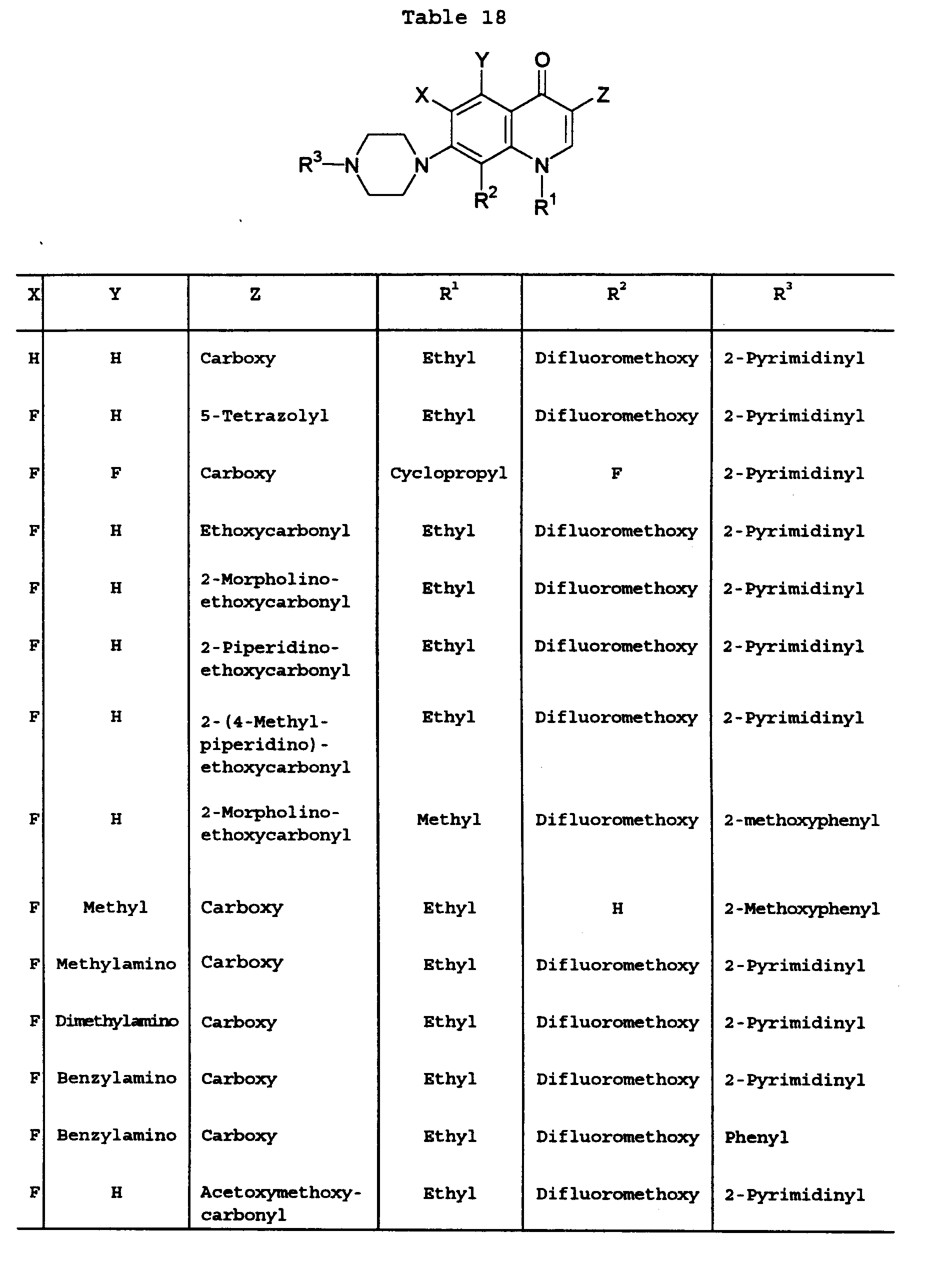

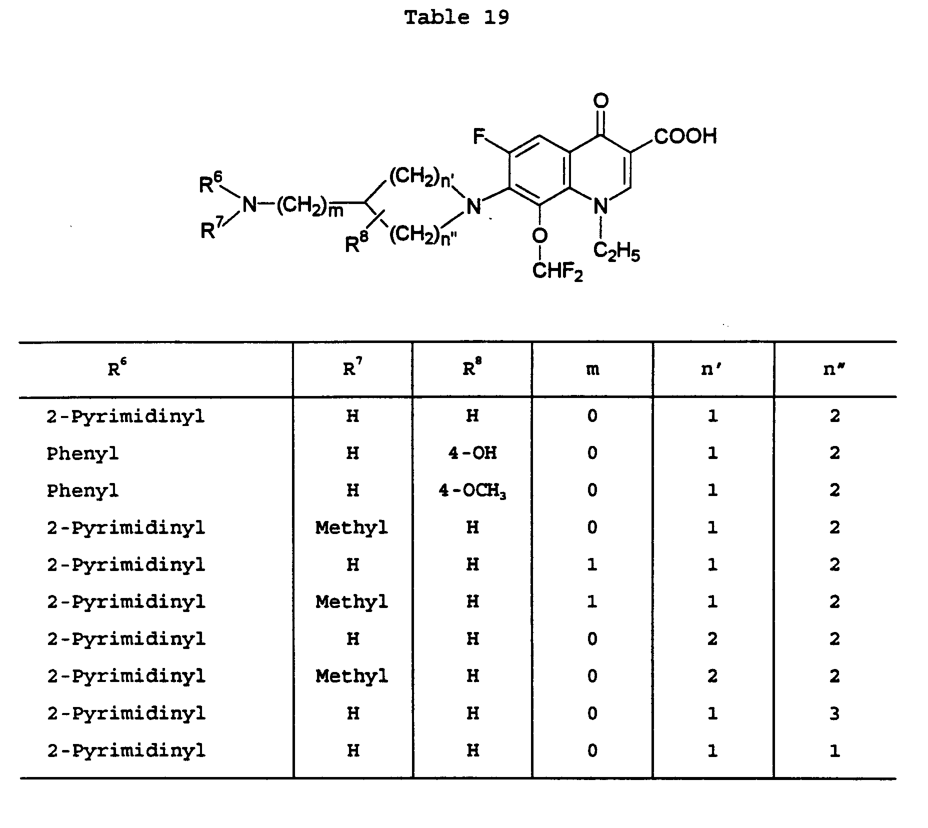

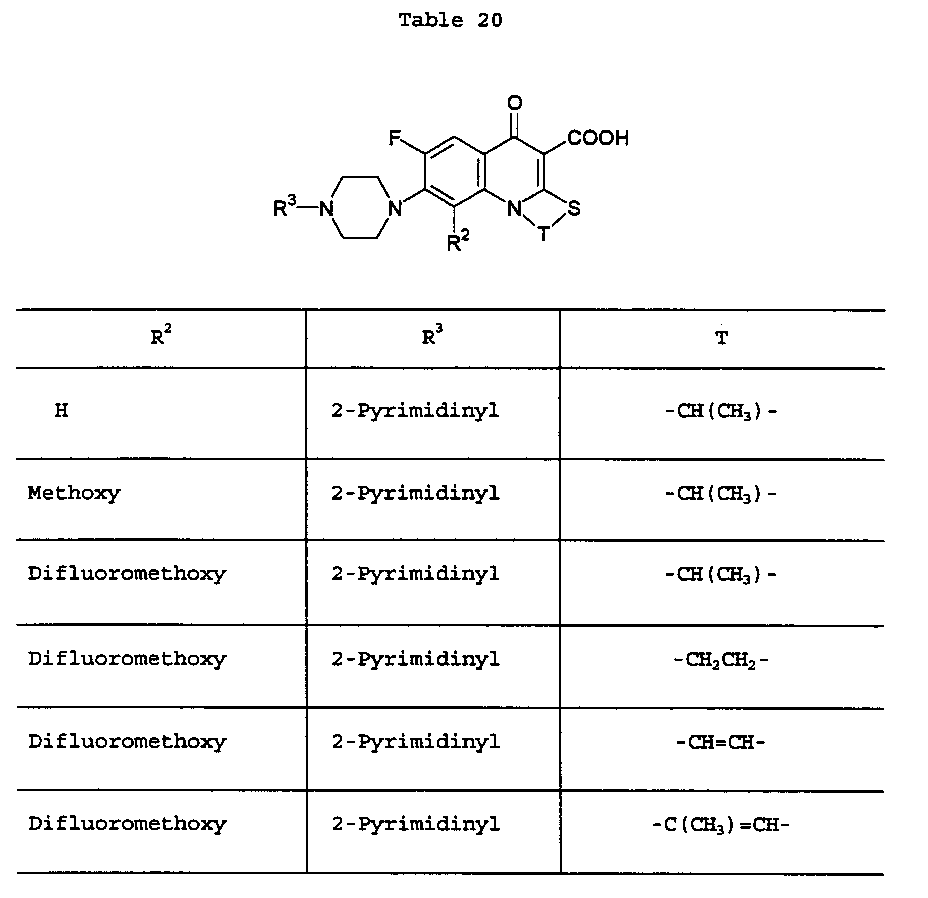

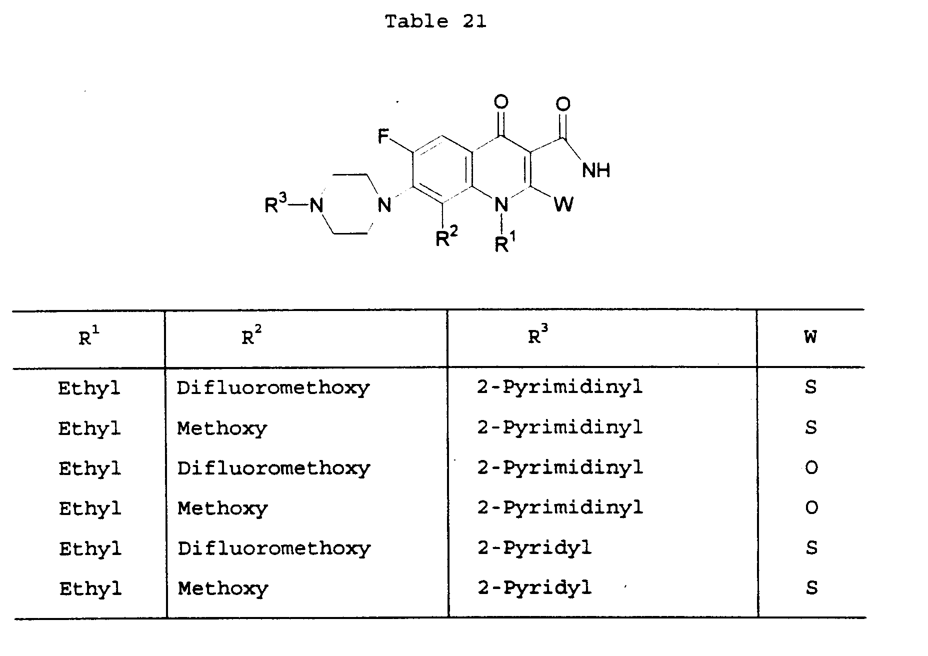

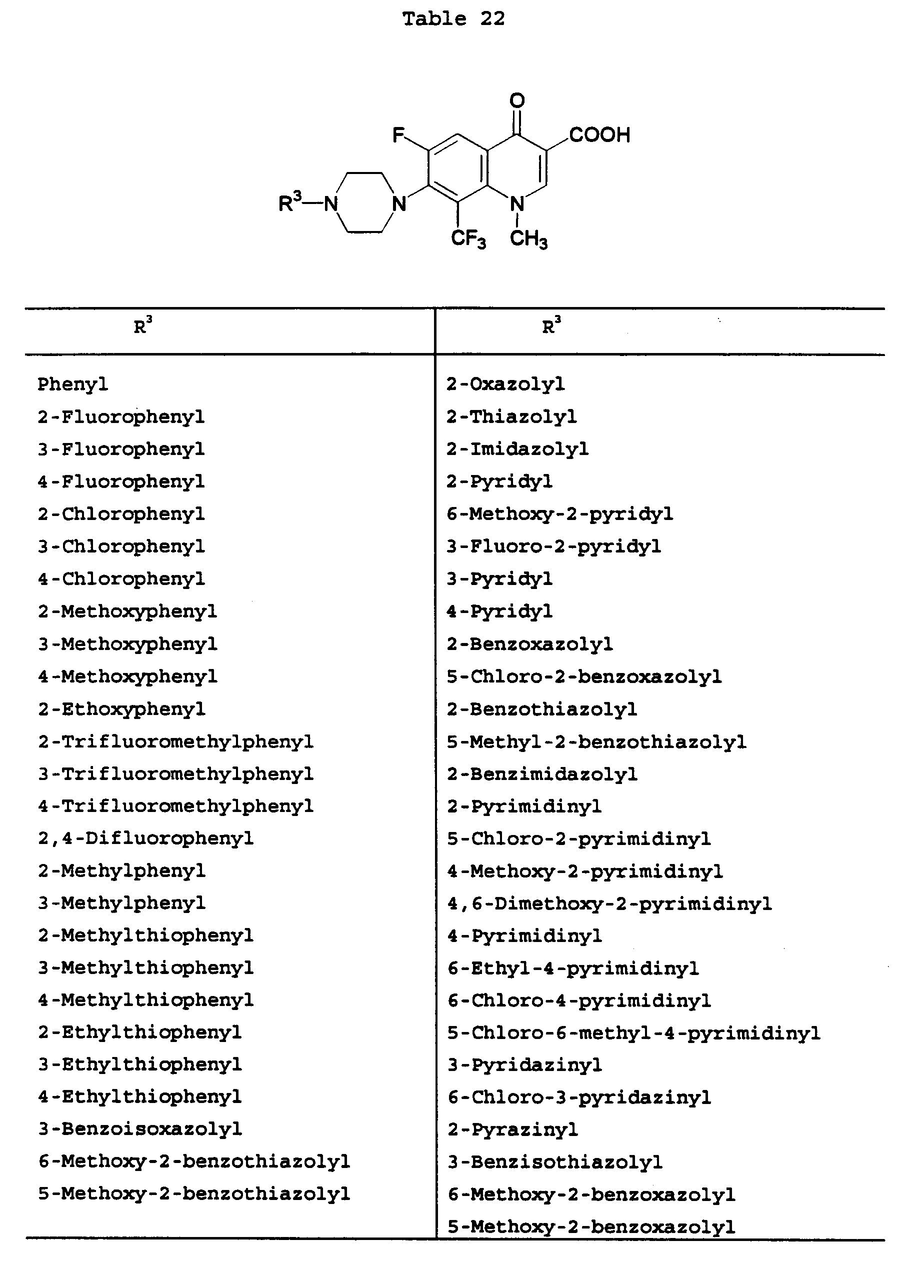

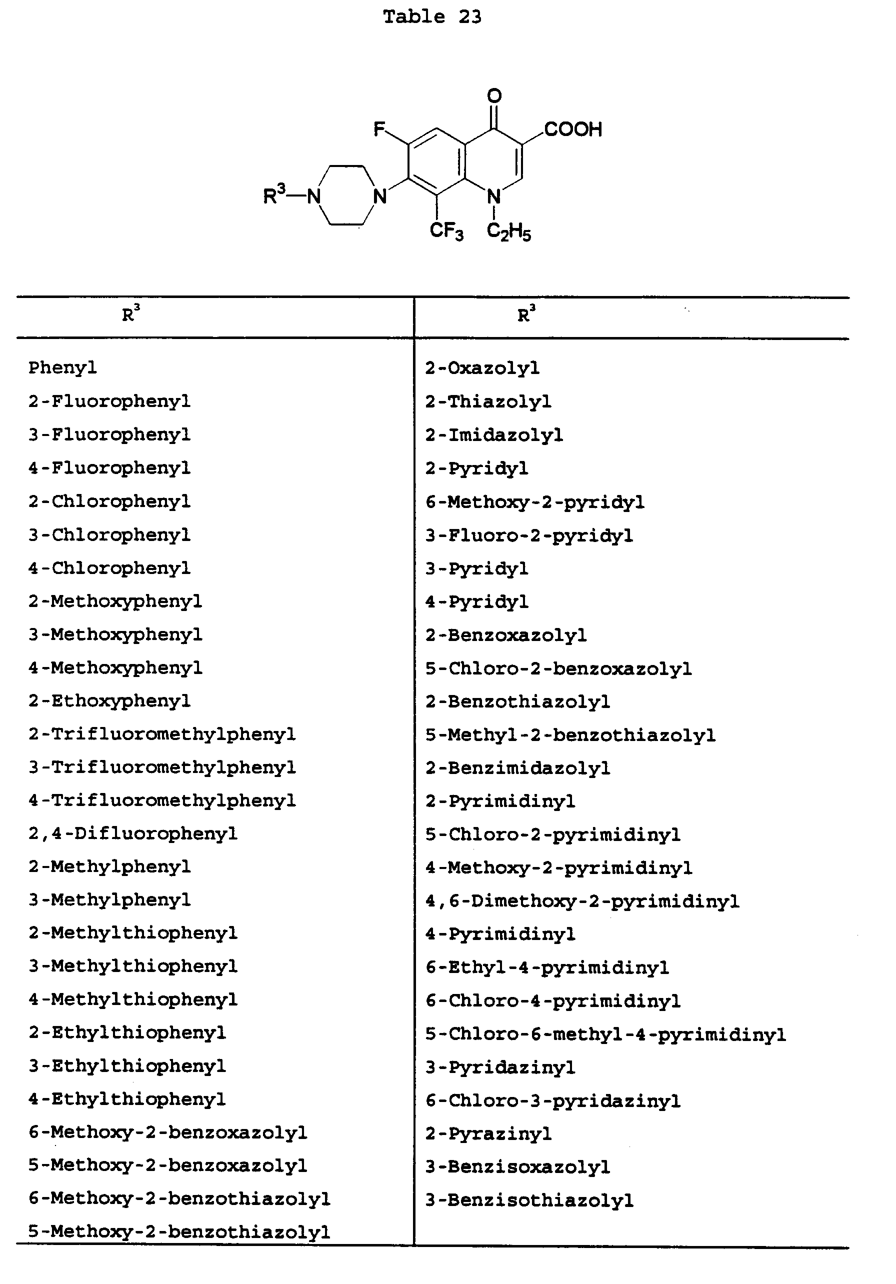

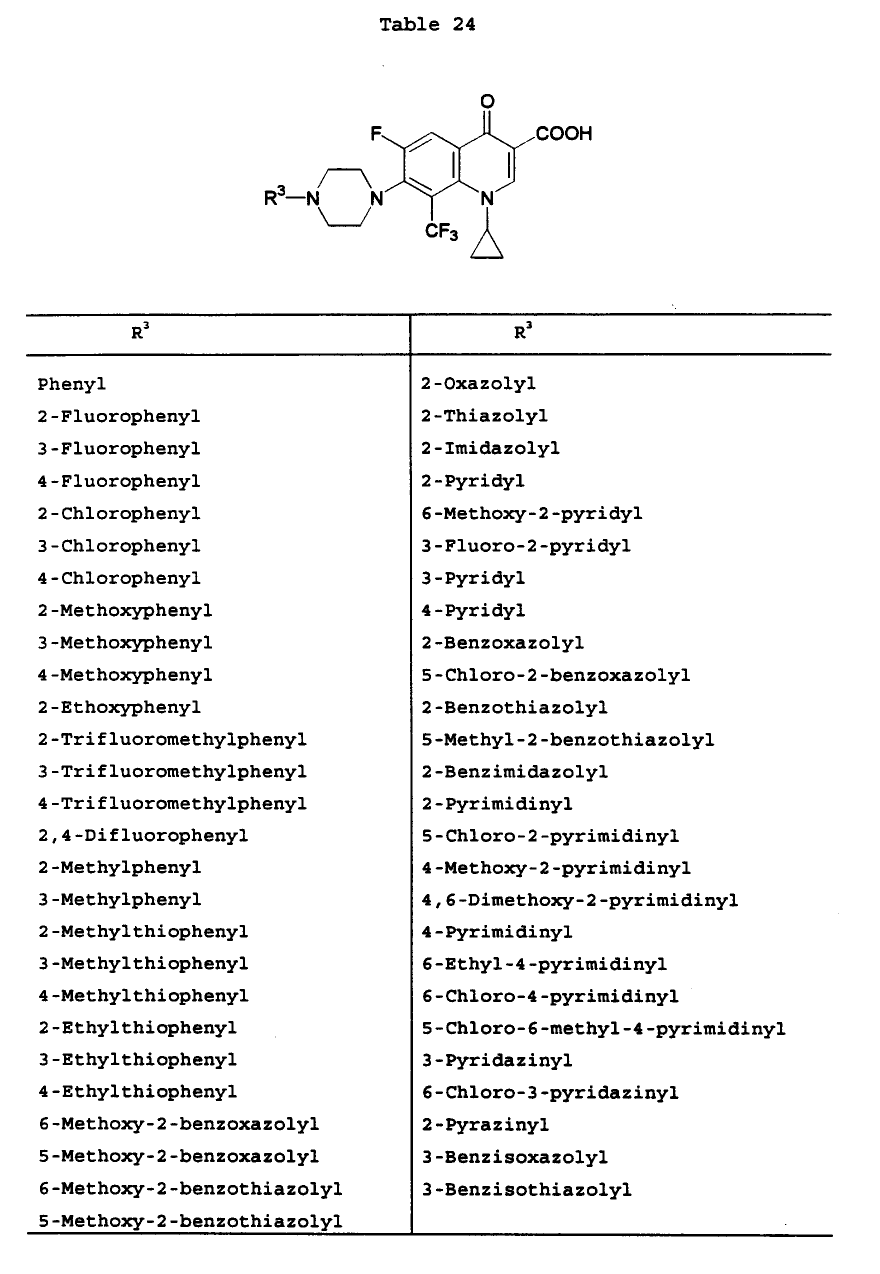

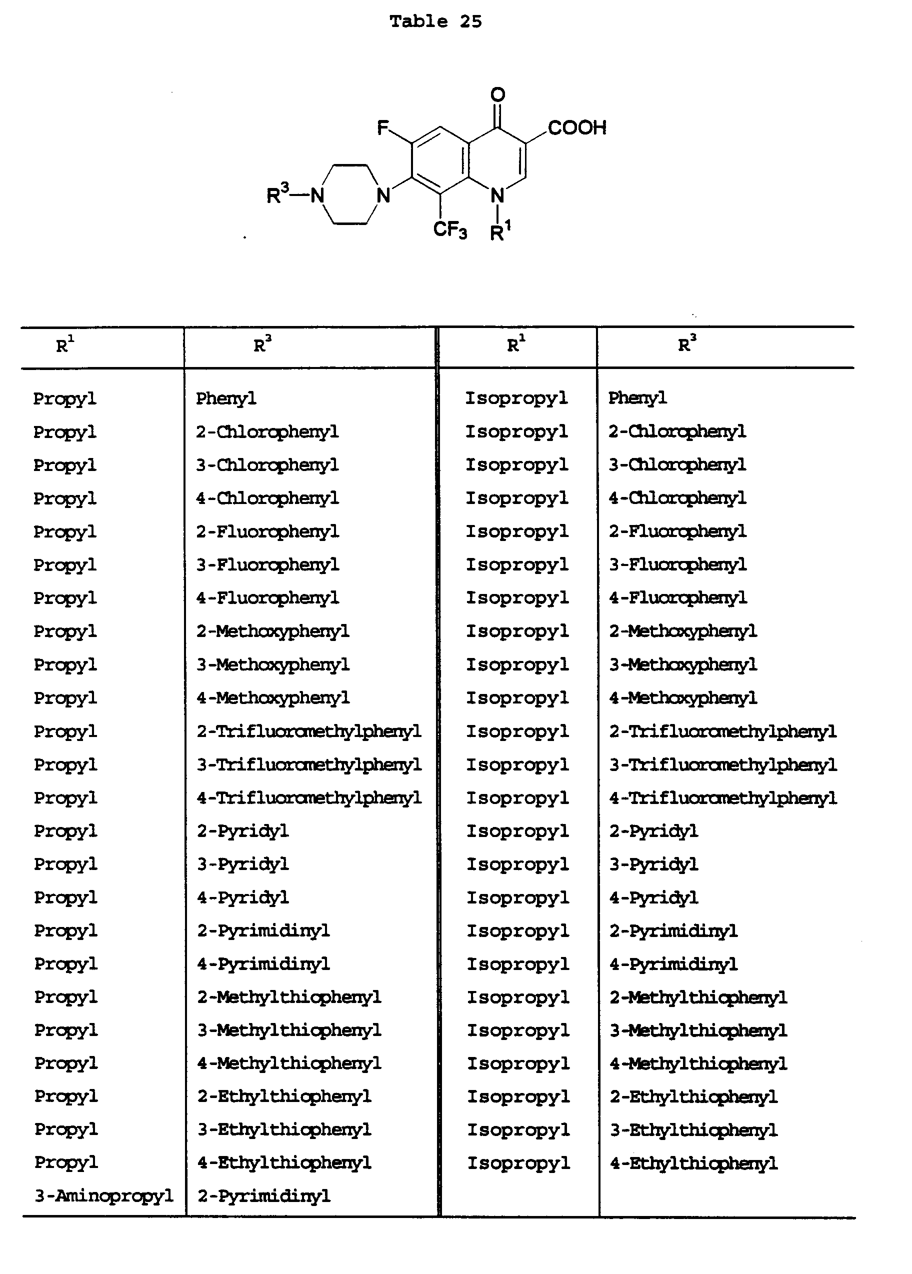

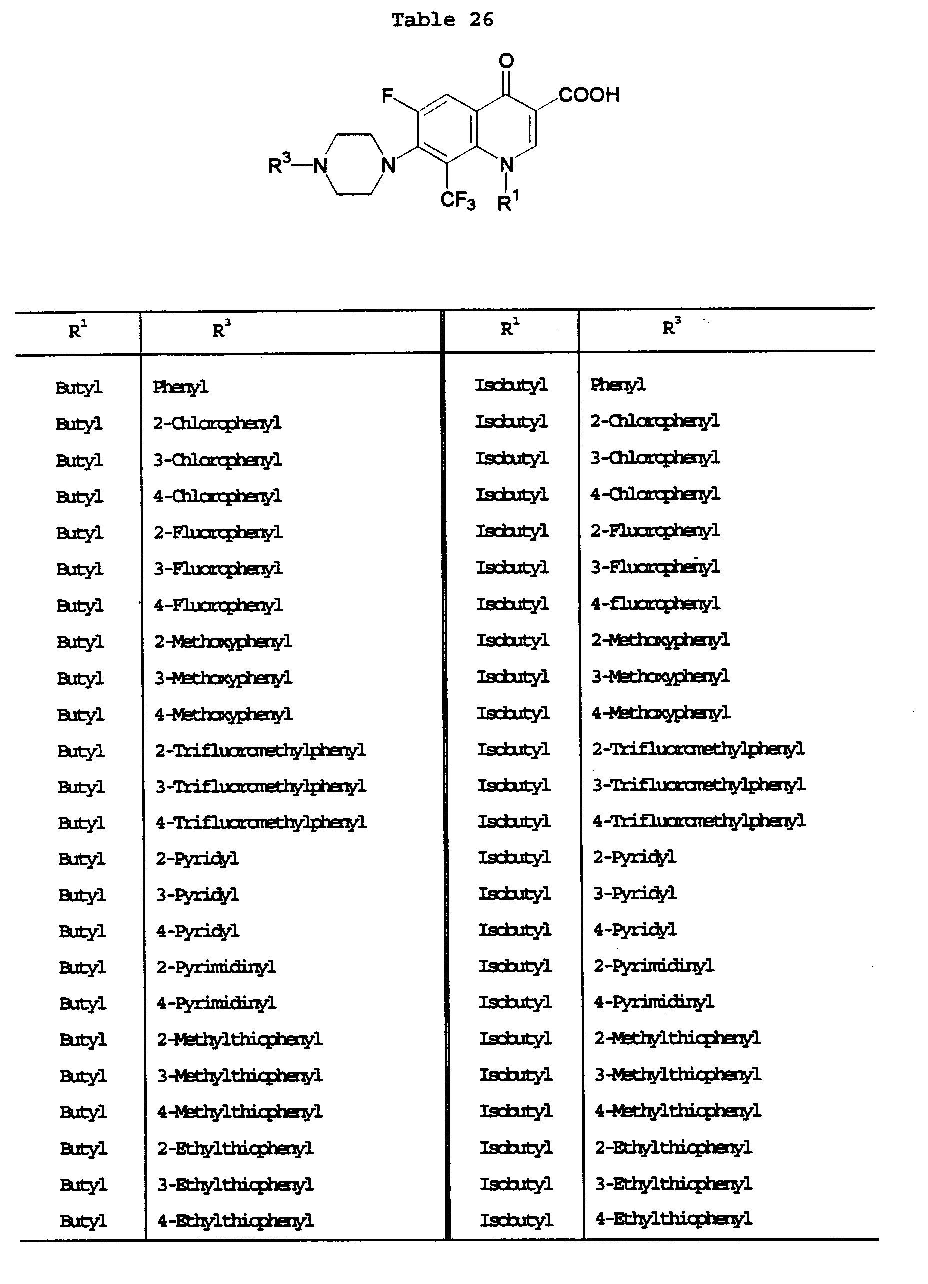

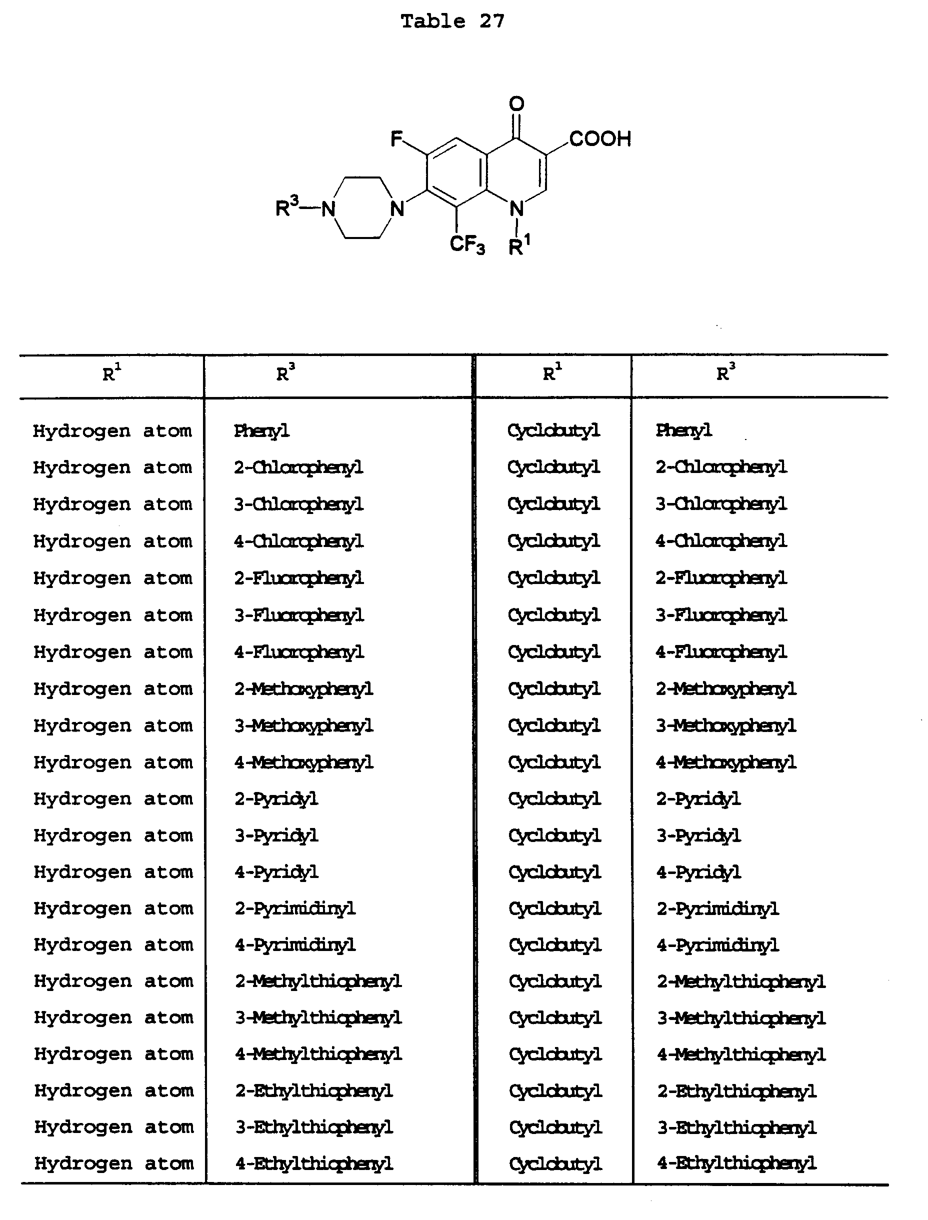

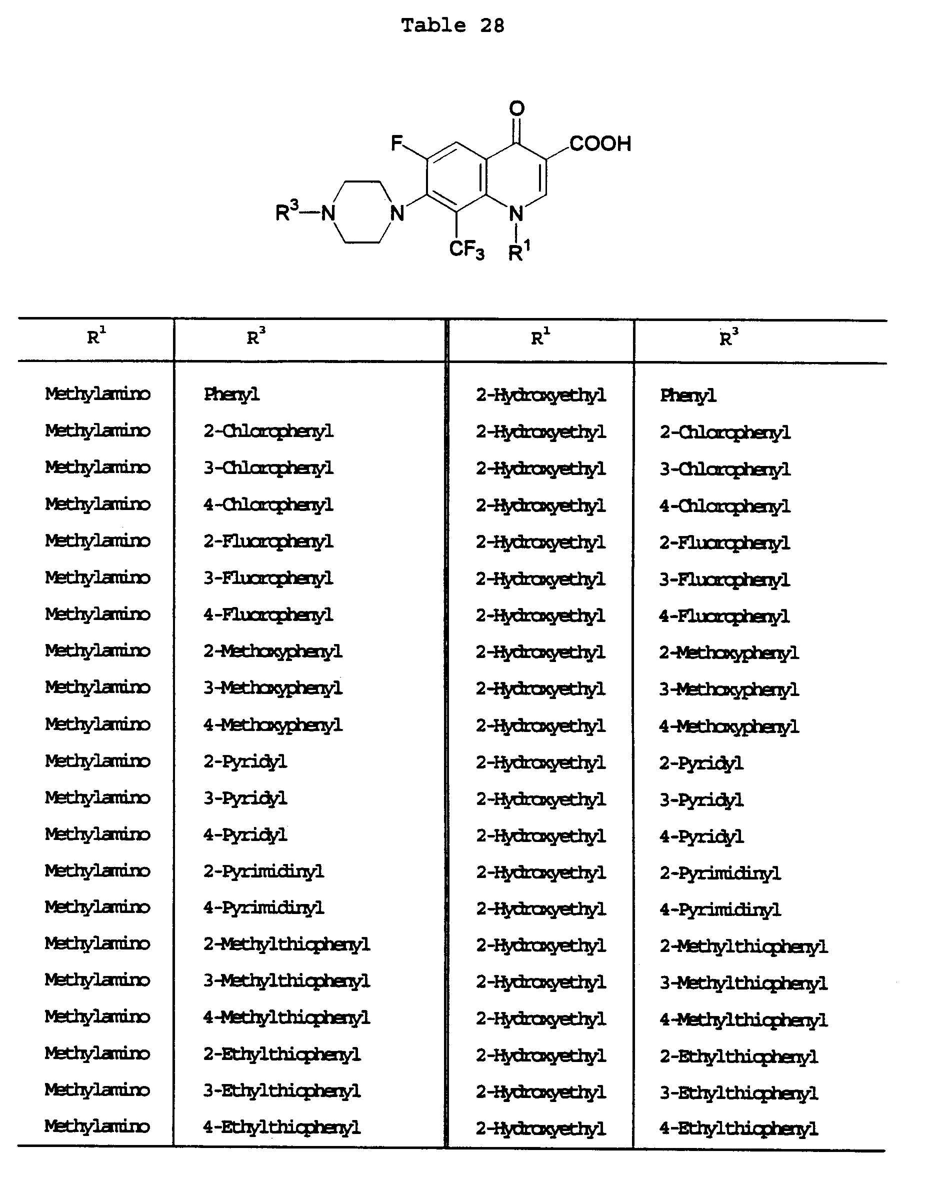

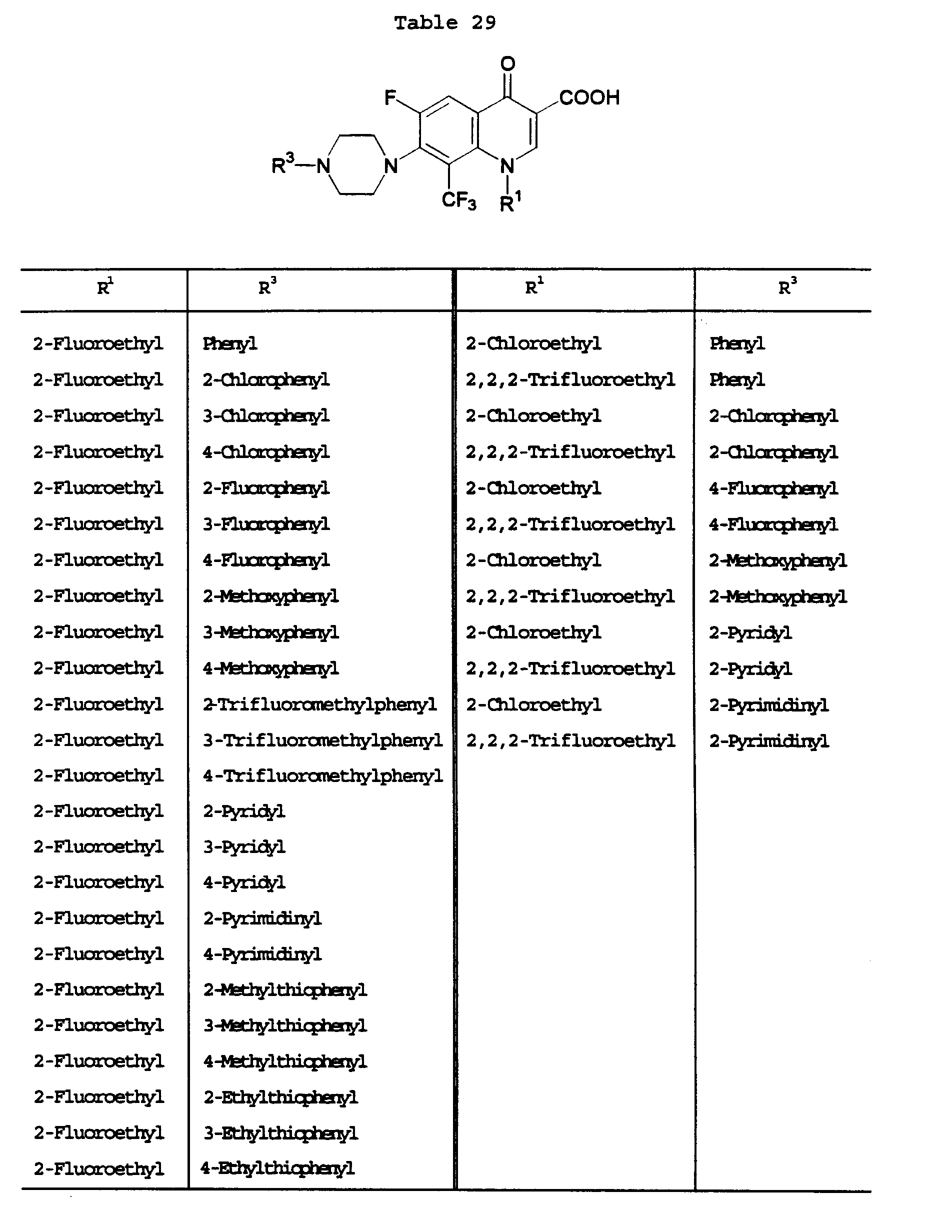

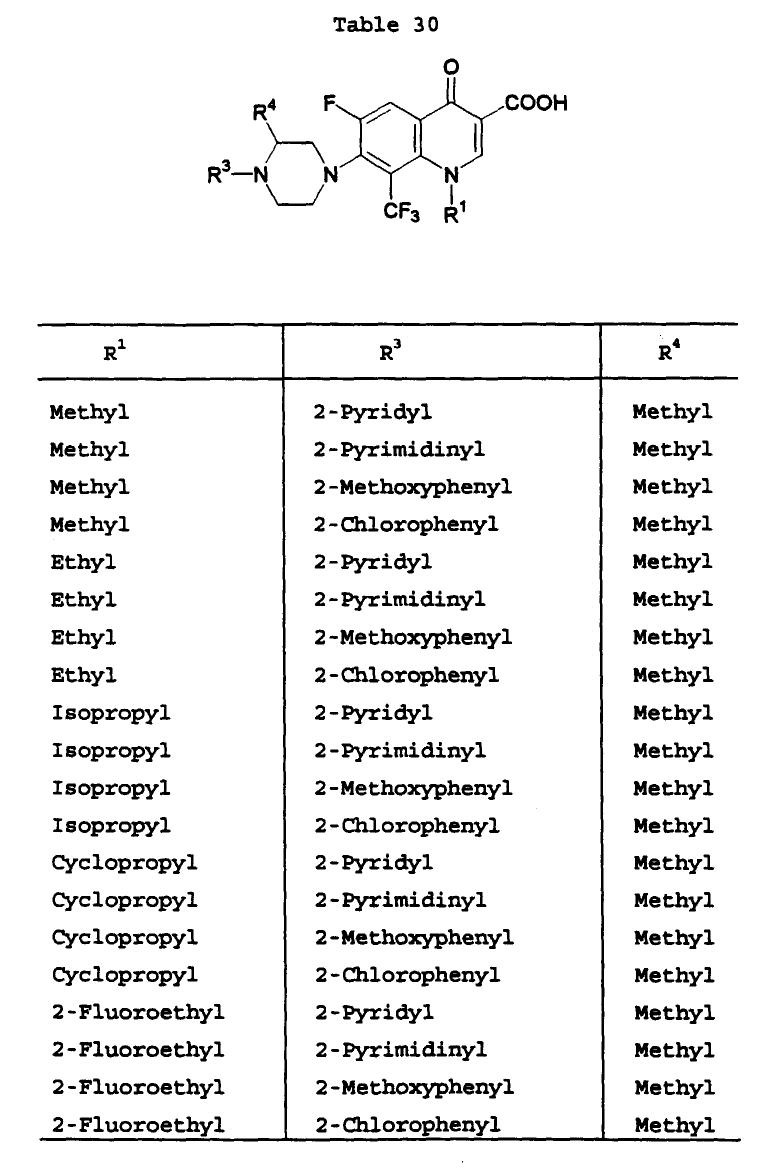

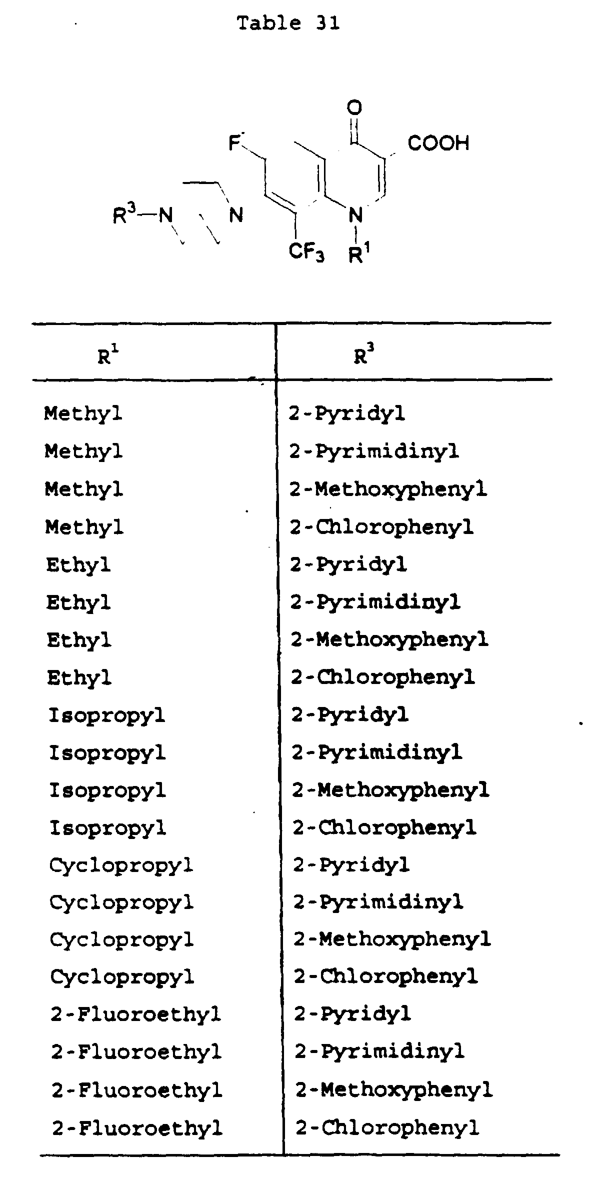

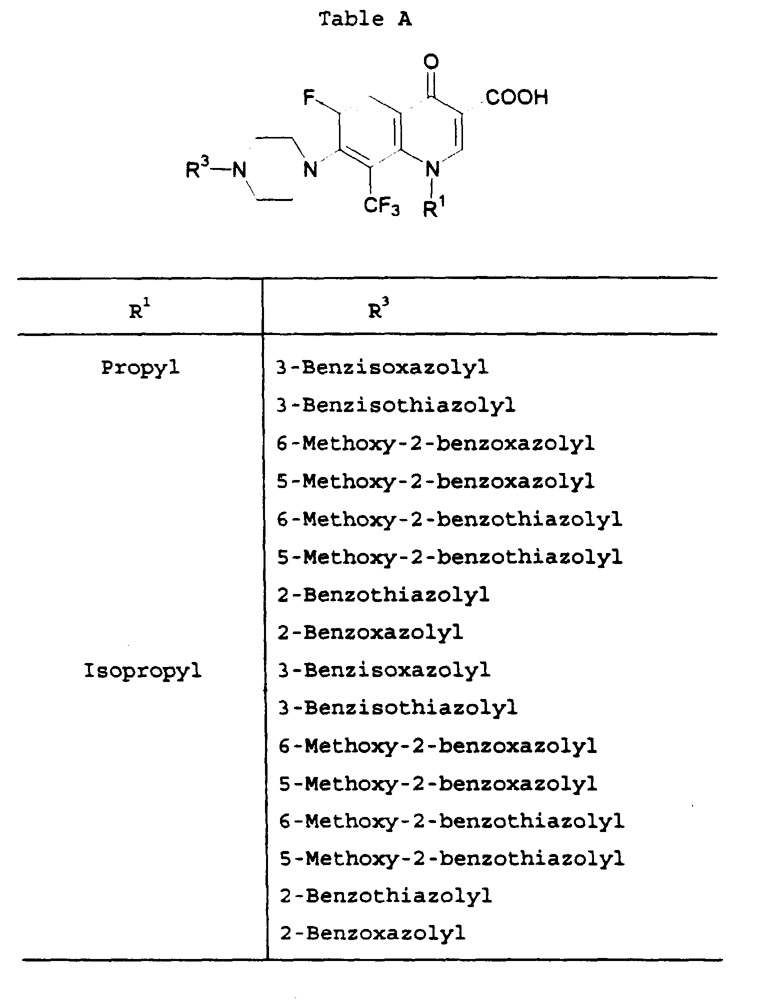

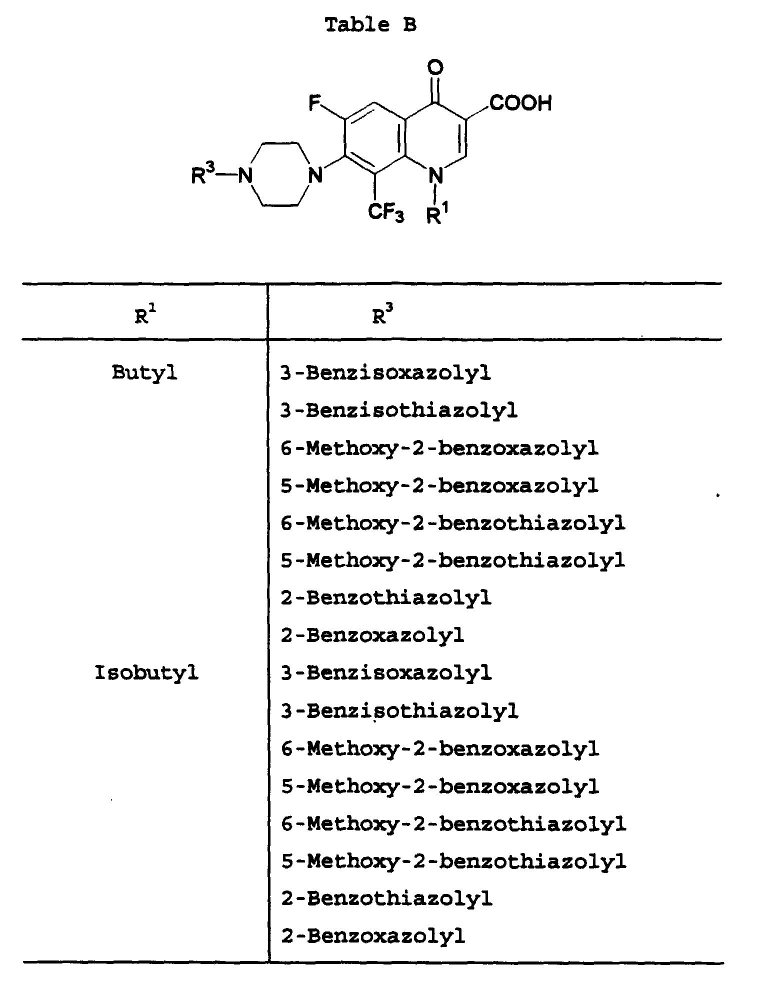

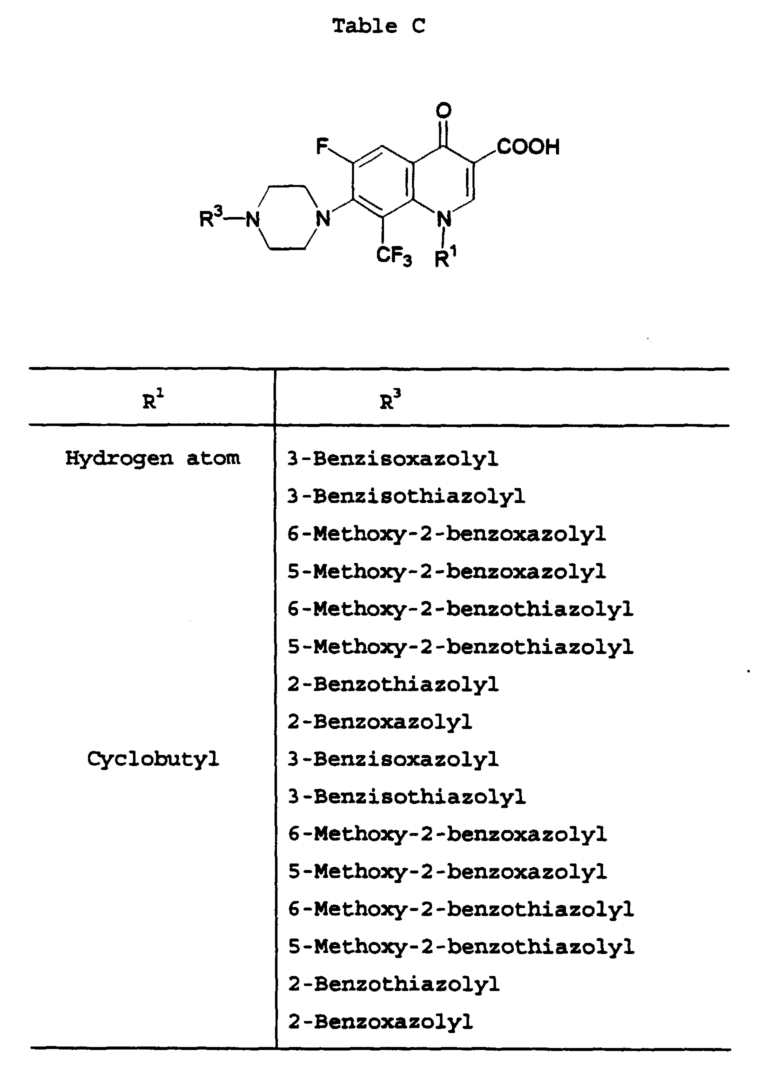

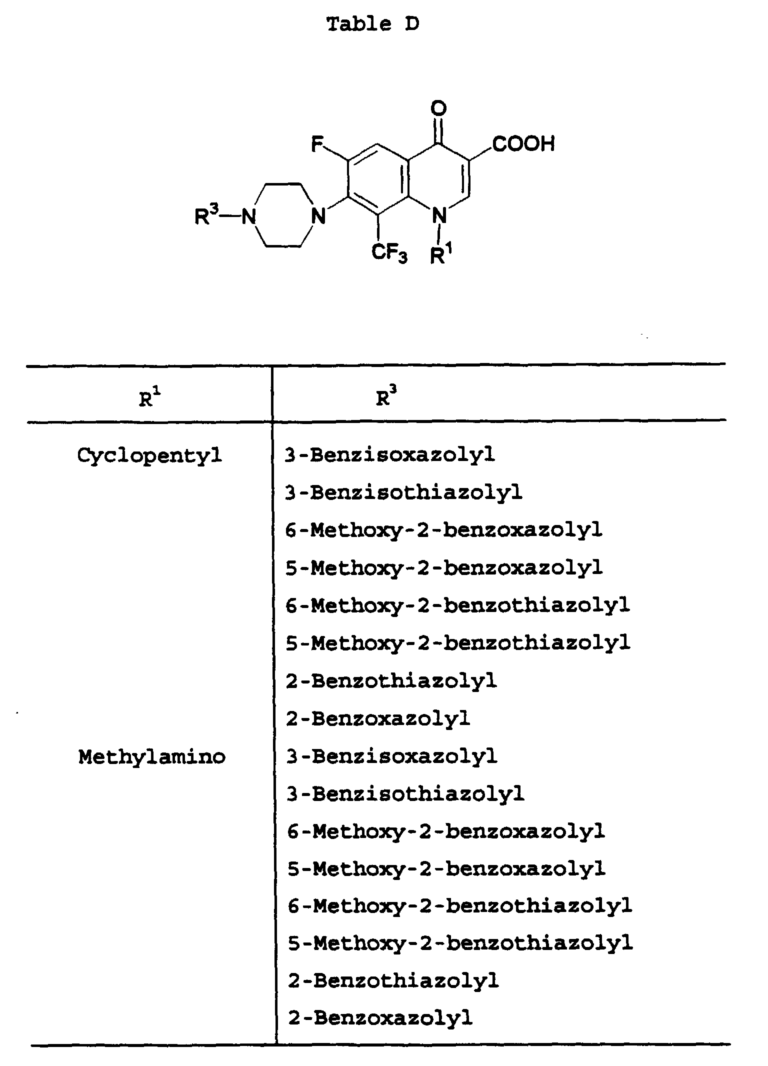

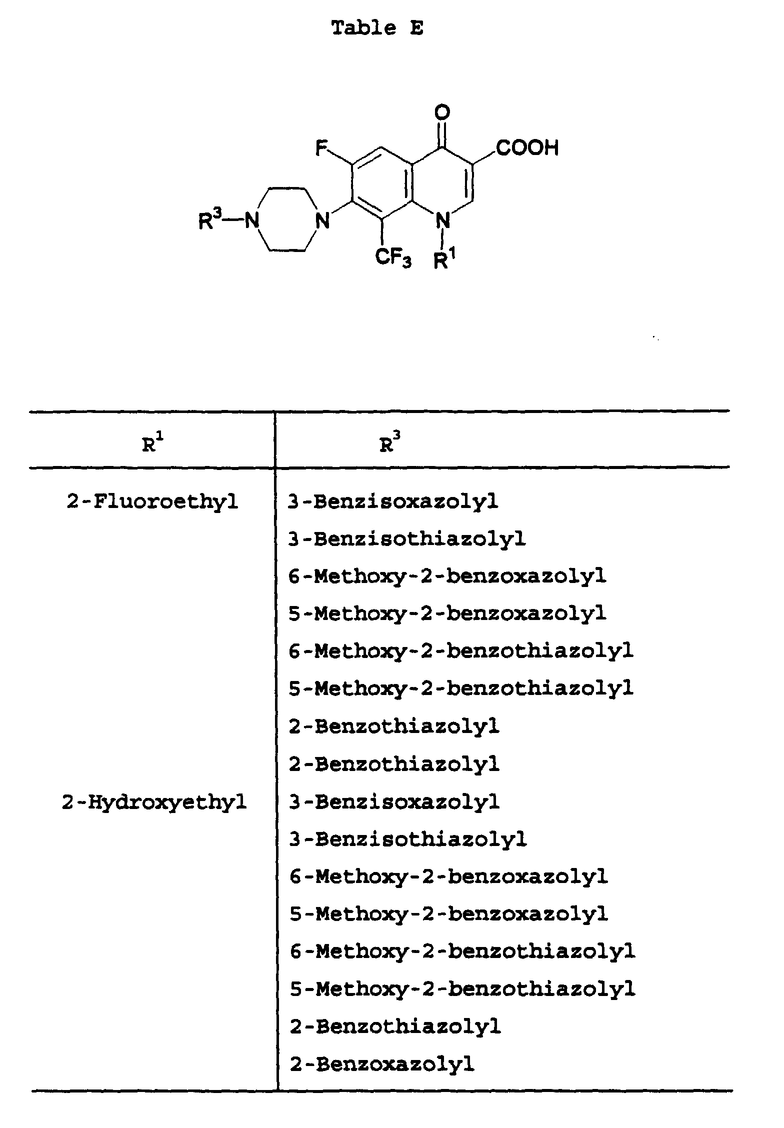

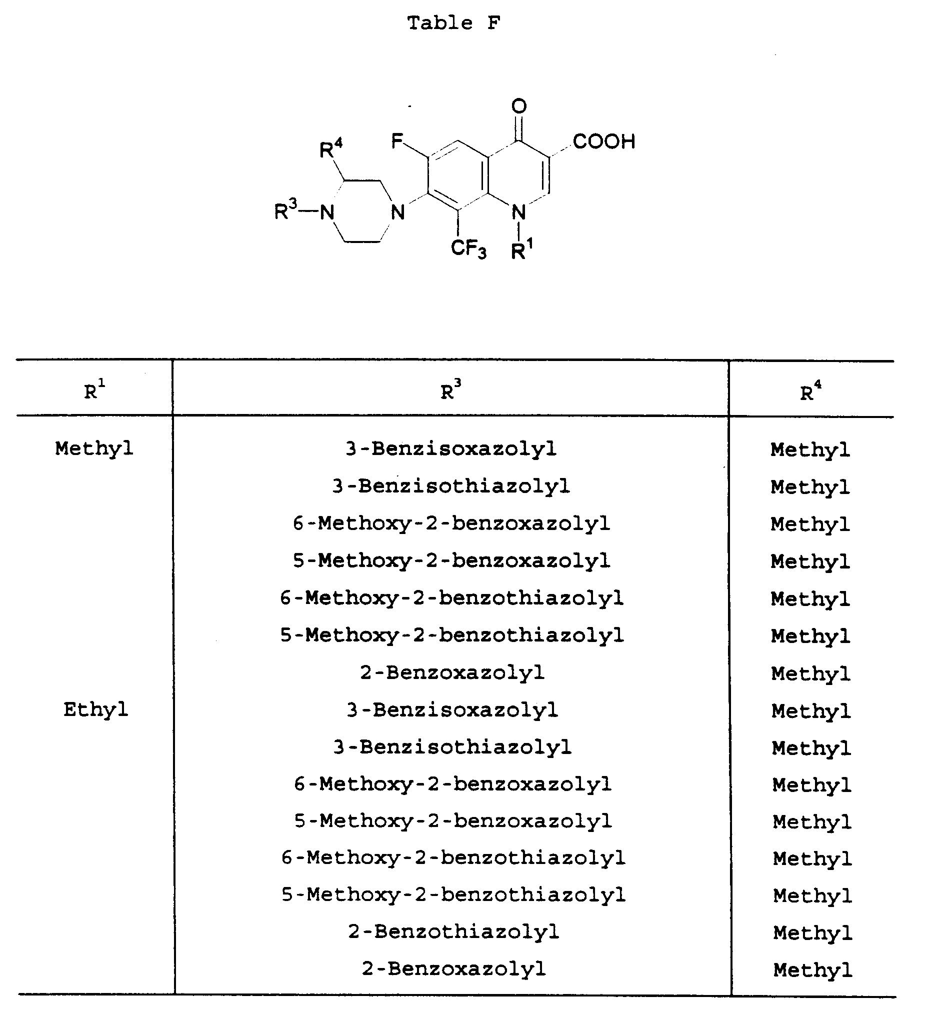

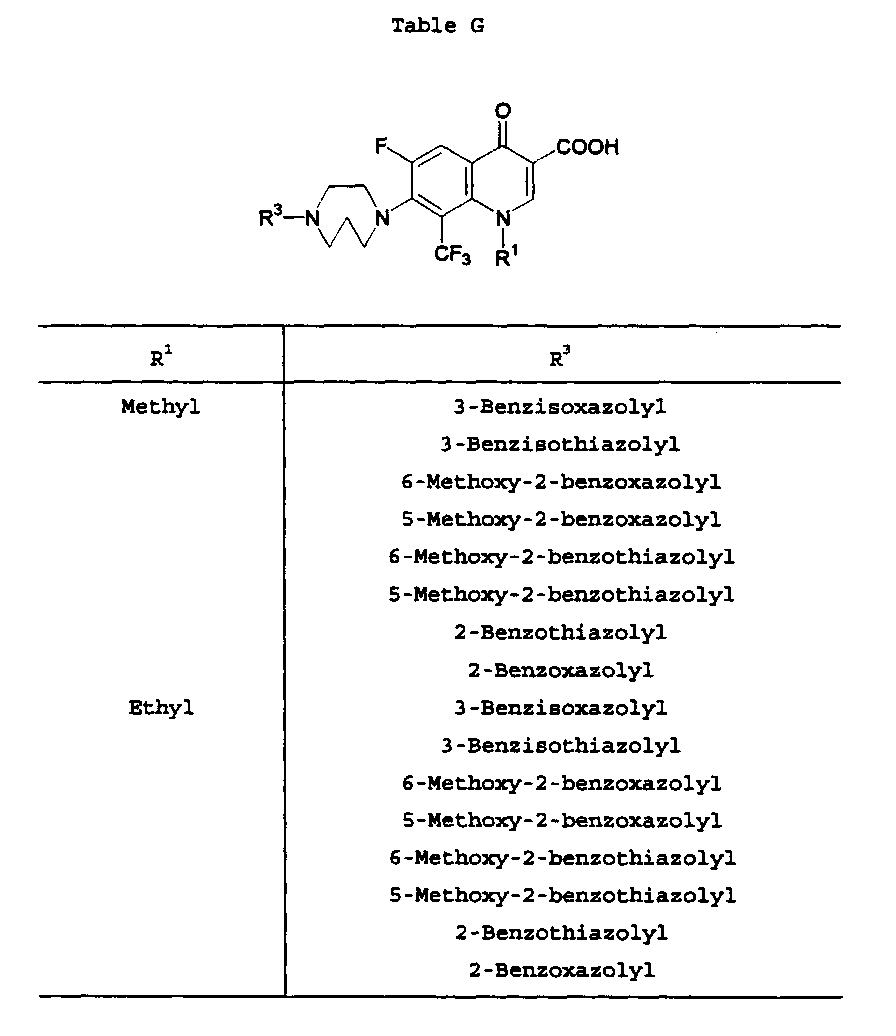

The compounds of the formula (Ia), (Ib) or (Ic) as the active ingredient of the present invention preferably include :
1-cyclopropyl-6-fluoro-8-trifluoromethyl-1,4-dihydro-4-oxo-7-[4-(2-pyrimidinyl)piperazin-1-yl]quinoline-3-carboxylic acid,
1-cyclopropyl-6-fluoro-8-trifluoromethyl-1,4-dihydro-4-oxo-7-[4-(2-pyridyl)piperazin-1-yl]quinoline-3-carboxylic acid,
1-cyclopropyl-6-fluoro-8-trifluoromethyl-1,4-dihydro-4-oxo-7-[4-(2-methoxyphenyl)piperazin-1-yl]quinoline-3-carboxylic acid,
1-cyclopropyl-6-fluoro-8-trifluoromethyl-1,4-dihydro-4-oxo-7-(4-phenylpiperazin-1-yl)quinoline-3-carboxylic acid,
1-cyclopropyl-6-fluoro-8-trifluoromethyl-1,4-dihydro-4-oxo-7-[4-(3-chlorophenyl)piperazin-1-yl]quinoline-3-carboxylic acid,
1-cyclopropyl-6-fluoro-8-trifluoromethyl-1,4-dihydro-4-oxo-7-[4-(4-fluorophenyl)piperazin-1-yl]quinoline-3-carboxylic acid,
1-cyclopropyl-6-fluoro-8-trifluoromethyl-1,4-dihydro-4-oxo-7-[4-(3-trifluoromethylphenyl)piperazin-1-yl]quinoline-3-carboxylic acid,
6-fluoro-8-trifluoromethyl-1,4-dihydro-4-oxo-1-methyl-7-[4-(2-pyrimidinyl)piperazin-1-yl]quinoline-3-carboxylic acid,
6-fluoro-8-trifluoromethyl-1,4-dihydro-4-oxo-1-methyl-7-[4-(2-pyridyl)piperazin-1-yl]quinoline-3-carboxylic acid,
1-ethyl-6-fluoro-8-trifluoromethyl-1,4-dihydro-4-oxo-7-[4-(2-pyrimidinyl)piperazin-1-yl]quinoline-3-carboxylic acid,
1-ethyl-6-fluoro-8-trifluoromethyl-1,4-dihydro-4-oxo-7-[4-(2-pyridyl)piperazin-1-yl]quinoline-3-carboxylic acid,
1-ethyl-6-fluoro-8-trifluoromethyl-1,4-dihydro-4-oxo-7-[4-(2-methoxyphenyl)piperazin-1-yl]quinoline-3-carboxylic acid,
6-fluoro-8-trifluoromethyl-1,4-dihydro-4-oxo-1-isopropyl-7-[4-(2-pyrimidinyl)piperazin-1-yl]quinoline-3-carboxylic acid,
6-fluoro-8-trifluoromethyl-1,4-dihydro-4-oxo-1-isopropyl-7-[4-(2-pyridyl)piperazin-1-yl]quinoline-3-carboxylic acid,
6-fluoro-8-trifluoromethyl-1,4-dihydro-4-oxo-1-isopropyl-7-[4-(2-methoxyphenyl)piperazin-1-yl]quinoline-3-carboxylic acid,
6-fluoro-1-(2-fluoroethyl)-8-trifluoromethyl-1,4-dihydro-4-oxo-7-[4-(2-pyrimidinyl)piperazin-1-yl]quinoline-3-carboxylic acid,
6-fluoro-1-(2-fluoroethyl)-8-trifluoromethyl-1,4-dihydro-4-oxo-7-[4-(2-pyridyl)piperazin-1-yl]quinoline-3-carboxylic acid,
6-fluoro-1-(2-fluoroethyl)-8-trifluoromethyl-1,4-dihydro-4-oxo-7-[4-(2-methoxyphenyl)piperazin-1-yl]quinoline-3-carboxylic acid,
6-fluoro-8-trifluoromethyl-1,4-dihydro-4-oxo-1-methyl-7-[4-(2-methoxyphenyl)piperazin-1-yl]quinoline-3-carboxylic acid,
6-fluoro-8-trifluoromethyl-1,4-dihydro-4-oxo-1-methyl-7-[4-(2-chlorophenyl)piperazin-1-yl]quinoline-3-carboxylic acid,
6-fluoro-8-trifluoromethyl-1,4-dihydro-4-oxo-1-methyl-7-(4-phenylpiperazin-1-yl)quinoline-3-carboxylic acid,
6-fluoro-8-trifluoromethyl-1,4-dihydro-4-oxo-1-methyl-7-[4-(4-fluorophenyl)piperazin-1-yl]quinoline-3-carboxylic acid,
6-fluoro-8-trifluoromethyl-1,4-dihydro-4-oxo-1-methyl-7-[4-(2-thiazolyl)piperazin-1-yl]quinoline-3-carboxylic acid,
6-fluoro-8-trifluoromethyl-1,4-dihydro-4-oxo-1-methyl-7-[4-(2-methylthiophenyl)piperazin-1-yl]quinoline-3-carboxylic acid,
1-ethyl-6-fluoro-8-trifluoromethyl-1,4-dihydro-4-oxo-7-[4-(4-methoxyphenyl)piperazin-1-yl]quinoline-3-carboxylic acid,
1-ethyl-6-fluoro-8-trifluoromethyl-1,4-dihydro-4-oxo-7-[4-(4-chlorophenyl)piperazin-1-yl]quinoline-3-carboxylic acid,
1-ethyl-6-fluoro-8-trifluoromethyl-1,4-dihydro-4-oxo-7-[4-(5-chloro-2-pyrimidinyl)piperazin-1-yl]quinoline-3-carboxylic acid,
1-ethyl-6-fluoro-8-trifluoromethyl-1,4-dihydro-4-oxo-7-[4-(2-fluorophenyl)piperazin-1-yl]quinoline-3-carboxylic acid,
1-ethyl-6-fluoro-8-trifluoromethyl-1,4-dihydro-4-oxo-7-[4-(3-methoxyphenyl)piperazin-1-yl]quinoline-3-carboxylic acid,
1-cyclopropyl-6-fluoro-8-trifluoromethyl-1,4-dihydro-4-oxo-7-[4-(2-pyridyl)piperazin-1-yl]quinoline-3-carboxylic acid 2-morpholinoethyl ester,
6-fluoro-8-trifluoromethyl-1,4-dihydro-4-oxo-1-methyl-7-[3-methyl-4-(2-pyrimidinyl)piperazin-1-yl]quinoline-3-carboxylic acid,
6-fluoro-8-trifluoromethyl-1,4-dihydro-4-oxo-1-methyl-7-[4-(2-pyrimidinyl)homopiperazin-1-yl]quinoline-3-carboxylic acid,
6-fluoro-8-trifluoromethyl-1,4-dihydro-4-oxo-1-methyl-7-[4-(2-benzothiazolyl)piperazin-1-yl]quinoline-3-carboxylic acid,
6-fluoro-8-trifluoromethyl-1,4-dihydro-4-oxo-1-methyl-7-[4-(2-benzoxazolyl)piperazin-1-yl]quinoline-3-carboxylic acid,
6-fluoro-8-difluoromethoxy-1,4-dihydro-4-oxo-1-ethyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(6-methoxy-2-benzothiazolyl)piperazin-1-yl]quinoline-3-carboxylic acid,
6-fluoro-8-difluoromethoxy-1,4-dihydro-4-oxo-1-(2-hydroxyethyl)-7-[4-(2-benzothiazolyl)piperazin-1-yl]quinoline-3-carboxylic acid, and
6-fluoro-8-difluoromethoxy-1,4-dihydro-4-oxo-1-methylamino-7-[4-(2-benzoxazolyl)piperazin-1-yl]quinoline-3-carboxylic acid,
   more preferably :
1-cyclopropyl-6-fluoro-8-trifluoromethyl-1,4-dihydro-4-oxo-7-[4-(2-pyridyl)piperazin-1-yl]quinoline-3-carboxylic acid,
6-fluoro-8-trifluoromethyl-1,4-dihydro-4-oxo-1-methyl-7-[4-(2-pyrimidinyl)piperazin-1-yl]quinoline-3-carboxylic acid,
6-fluoro-8-trifluoromethyl-1,4-dihydro-4-oxo-1-methyl-7-[4-(2-pyridyl)piperazin-1-yl]quinoline-3-carboxylic acid,
1-ethyl-6-fluoro-8-trifluoromethyl-1,4-dihydro-4-oxo-7-[4-(2-pyridyl)piperazin-1-yl]quinoline-3-carboxylic acid,
6-fluoro-8-trifluoromethyl-1,4-dihydro-4-oxo-1-methyl-7-[4-(4-fluorophenyl)piperazin-1-yl]quinoline-3-carboxylic acid,
6-fluoro-8-trifluoromethyl-1,4-dihydro-4-oxo-1-methyl-7-[4-(2-thiazolyl)piperazin-1-yl]quinoline-3-carboxylic acid,
6-fluoro-8-trifluoromethyl-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-ethyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(6-methoxy-2-benzothiazolyl)piperazin-1-yl]quinoline-3-carboxylic acid,
6-fluoro-8-difluoromethoxy-1,4-dihydro-4-oxo-1-(2-hydroxyethyl)-7-[4-(2-benzothiazolyl)piperazin-1-yl]quinoline-3-carboxylic acid, and
6-fluoro-8-difluoromethoxy-1,4-dihydro-4-oxo-1-methylamino-7-[4-(2-benzoxazolyl)piperazin-1-yl]quinoline-3-carboxylic acid,
   still more preferably :
1-cyclopropyl-6-fluoro-8-trifluoromethyl-1,4-dihydro-4-oxo-7-[4-(2-pyridyl)piperazin-1-yl]quinoline-3-carboxylic acid,
6-fluoro-8-trifluoromethyl-1,4-dihydro-4-oxo-1-methyl-7-[4-(2-pyrimidinyl)piperazin-1-yl]quinoline-3-carboxylic acid,
6-fluoro-8-trifluoromethyl-1,4-dihydro-4-oxo-1-methyl-7-[4-(2-pyridyl)piperazin-1-yl]quinoline-3-carboxylic acid,
6-fluoro-8-trifluoromethyl-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-ethyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-
(2-benzothiazolyl)piperazin-1-yl]quinoline-3-carboxylic acid, 6-fluoro-8-difluoromethoxy-1,4-dihydro-4-oxo-1-methyl-7-[4-(6-methoxy-2-benzothiazolyl)piperazin-1-yl]quinoline-3-carboxylic acid,
6-fluoro-8-difluoromethoxy-1,4-dihydro-4-oxo-1-(2-hydroxyethyl)-7-[4-(2-benzothiazolyl)piperazin-1-yl]quinoline-3-carboxylic acid, and
6-fluoro-8-difluoromethoxy-1,4-dihydro-4-oxo-1-methylamino-7-[4-(2-benzoxazolyl)piperazin-1-yl]quinoline-3-carboxylic acid, and
   most preferably :
6-fluoro-8-trifluoromethyl-1,4-dihydro-4-oxo-1-methyl-7-[4-(2-pyrimidinyl)piperazin-1-yl]quinoline-3-carboxylic acid,
6-fluoro-8-difluoromethoxy-1,4-dihydro-4-oxo-1-ethyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(6-methoxy-2-benzothiazolyl)piperazin-1-yl]quinoline-3-carboxylic acid,
6-fluoro-8-difluoromethoxy-1,4-dihydro-4-oxo-1-(2-hydroxyethyl)-7-[4-(2-benzothiazolyl)piperazin-1-yl]quinoline-3-carboxylic acid, and
6-fluoro-8-difluoromethoxy-1,4-dihydro-4-oxo-1-methylamino-7-[4-(2-benzoxazolyl)piperazin-1-yl]quinoline-3-carboxylic acid.

The compounds of the formulae (Ia), (Ib) and (Ic) as the active ingredients of the present invention can be prepared according to the method described in EP-A-572,259 (JP-A-H6-116241) and/or the method described in WO/02512 and analogous methods thereof.

The reverse transcriptase inhibitor which is an active ingredient of the present invention refers to a drug that inhibits reverse transcriptase encoded by HIV gene, typical examples of which include nucleoside type compounds and dipyridodiazepin type compounds, preferably, ZDV (zidovudine), DDI (didanosine), DDC (zalcitabine), d4T (stavudine), 3TC (lamivudine), FTC (524W91) and nevirapine, more preferably ZDV, DDI and nevirapine, and most preferably ZDV.

Structural formulae of the typical examples of reverse transcriptase inhibitors are shown below.

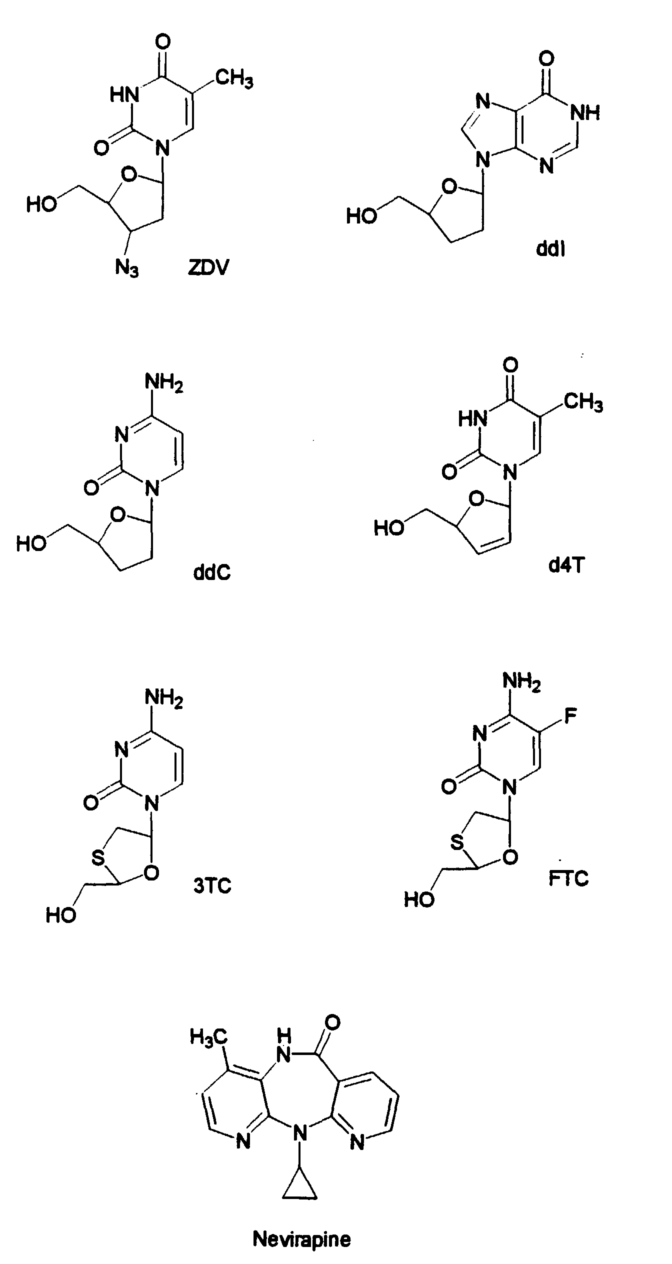

ZDV (zidovudine: 1-(3-azido-2,3-dideoxy-β-D-erythropentofuranosyl)-5-methyl-2(1H)-pyrimidone) is described in EP-A-287,215.

DDI (didanosine: 2',3'-dideoxyinosine) is described in USP-4,835,104.

DDC (zalcitabine:2',3,-dideoxycytidine) is described in EP-A-285,884.

d4T (stavudine: 2',3'-dideoxy-2'3'-dehydrothymidine) is described in EP-A-334,368.

3TC (lamivudine: 4-amino-1-(2-hydroxymethyl-1,3-oxathioran-5-yl)-(1H)-pyrimidin-2-one) is described in WO 91/17159.

FTC (524W91: 4-amino-5-fluoro-1-(2-hydroxmethyl-1,3-oxathioran)-(1H)-pyrimidin-2-one) is described in EP-A-526,253.

Nevirapine (11-cyclopropyl-5,11-dihydro-4-methyl-6H-dipyrido[3,2-b:2',3'-e] [1,4] diazepin-6-one) is described in EP-A-429,987.

In addition, the reverse transcriptase inhibitor of the present invention also includes hydrates of the above-mentioned compounds.

The HIV protease inhibitor which is an active ingredient of the present invention refers to a drug that inhibits HIV protease, typical examples of which include dipeptide type compounds and tripeptide type compounds, preferably VX-478, KNI-272, AG-1343, saquinavir, ritonavir, indinavir and compound A, and more preferably AG-1343, saquinavir, ritonavir and indinavir.

Structural formulae of some typical examples of HIV protease inhibitors are shown below.

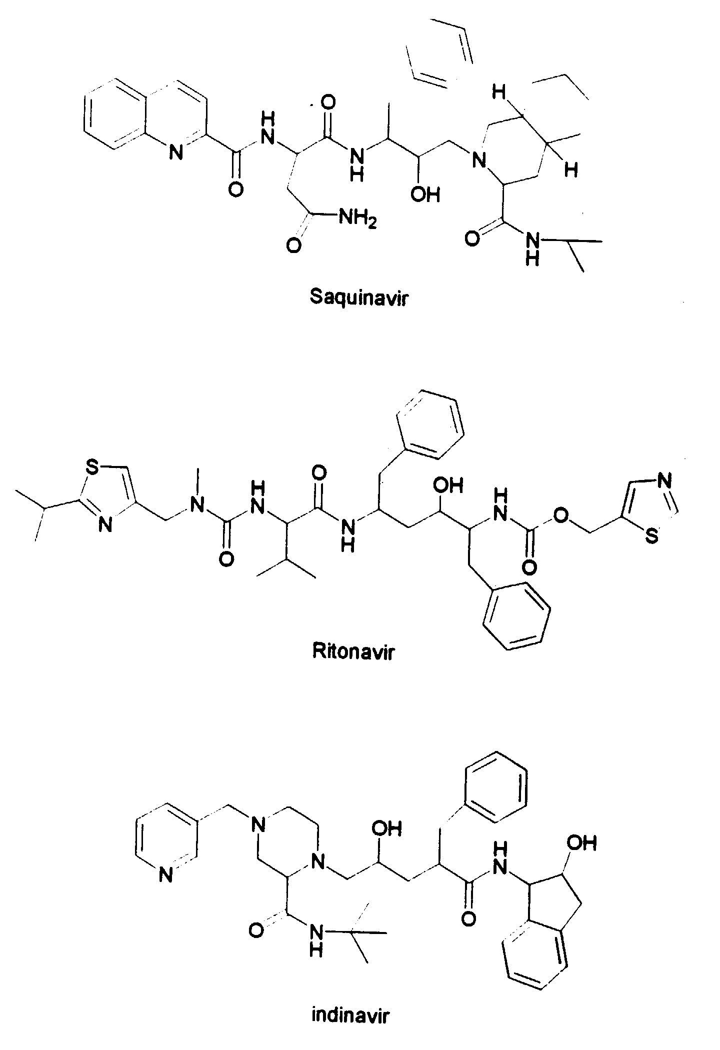

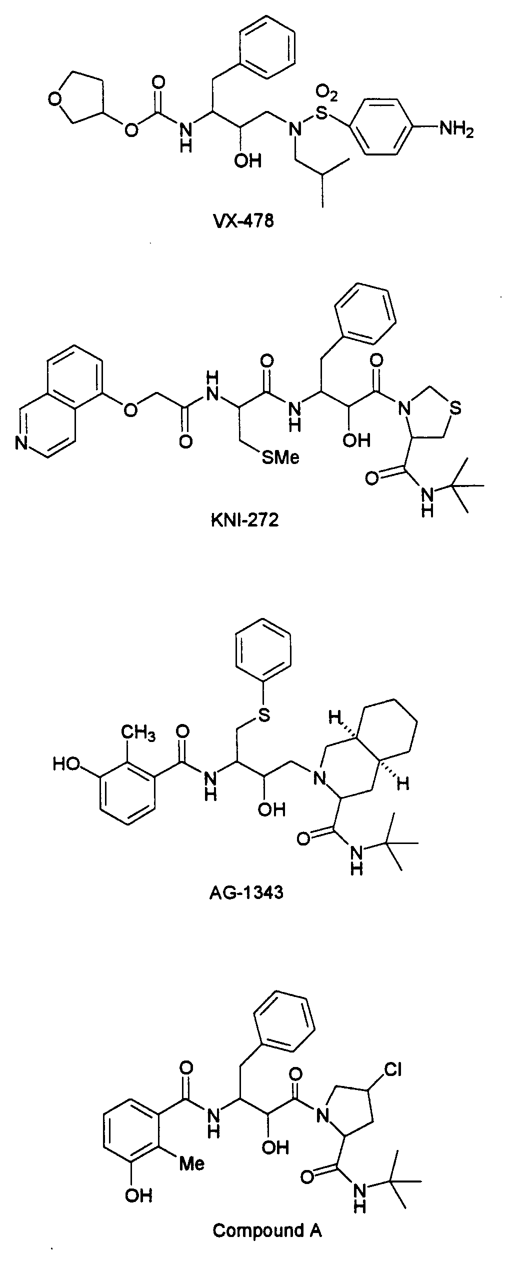

VX-478 ([2R-hydroxy-3-[[(4-aminophenyl)sulfonyl](2-methylpropyl)amino]-1S-(phenylmethyl)propyl]carbamic acid 3S-tetrahydrofuranyl ester) is described in WO 94/05639, WO 95/33464 and so forth.

KNI-272 ([4R-[3[2S*,3*S(R*)], 4R*]]-N-(1,1-dimethylethyl)-3-[2-hydroxy-3-[[2-[[5-isoquinolinyloxy) acetyl]amino]-3-(methylthio)-1-oxopropyl] amino]-1-oxo-4-phenylbutyl-4-carboxamide) is described in EP-A-574,135.

AG-1343([3S-(3R*, 4aR*, 8aR*, 2'S*, 3'S*)]-2-[2'-hydroxy-3'-phenylthiomethyl]-4'-aza-5'-oxo-5'-(2''-methyl-3''-hydroxyphenyl)pentyl]decahydroisoquinoline-3-N-t-butylcarboxamide) is described in, for example, WO 95/09843.

Saquinavir (N-t-butyl-decahydro-2-[2(R)-hydroxy-4-phenyl-3(S)-[[N-(2-quinolylcarbonyl)-L-asparagyl]amino]butyl]-(4aS,8aS)-isoquinoline-3(S)-carboxamide) is described in, for example, EP-A-432,695.

Ritonavir ((2S,3S,5S)-5-(N-(N-((N-methyl-N-((2-isopropyl-4-thiazolyl)methyl)amino) carbonyl)valinyl)amino)-2-(N-((5-thiazolyl)methoxycarbonyl) amino)-1,6-diphenyl-3-hydroxyhexane) is described in, for example, WO 94/14436.

Indinavir (N-(2(R)-hydroxy-1(S)-indanyl)-2(R)-phenylmethyl-4(S)-hydroxy-5-(1-(4-(3-pyridylmethyl)-2(S)-N'-(t-butylcarboxamido)-piperazinyl))-pentaneamide) is described in, for example, in EP-A-541,168.

Compound A ((2S,3S)-3-(3-hydroxy-2-methylbenzoyl)amino-2-hydroxy-4-phenylbutanoyl-[4(S)-chloro]-L-proline tert-butylamide) is described in WO 96/28423.

In addition, the HIV protease inhibitor of the present invention also includes hydrates of the above-mentioned compounds.

In the present invention, while one kind or two or more kinds of a quinolone carboxylic acid having anti-HIV activity is used, one kind or two or more kinds of a reverse transcriptase inhibitor is used, and one kind or two or more kinds of an HIV protease inhibitor is used, preferably,
1) one kind of a quinolone carboxylic acid having anti-HIV activity and one kind of a reverse transcriptase inhibitor,
2) one kind of a quinolone carboxylic acid having anti-HIV activity and one kind of an HIV protease inhibitor, and
3) one kind of a quinolone carboxylic acid having anti-HIV activity, one kind of a reverse transcriptase inhibitor and one kind of an HIV protease inhibitor are used combinedly.

The AIDS therapeutic agent or preventive agent of the present invention is, preferably,
1) the therapeutic or preventive agent wherein the quinolone carboxylic acid having anti-HIV activity as an active ingredient is a compound of formula (Ia), (Ib) or (Ic),
2) the therapeutic or preventive agent wherein the quinolone carboxylic acid having anti-HIV activity as an active ingredient is a compound of formula (Ia),
3) the therapeutic or preventive agent wherein the quinolone carboxylic acid having anti-HIV activity as an active ingredient is a compound of formula (Ia), wherein
   X is a fluorine atom,
   Y is a hydrogen or fluorine atom, or an amino, methyl or ethyl group,
   Z is an optionally protected carboxyl group,
   Q is a group of the formula (d), and R² of the formula (d) is a methoxy, difluoromethoxy or trifluoromethyl group,
   R¹ is a hydrogen atom; or a methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group, and
   R is a 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group.
4) the therapeutic or preventive agent in which the quinolone carboxylic acid having anti-HIV activity as the active ingredient is a compound of formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a methyl, ethyl, 2-hydroxyethyl, 2-fluoroethyl, cyclopropyl or methylamino group,
   R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group,
5) the therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid,
6) the therapeutic or preventive agent in which the reverse transcriptase inhibitor as the active ingredient is ZDV (zidovudine), DDI (didanosine), DDC (zalcitabine), d4T (stavudine), 3TC (lamivudine), FTC (524W91) or Nevirapine,
7) the therapeutic or preventive agent in which the reverse transcriptase inhibitor as the active ingredient is ZDV, DDI and Nevirapine,
8) the therapeutic or preventive agent in which the reverse transcriptase inhibitor as the active ingredient is ZDV,
9) the therapeutic or preventive agent in which the HIV protease inhibitor as the active ingredient is VX-478, KNI-272, AG-1343, Saquinavir, Ritonavir, indinavir and Compound A, and
10) the therapeutic or preventive agent in which the HIV protease inhibitor as the active ingredient is AG-1343, Saquinavir, Ritonavir or indinavir.
   Further, the quinolone carboxylic acid having anti-HIV activity as the active ingredient is selected from 1) to 5), the reverse transcriptase inhibitor as the active ingredient is selected from 6) to 8), the HIV protease inhibitor as the active ingredient is selected from 9) and 10), and the therapeutic agent or the preventive agent obtained by combining those are preferable and, for example include the following therapeutic agents or preventive agents,
11) the therapeutic or preventive agent in which the quinolone carboxylic acid having anti-HIV activity as the active ingredient is the above-mentioned formula (Ia), (Ib) or (Ic) and the reverse transcriptase inhibitor as the active ingredient is ZDV, DDI, DDC, d4T, 3TC, FTC or Nevirapine,
12) a therapeutic or preventive agent in which the quinolone carboxylic acid having anti-HIV activity as the active ingredient is the above-mentioned formula (Ia) and the reverse transcriptase inhibitor as the active ingredient is ZDV, DDI, DDC, d4T, 3TC, FTC or Nevirapine.
13) a therapeutic or preventive agent in which the quinolone carboxylic acid having anti-HIV activity as the active ingredient is a compound represented by the formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a hydrogen atom; or a methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group,
   R is a 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group, and the reverse transcriptase inhibitor as the active ingredient is ZDV, DDI and Nevirapine,
14) a therapeutic or preventive agent in which the quinolone carboxylic acid having anti-HIV activity is a compound of the formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a methyl, ethyl, 2-hydroxyethyl, 2-fluoroethyl, cyclopropyl or methylamino group,
   R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group, and the reverse transcriptase inhibitor as the active ingredient is ZDV, DDI and Nevirapine.
15) a therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the reverse transcriptase inhibitor as the active ingredient is ZDV, DDI, DDC, d4T, 3TC, FTC or Nevirapine.
16) a therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the reverse transcriptase inhibitor as the active ingredient is ZDV, DDI and Nevirapine.
17) a therapeutic or preventive agent in which the quinolone carboxylic acid having anti-HIV activity as the active ingredient is,
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoxymethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the reverse transcriptase inhibitor as the active ingredient is ZDV.
18) a therapeutic or preventive agent in which the quinolone carboxylic having anti-HIV activity as the active ingredient is the above-mentioned formula (Ia), (Ib) or (Ic), and the HIV protease inhibitor as the active ingredient is VX-478, KNI-272, AG-1343, Saquinavir, Ritonavir, indinavir and Compound A,
19) a therapeutic or preventive agent in which the quinolone carboxylic having anti-HIV activity as the active ingredient is the above-mentioned formula (Ia), and the HIV protease inhibitor as the active ingredient is VX-478, KNI-272, AG-1343, Saquinavir, Ritonavir, indinavir and Compound A,
20) a therapeutic or preventive agent in which the quinolone carboxylic acid having anti-HIV activity as the active ingredient is a compound of the formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a hydrogen atom; or a methyl, ethyl, propyl, isopropyl; 2-hydroxyethyl; carboxyethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group,
   R is a 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group,
      and the HIV protease inhibitor as the active ingredient is VX-478, KNI-272, AG-1343, Saquinavir, Ritonavir, indinavir and Compound A,
21) a therapeutic or preventive agent in which the quinolone carboxylic acid having anti-HIV activity as the active ingredient is a compound represented by the formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a methyl, ethyl, 2-hydroxyethyl, 2-fluoroethyl, cyclopropyl or methylamino group,
   R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group,
      and the HIV protease inhibitor as the active ingredient is VX-478, KNI-272, AG-1343, Saquinavir, Ritonavir, indinavir and Compound A,
22) a therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl]piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the HIV protease inhibitor as the active ingredient is VX-478, KNI-272, AG-1343, Saquinavir, Ritonavir, indinavir and Compound A,
23) a therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the HIV protease inhibitor as the active ingredient is AG-1343, Saquinavir, Ritonavir or indinavir,
24) a therapeutic or preventive agent in which the quinolone carboxylic acid having anti-HIV activity as the active ingredient is the above-mentioned formula (Ia), (Ib) or (Ic), the reverse transcriptase inhibitor as the active ingredient is ZDV, DDI, DDC, d4T, 3TC, FTC or Nevirapine, and the HIV protease inhibitor as the active ingredient is VX-478, KNI-272, AG-1343, Saquinavir, Ritonavir, indinavir and Compound A,
25) a therapeutic or preventive agent in which the quinolone carboxylic acid having anti-HIV activity as the active ingredient is the above-mentioned formula (Ia), the reverse transcriptase inhibitor as the active ingredient is ZDV, DDI, DDC, d4T, 3TC, FTC or Nevirapine, and the HIV protease inhibitor as the active ingredient is VX-478, KNI-272, AG-1343, Saquinavir, Ritonavir, indinavir and Compound A,
26) a therapeutic or preventive agent in which the quinolone carboxylic acid having anti-HIV activity as the active ingredient is a compound represented by the formula (Ia), wherein
   X is a fluorine atom,
   Y is a hydrogen or fluorine atom, or an amino, methyl or ethyl group,
   Z is an optionally protected carboxylic group,
   Q is a group of the formula (d) and R² of the formula (d) is a methoxy, difluoromethoxy or trifluoromethyl group,
   R¹ is a hydrogen atom; or a methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group,
   R is a 4-(2-pyrimidinyl) piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group, and the reverse transcriptase inhibitor as the active ingredient is ZDV, DDI and Nevirapine, and the HIV protease inhibitor as the active ingredient is VX-478, KNI-272, AG-1343, Saquinavir, Ritonavir, indinavir and Compound A,
27) a therapeutic or preventive agent in which the quinolone carboxylic acid having anti-HIV activity as the active ingredient is a compound represented by the formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a methyl, ethyl, 2-hydroxyethyl, 2-fluoroethyl, cyclopropyl or methylamino group,
   R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group, and the reverse transcriptase inhibitor as the active ingredient is ZDV, DDI and Nevirapine, and the HIV protease inhibitor as the active ingredient is VX-478, KNI-272, AG-1343, Saquinavir, Ritonavir, indinavir and Compound A,
28) a therapeutic or preventive agent in which the quinolone carboxylic acid having anti-HIV activity as the active ingredient is a compound represented by the formula (Ia), wherein
   X is a fluorine atom,
   Y is a hydrogen or fluorine atom, or an amino, methyl or ethyl group,
   Z is an optionally protected carboxyl group,
   Q is a group of the formula (d) and R² of the formula (d) is a methoxy, difluoromethoxy or trifluoromethyl group,
   R¹ is a methyl, ethyl, 2-hydroxyethyl, 2-fluoroethyl, cyclopropyl or methylamino group,
   R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group, and the reverse transcriptase inhibitor as the active ingredient is ZDV, DDI and Nevirapine, and the HIV protease inhibitor as the active ingredient is AG-1343, Saquinavir, Ritonavir or indinavir,
29) a therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the reverse transcriptase inhibitor as the active ingredient is ZDV, and the HIV protease inhibitor as the active ingredient is VX-478, KNI-272, AG-1343, Saquinavir, Ritonavir, indinavir and Compound A,
30) a therapeutic or preventive agent in which the quinolone carboxylic acid having anti-HIV activity as the active ingredient is
   1-cyclopropyl-6-fluoro-8-trifluoromethyl-1,4-dihydro-4-oxo-7-[4-(2-pyridyl)piperazin-1-yl]quinoline-3-carboxylic acid,
   6-fluoro-8-trifluoromethyl-1,4-dihydro-4-oxo-1-methyl-7-[4-(2-pyrimidinyl)piperazin-1-yl]quinoline-3-carboxylic acid,
   6-fluoro-8-trifluoromethyl-1,4-dihydro-4-oxo-1-methyl-7-[4-(2-pyridyl)piperazin-1-yl]quinoline-3-carboxylic acid,
   6-fluoro-8-trifluoromethyl-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-ethyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(2-benzothiazolyl)piperazin-1-yl]quinoline-3-carboxylic acid,
   6-fluoro-8-difluoromethoxy-1,4-dihydro-4-oxo-1-methyl-7-[4-(6-methoxy-2-benzothiazolyl)piperazin-1-yl]quinoline-3-carboxylic acid,
   6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
   6-fluoro-8-difluoromethoxy-1,4-dihydro-4-oxo-1-methylamino-7-[4-(2-benzoxazolyl)piperazin-1-yl]quinoline-3-carboxylic acid, and the reverse transcriptase inhibitor as the active ingredient is ZDV, and the HIV protease inhibitor as the active ingredient is AG-1343, Saquinavir, Ritonavir or indinavir.

In the present invention specific combinations of quinolone carboxylic acid having anti-HIV activity and/or reverse transcriptase inhibitor and/or HIV protease inhibitor used in combination for the treatment and prevention of AIDS include the combinations described below, but the present invention is not limited to these combinations.

The quinolone carboxylic acid having anti-HIV activity shown in Table H are indicated with the numbers of the Preparation examples in which they are prepared.

**Table H**

| No. | Quinolone carboxylic acid | Reverse transcriptase inhibitor | Protease inhibitor |
|---|---|---|---|
| 1 | 117 | ZDV | - |
| 2 | 144 | ZDV | - |
| 3 | 147 | ZDV | - |
| 4 | 149 | ZDV | - |
| 5 | 151 | ZDV | - |
| 6 | 156 | ZDV | - |
| 7 | 117 | DDI | - |
| 8 | 144 | DDI | - |
| 9 | 147 | DDI | - |
| 10 | 117 | Nevirapine | - |
| 11 | 144 | Nevirapine | - |
| 12 | 147 | Nevirapine | - |
| 13 | 117 | ZDV | AG-1343 |
| 14 | 144 | ZDV | AG-1343 |
| 15 | 147 | ZDV | AG-1343 |
| 16 | 149 | ZDV | AG-1343 |
| 17 | 151 | ZDV | AG-1343 |
| 18 | 156 | ZDV | AG-1343 |
| 19 | 117 | ZDV | Saquinavir |
| 20 | 144 | ZDV | Saquinavir |
| 21 | 147 | ZDV | Saquinavir |
| 22 | 149 | ZDV | Saquinavir |
| 23 | 151 | ZDV | Saquinavir |
| 24 | 156 | ZDV | Saquinavir |
| 25 | 117 | ZDV | Ritonavir |
| 26 | 144 | ZDV | Ritonavir |
| 27 | 147 | ZDV | Ritonavir |
| 28 | 149 | ZDV | Ritonavir |
| 29 | 151 | ZDV | Ritonavir |
| 30 | 156 | ZDV | Ritonavir |

In the present invention, outstanding effects superior to those obtained in the case of using each agent alone are demonstrated by combined use of the quinolone carboxylic acid having anti-HIV activity, reverse transcriptase inhibitor and HIV protease inhibitor.

In addition, these effects are brought about even if two or three kinds of the agents are not present simultaneously in the body. Namely, the effects are demonstrated even if two or three kinds of the agents are not simultaneously detected in the blood.

In the treatment of AIDS, there is a tendency towards combination therapy, and it is convenient to administer two or three kinds of drugs simultaneously. For this reason, the quinolone carboxylic acid having anti-HIV activity, reverse transcriptase inhibitors and/or HIV protease inhibitors can be administered in the form of a blended agent. In terms of preparation technology, when it is not desirable to simultaneously mix the agents physically, each single agent can be administered simultaneously. In addition, as was previously stated, since outstanding effects are demonstrated even when two or three kinds of the agents are not administered simultaneously, one of the agents can also be administered at suitable intervals after administration of the other(s). The allowed maximum limit on the administration interval of the two or three kinds of drugs to achieve the outstanding effects brought about by the two or three kinds of drugs can be confirmed through clinical testing or animal experiments.

The administration route of the quinolone carboxylic acid having anti-HIV activity, reverse transcriptase inhibitor and HIV protease inhibitor used in the present invention is an oral route and a non-oral route.

Thus, two or three kinds of drugs can be separately prepared in unit administration forms or physically in a single unit administration form by mixing the two or three kinds of drugs. Examples of unit administration forms include formulations for oral administration preparations such as tablets, capsules, granules, powders, syrups, etc. or formulations for non-oral administration such as intravenous injections, intramuscular injections, suppositories, etc., and these can be prepared by routine preparation technology.

The doses and dosing ratios of the quinolone carboxylic acid having anti-HIV activity, reverse transcriptase inhibitor and HIV protease inhibitor used in the present invention will vary over a wide range depending upon the activities of the individual drugs and various other conditions including the symptoms, age and body weight of the patient.

In addition, the dose of the quinolone carboxylic acid having anti-HIV activity, reverse transcriptase inhibitor and HIV protease inhibitor is lower than the case of using individual agents due to the outstanding effects produced by combined use of these two or three kinds of drugs.

For example, in the case of a quinolone carboxylic acid having anti-HIV activity and a reverse transcriptase inhibitor, the doses are 15 mg and 100 mg, respectively, in the case of quinolone carboxylic acid having anti-HIV activity and an HIV protease inhibitor, the doses are 5 mg and 300 mg, respectively, and in the case of a quinolone carboxylic acid having anti-HIV activity, a reverse transcriptase inhibitor and an HIV protease inhibitor, the doses are 3 mg, 20 mg and 180 mg, respectively.

Although the doses of quinolone carboxylic acid having anti-HIV activity, reverse transcriptase inhibitor and HIV protease inhibitor used in the present invention will vary over a wide range, in general, their doses (mg of drug used/day) are about 1 to 100 mg, 10 to 500 mg and 10 to 500 mg, respectively.

The ratios of the doses of these two or three kinds of drugs will vary over a wide range, in general, in terms of weight ratio, the ratio is within a range from 1:500 to 1:5 in the case of a quinolone carboxylic acid having anti-HIV activity and a reverse transcriptase inhibitor, within the range from 1:500 to 1:5 in the case of a quinolone carboxylic acid having anti-HIV activity and an HIV protease inhibitor, and in the case of a quinolone carboxylic acid having anti-HIV activity, a reverse transcriptase inhibitor and an HIV protease inhibitor, the ratio of the largest amount of drug to the smallest amount of drug is within a range from 1:500 to 1:5.

In the present invention, the quinolone carboxylic acid having anti-HIV activity, reverse transcriptase inhibitor and HIV protease inhibitor are administered once per day in the above-mentioned doses or at several times per day simultaneously or at different times.

The present invention will be described in more detail by way of examples, but the scope of the present invention is not limited to these examples.

### Best Mode for Carrying out the Invention

### (Example) In Vitro Combined Test

### 1) Cells and Virus Strain

Human T cell transformed cell line MT4 cells were cultured in RPMI 1640 medium containing 10% fetal bovine serum. HIV-1_{IIIB} strain was obtained from HIV-1_{IIIB} chronically infected Molt-4 cells as a supernatant fluid and used as the source of infectious viruses. The 50% tissue culture infectious dose (TCID₅₀) of this fluid was determined by the endpoint titration methods.

### 2) Drug Combined Test

The compound of Preparation example 117 was added in a serial dilution in the vertical direction to six 96-well microtiter plates. As a result of adding ZDV (Sigma) in a serial dilution in the horizontal direction, diluted solutions containing mixtures of various concentrations were prepared in 96-well microtiter plates (n=4).

MT4 cells were exposed to HIV-1_{IIIB} fluid at a multiplicity of infection (m.o.i) 0.001 TCID₅₀/ml at 37°C for 1 hour, then the cells were washed twice with PBS, and aliquots (5 x 10⁴ cells/well) were placed in the plates containing various concentration of agent prepared as described above. After incubation for 6 days, the survival rate of infected MT4 cells was determined by the cell survival test using MTT (Sigma). Incidentally, in this test, the absorbance at O.D. 540 nm was measured by a plate reader, and the survival rate was determined by drawing a cell survival curve.

### 3) Analysis of Combined Test Data

Analysis was performed according to the method of Barenbaum using an isobologram and combination index (CI) (Barenbaum, M.C., Pharmacol. Rev., 41, 93-141, 1989). In this case, the CI value was calculated by the general isobole equation. Determination of synergy, addition and competitive inhibition was performed by isobologram curve patterns and/or statistical comparison of experimental CI values with CI values in typical additive effects (CI values when identical compounds are used in combination (0.85±0.11, Taylor, D.L. et al., Antiviral Chem. Chemother., 6, 143-152, 1995)).

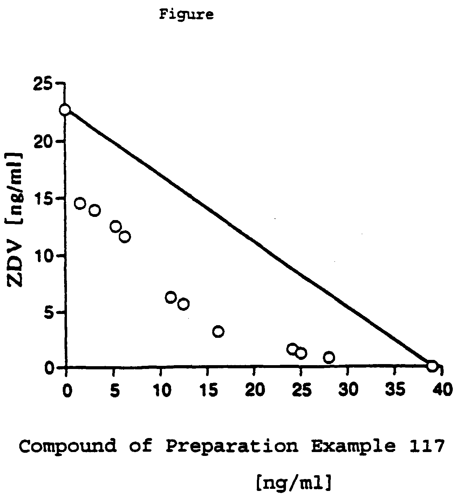

When the IC₅₀ values in the case of combined use of the compound of Preparation example 117 with ZDV were plotted in an isobologram, the line resulting from connecting the plotted points was located considerably below the straight line indicating the theoretical additive effects, and a curve that indicates the typical synergistic effects was drawn (Fig. 1). In this case, CI values were 0.55-0.75 and indicated statistically significant synergistic effects. Similarly, synergistic effects were also observed in the case of combined use of the compound of Preparation example 117 and DDI and in the case of combined use of the compound of Preparation example 117 and 3TC.

**Table 32**

| Preparation example 117 [ng/ml] | ZDV [ng/ml] | Combination index | Student t-test | Evaluation |
|---|---|---|---|---|
| 39.0 | 0 | - | | |
| 28.0 | 0.78 | 0.75 | | |
| 25.0 | 1.20 | 0.69 | | |
| 24.1 | 1.56 | 0.69 | | |
| 16.2 | 3.13 | 0.55 | | |
| 12.5 | 5.60 | 0.57 | P<0.001 | Synergistic effect |
| 11.1 | 6.25 | 0.56 | | |
| 6.25 | 11.6 | 0.67 | | |
| 5.30 | 12.5 | 0.69 | | |
| 3.13 | 13.9 | 0.69 | | |
| 1.56 | 14.5 | 0.68 | | |
| 0 | 22.7 | - | | |

**Table 33**

| Preparation example 117 [ng/ml] | 3TC [µg/ml] | Combination index | Student t-test | Evaluation |
|---|---|---|---|---|
| 0 | 0.60 | - | | |
| 0.96 | 0.50 | 0.89 | | |
| 0.38 | 0.50 | 0.86 | | |
| 0.78 | 0.52 | 0.92 | | |
| 1.56 | 0.43 | 0.81 | | |
| 3.13 | 0.34 | 0.76 | P<0.001 | Synergistic effect |
| 4.57 | 0.25 | 0.70 | | |
| 6.25 | 0.19 | 0.71 | | |
| 8.0 | 0.125 | 0.71 | | |
| 12.4 | 0.063 | 0.88 | | |
| 12.7 | 0.0313 | 0.85 | | |
| 13.0 | 0.0156 | 0.84 | | |
| 16.0 | 0 | - | | |

**Table 34**

| Preparation example 117 [ng/ml] | DDI [µM] | Combination index | Student t-test | Evaluation |
|---|---|---|---|---|
| 0 | 5.40 | - | | |
| 0.39 | 4.27 | 0.82 | | |
| 0.78 | 4.38 | 0.87 | | |
| 1.56 | 3.65 | 0.79 | | |
| 3.13 | 2.80 | 0.74 | | |
| 3.85 | 2.50 | 0.74 | P<0.001 | Synergistic effect |
| 6.25 | 1.67 | 0.76 | | |
| 7.70 | 1.25 | 0.78 | | |
| 10.0 | 0.63 | 0.83 | | |
| 10.5 | 0.313 | 0.81 | | |
| 11.0 | 0.156 | 0.81 | | |
| 14.0 | 0 | - | | |

### Brief Description of the Drawing

Fig. 1 is a graph showing a plot of the IC₅₀ values of the compound of Preparation example 117 or ZDV under a fixed concentration of ZDV or the compound of Preparation example 117. The concentration of ZDV is plotted on the vertical axis, while the concentration of the compound of Preparation example 117 is plotted on the horizontal axis. The straight line indicates the theoretical straight line in the case of exhibiting additive effects.
Pharmaceutical preparation examples, Preparation examples and Reference examples are shown below.

### (formulation example 1) formulation of a mixture of the Compound of Preparation example 117 and ZDV

### 1) Capsules

15 g of the compound of Preparation example 117, 100 g of ZDV, 140.5 g of lactose, 15 g of crospovidone, 3 g of magnesium stearate and 1.5 g of sodium lauryl sulfate were uniformly mixed to obtain a mixture, and 275 mg aliquots of the mixture are filled into No. 2 capsules. The capsules contain 15 mg of the compound of Preparation example 117 and 100 mg of ZDV per capsule.

### 2) Tablets

After 15 g of the compound of Preparation example 117, 100 g of ZDV, 150 g of anhydrous lactose, 79 g of crystalline cellulose, 25 g of sodium cross carmelose, 4 g of magnesium stearate and 2 g of light silicic anhydride were uniformly mixed, tablets having a diameter of 10 mm and weighing 375 mg were obtained using a tablet machine. These tablets contain 15 mg of the compound of Preparation example 117 and 100 mg of ZDV per tablet.

### 3) Fine Grains

After uniformly mixing 30 g of the compound of Preparation example 117, 200 g of ZDV, 640 g of lactose and 100 g of low-substituted hydroxypropyl cellulose, the mixture was atomized with an aqueous solution of hydroxypropyl cellulose (30 g as solid) and formed into granules using a fluid bed granulating machine to obtain fine grains. These fine grains contain 30 mg of the compound of Preparation example 117 and 200 mg of ZDV per gram.

### 4) Granules

After uniformly mixing 30 g of the compound of Preparation example 117, 200 g of ZDV, 800 g of lactose, 275 g of crystalline cellulose, 150 g of low-substituted hydroxypropyl cellulose and 45 g of hydroxypropyl cellulose, purified water is added to the mixture and after kneading of the mixture with a high-speed stirring granulating machine, wet granules were made with an extrusion granulating machine followed by drying with a dryer to obtain granules. These granules contain 30 mg of the compound of Preparation example 117 and 200 mg of ZDV per 1.5 g of granules.

### (formulation example 2) formulation of a mixture of the Compound of Preparation example 117 and Compound A

### 1) Capsules

5 g of the compound of Preparation example 117, 300 g of Compound A, 162.5 g of lactose, 25 g of crospovidone, 5 g of magnesium stearate and 2.5 g of sodium lauryl sulfate were uniformly mixed to obtain a mixture, and 500 mg aliquots of the mixture are filled into No. 0 capsules. The capsules contain 5 mg of the compound of Preparation example 117 and 300 mg of Compound A per capsule.

### 2) Tablets

After uniformly mixing 5 g of the compound of Preparation example 117, 300 g of Compound A, 115 g of anhydrous lactose, 40 g of crystalline cellulose, 25 g of sodium cross carmelose, 10 g of magnesium stearate and 5 g of light silicic anhydride, tablets having a length of 15 mm, width of 7.5 mm and weighing 500 mg were obtained using a tablet making machine. These tablets contain 5 mg of the compound of Preparation example 117 and 300 mg of Compound A per tablet.

### 3) Fine Grains

After uniformly mixing 10 g of the compound of Preparation example 117, 600 g of Compound A, 695 g of lactose and 150 g of low-substituted hydroxypropyl cellulose, the mixture was atomized with an aqueous solution of hydroxypropyl cellulose (45 g as solid) and formed into granules using a fluid bed granulating machine to obtain fine grains. These fine grains contain 10 mg of the compound of Preparation example 117 and 600 mg of Compound A per 1.5 g.

### 4) Granules

After uniformly mixing 10 g of the compound of Preparation example 117, 600 g of Compound A, 850 g of lactose, 280 g of crystalline cellulose, 200 g of low-substituted hydroxypropyl cellulose and 60 g of hydroxypropyl cellulose, purified water is added to the mixture and after kneading of the mixture with a high-speed stirring granulating machine, wet granules were made with an extrusion granulating machine followed by drying with a dryer to obtain granules. These granules contain 10 mg of the compound of Preparation example 117 and 600 mg of Compound A per 2 g of granules.

### (formulation example 3) formulation of a mixture of the Compound of Preparation example 117, ZDV and Compound A

### 1) Capsules

3 g of the compound of Preparation example 117, 20 g of ZDV, 180 g of Compound A, 119 g of lactose, 18 g of crospovidone, 8 g of magnesium stearate and 2 g of sodium lauryl sulfate were uniformly mixed to obtain a mixture, and 350 mg aliquots of the mixture are filled into No. 1 capsules. The capsules contain 3 mg of the compound of Preparation example 117, 20 mg of ZDV and 180 mg of Compound A per capsule.

### 2) Tablets

After uniformly mixing 3 g of the compound of Preparation example 117, 20 g of ZDV, 180 g of Compound A, 190 g of anhydrous lactose, 67 g of crystalline cellulose, 25 g of sodium croscarmellose, 10 g of magnesium stearate and 5 g of light silicic anhydride, tablets having a length of 15 mm, width of 7.5 mm and weighing 500 mg were obtained using a tablet making machine. These tablets contain 5 mg of the compound of Preparation example 117, 20 mg of ZDV and 180 mg of Compound A per tablet.

### 3) Fine Grains

After uniformly mixing 3 g of the compound of Preparation example 117, 20 g of ZDV, 180 g of Compound A, 667 g of lactose and 100 g of low-substituted hydroxypropyl cellulose, the mixture was atomized with an aqueous solution of hydroxypropyl cellulose (30 g as solid) and formed into granules using a fluid bed granulating machine to obtain fine grains. These fine grains contain 3 mg of the compound of Preparation example 117, 20 mg of ZDV and 180 mg of Compound A per gram.

### 4) Granules

After uniformly mixing 3 g of the compound of Preparation example 117, 20 g of ZDV, 180 g of Compound A, 827 g of lactose, 275 g of crystalline cellulose, 150 g of low-substituted hydroxypropyl cellulose and 45 g of hydroxypropyl cellulose, purified water is added to the mixture and after kneading of the mixture with a high-speed stirring granulating machine, wet granules were made with an extrusion granulating machine followed by drying with a dryer to obtain granules. These granules contain 3 mg of the compound of Preparation example 117, 20 mg of ZDV and 180 mg of Compound A per 1.5 g of granules.

### (Preparation example 1)

### Synthesis of 1-cyclopropyl-6-fluoro-8-difluoromethoxy-7-[4-(2-methoxyphenyl)piperazin-1-yl]-1,4-dihydro-4-oxoquinoline-3-carboxylic acid·hydrochloride

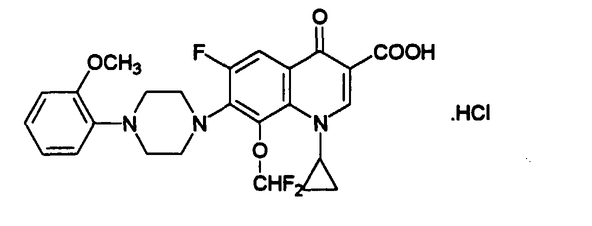

1.66 g (0.005 mol) of 1-cyclopropyl-6,7-difluoro-8-difluoromethoxy-1,4-dihydro-4-oxoquinoline-3-carboxylic acid and 2.4 g (0.0125 mol) of 1-(2-methoxyphenyl)piperazine were dissolved in 20 ml of pyridine and the solution was stirred at 105 to 110°C for 3 hours, followed by evaporation of the solvent under reduced pressure. The residue was subjected to silica gel column chromatography (eluting solvent : mixture of chloroform:methanol=9.5:0.5) to obtain 1.33 g of a free form of the desired compound. Then, 1.33 g of the free form was dissolved in 100 ml of a mixture of chloroform and methanol (4:1), and 2 ml of concentrated hydrochloric acid was added to the solution, followed by concentration of the mixture under reduced pressure. The residue was washed with a mixture of methanol and ethanol (4:1) and followed by distilling off the solvent to obtain 1.08 g of the title compound as a white powder.
m.p.: 223-225°C
NMR (DMSO-d₆, δ): 1.04-1.07 (2H, m), 1.16-1.17 (2H, m), 3.30 (4H, br.s), 3.47 (4H, br.s), 3.86 (3H, s), 4.09-4.12 (1H, m), 6.90-7.27 (5H, m), 7.95-7.98 (1H, d, J=12.1Hz), 8.79 (1H, s)
MS spectrum (CI): m/e 504 (M⁺ +1)

### (Preparation examples 2 to 62)

Compounds shown in Tables 35 to 38 were prepared in similar procedures to that described in Preparation example 1.

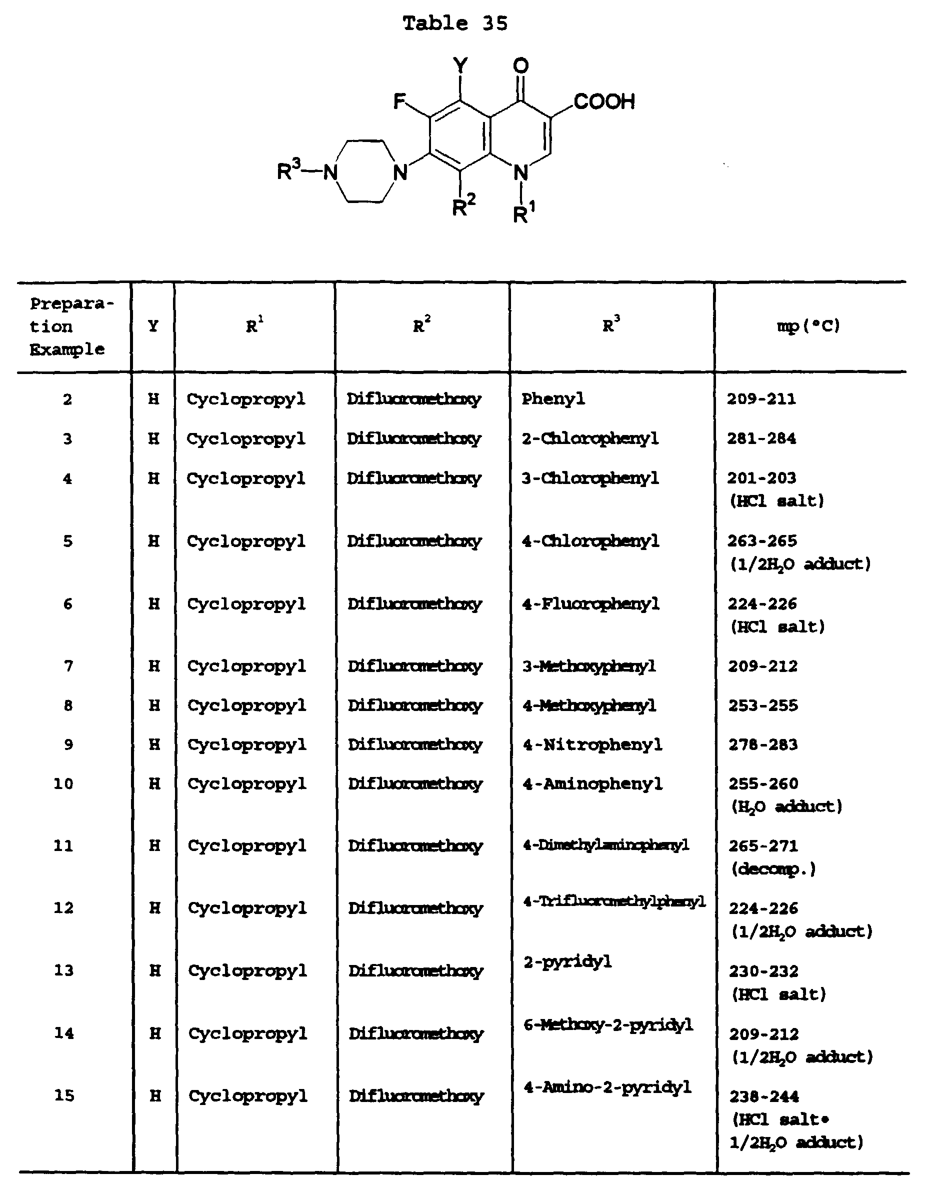

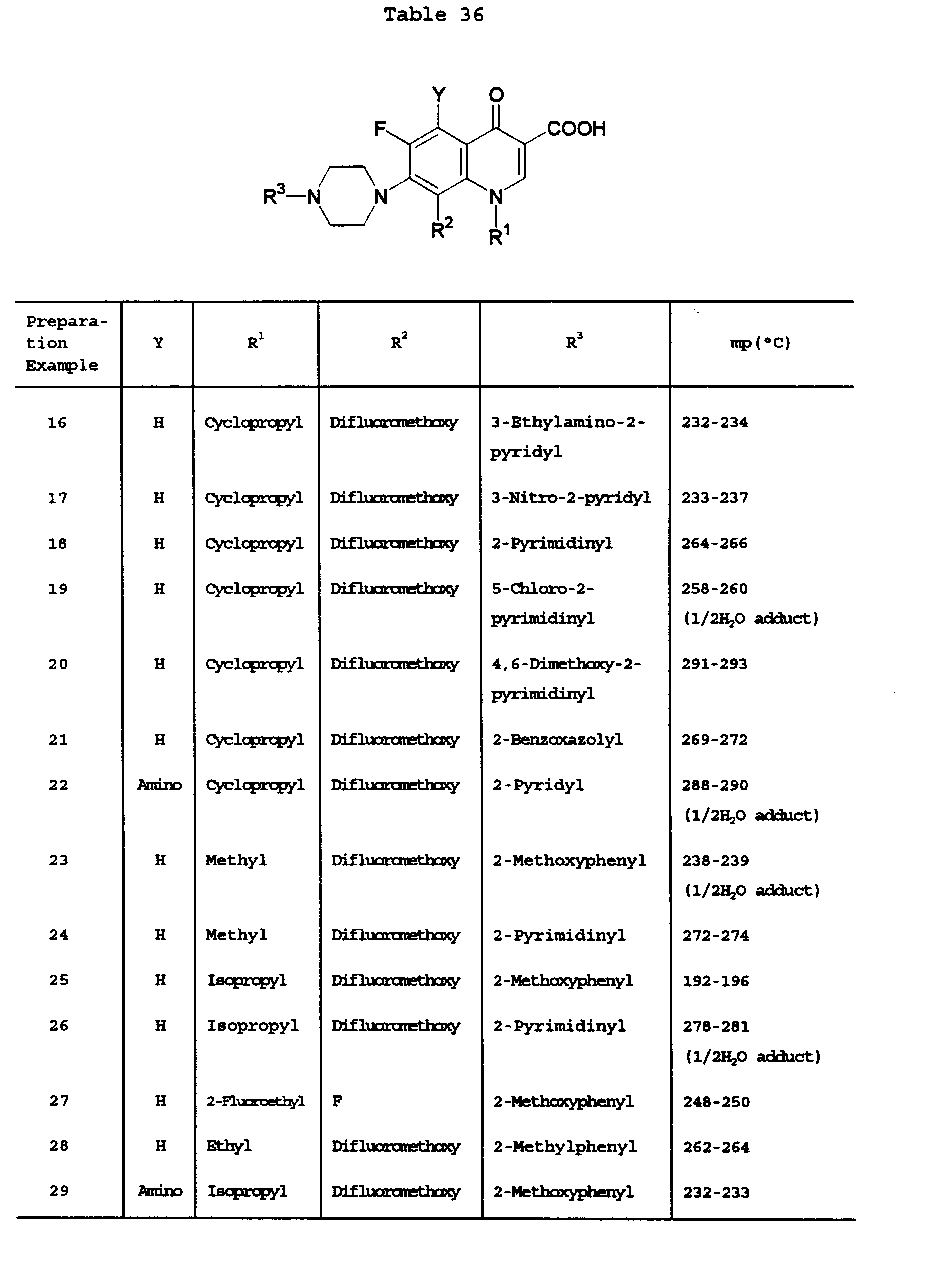

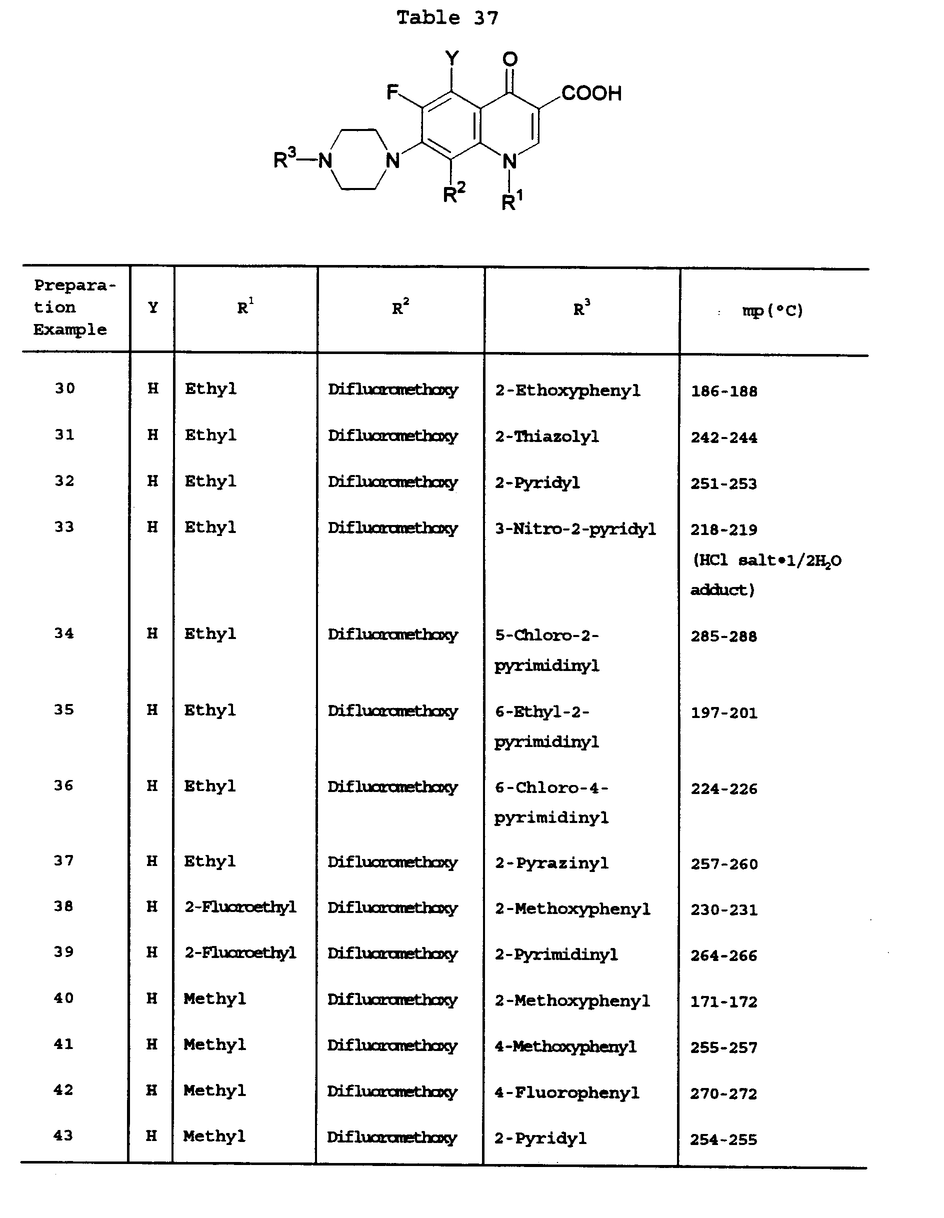

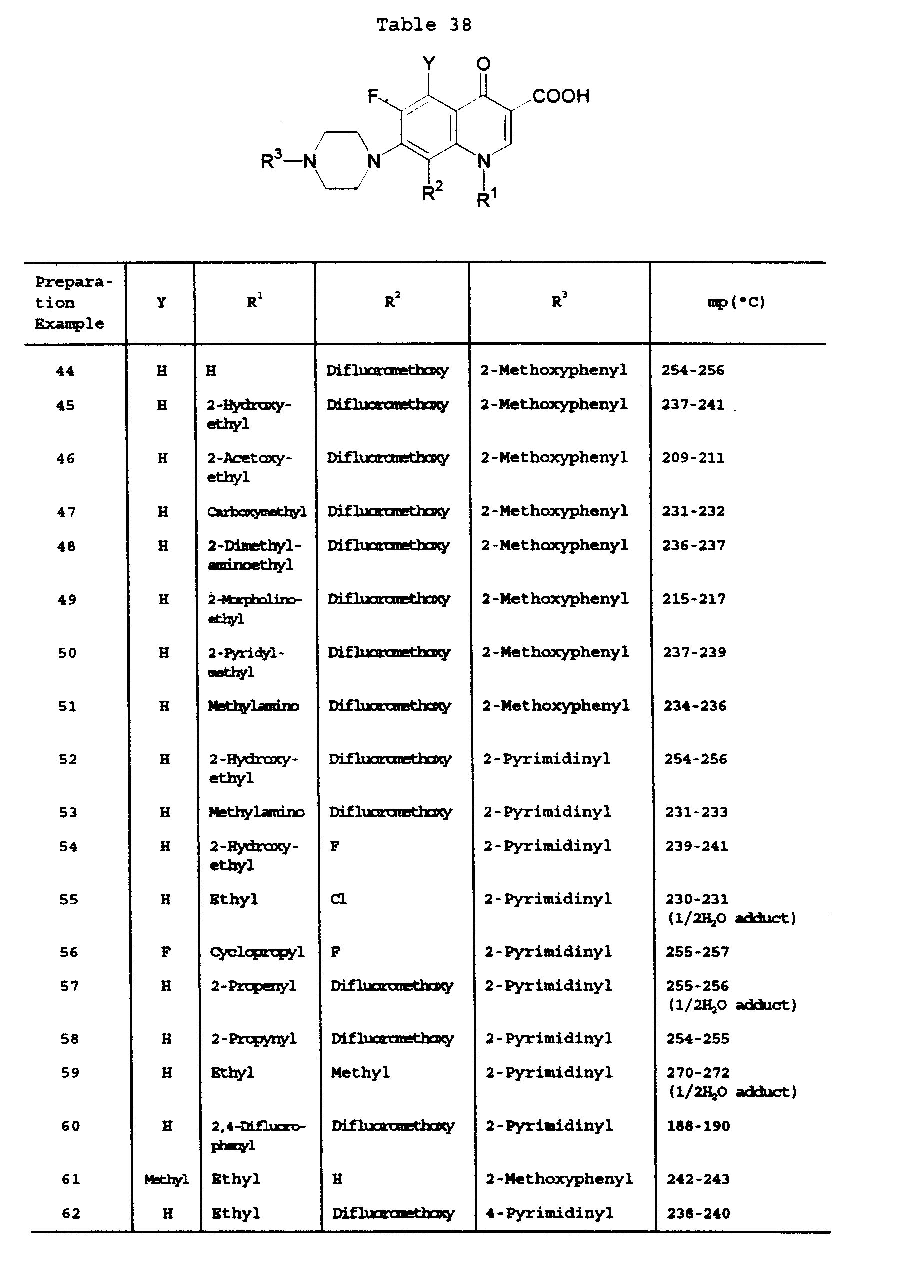

### (Preparation example 63)

### Synthesis of 1-ethyl-6-fluoro-8-difluoromethoxy-7-[4-(2-methoxyphenyl)piperazin-1-yl]-1,4-dihydro-4-oxoquinoline-3-carboxylic acid

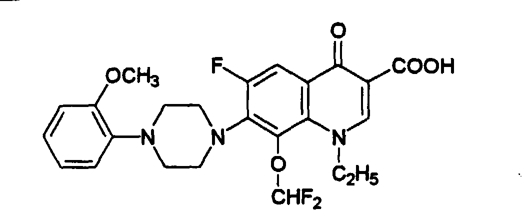

4.54 g (0.032 mol) of boron trifluoride diethyl ether complex was added to a suspension of 5.0 g (0.016 mol) of 1-ethyl-6,7-difluoro-8-difluoromethoxy-1,4-dihydro-4-oxoquinoline-3-carboxylic acid in 20 ml of methyl isobutyl ketone and the mixture was refluxed with stirring for 6 hours. After cooling the reaction mixture precipitate was separated from the mixture by filtration, followed by washing with ether and chloroform to obtain 32 g of a 1-ethyl-6,7-difluoro-8-difluoromethoxy-1,4-dihydro-oxoquinoline-3-carboxylic acid · BF₂ chelate compound as pale pink crystals.

0.5 g (0.00136 mol) of the chelate compound thus obtained, 1.3 g (0.0068 mol) of 1-(2-methoxyphenyl)piperazine and 2 ml of triethylamine were added to 5 ml of dimethyl sulfoxide, and the mixture was stirred at room temperature for 5 hours and allowed to stand overnight. Water was added to the reaction mixture and yellow precipitate was separated from the mixture by filtration, followed by washing with water. The crystals were dissolved in 100 ml of 80% methanol including 2.5 ml of triethylamine, and the solution was refluxed for 12 hours. The solvent was evaporated under reduced pressure and the residue was washed with a mixture of ethanol and water and followed by distilling off the solvent to obtain 0.5 g of the title compound as a pale red powder.
m.p.: 219-222°C
NMR (DMSO-d₆, δ): 1.28 (3H, t, J=7.0Hz), 3.11 (4H, br.s), 3.47 (4H, Br.s), 3.81 (3H, s), 4.74 (2H, q, J=7.0Hz), 6.92-7.32 (5H, m), 8.01-8.04 (1H, d, J=12.1Hz), 8.96 (1H, s)
MS spectrum (CI): m/e 492 (M⁺ +1)

### (Preparation examples 64 to 89)

### Compounds shown in Tables 39 to 41 were prepared in similar procedures to that described in Preparation example 63.

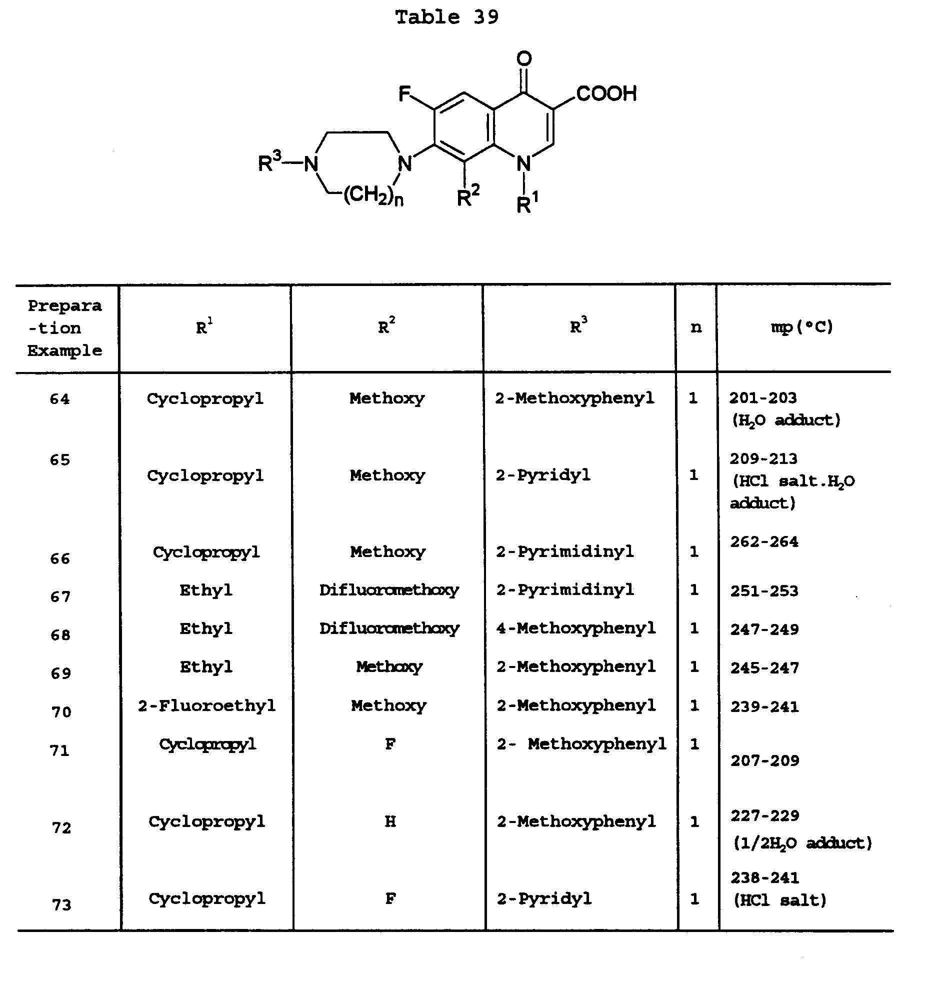

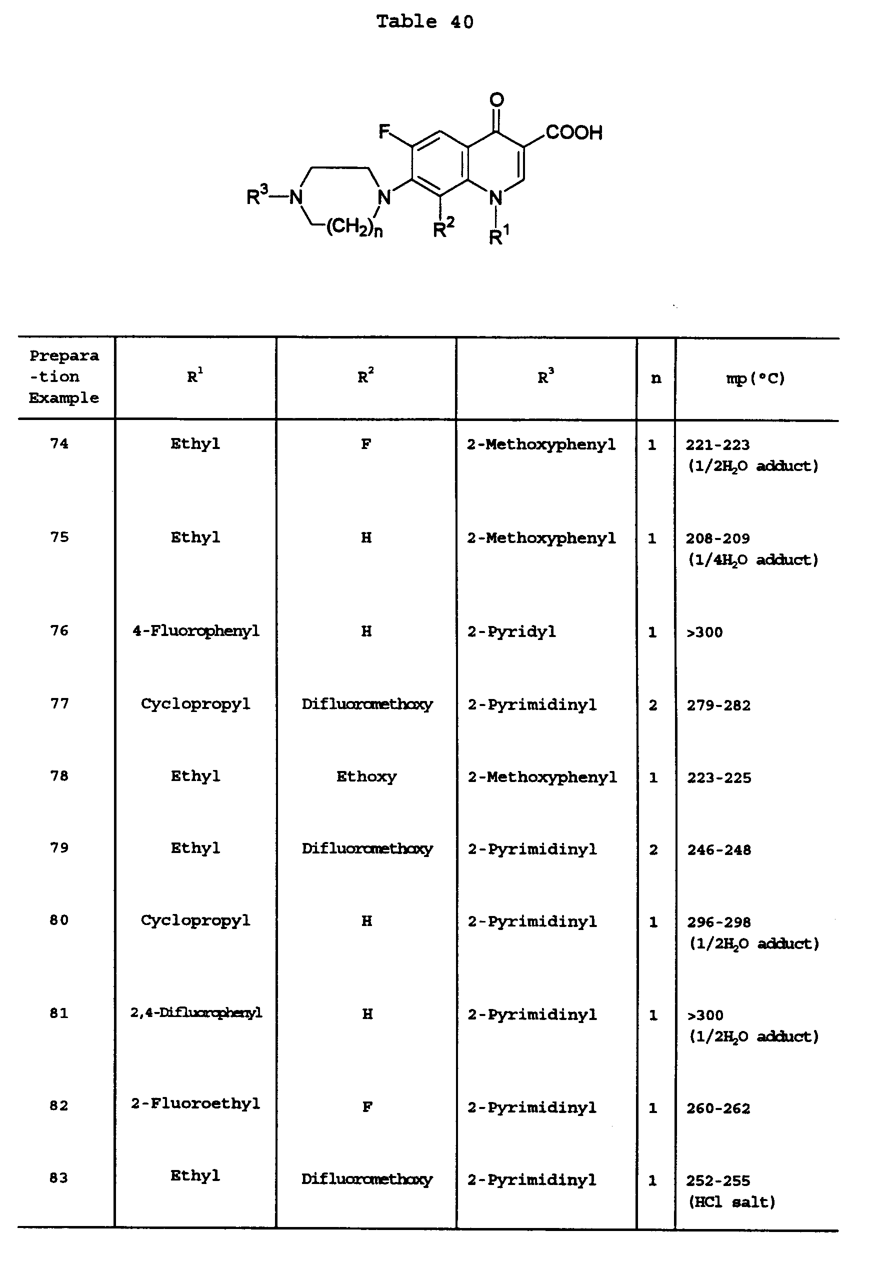

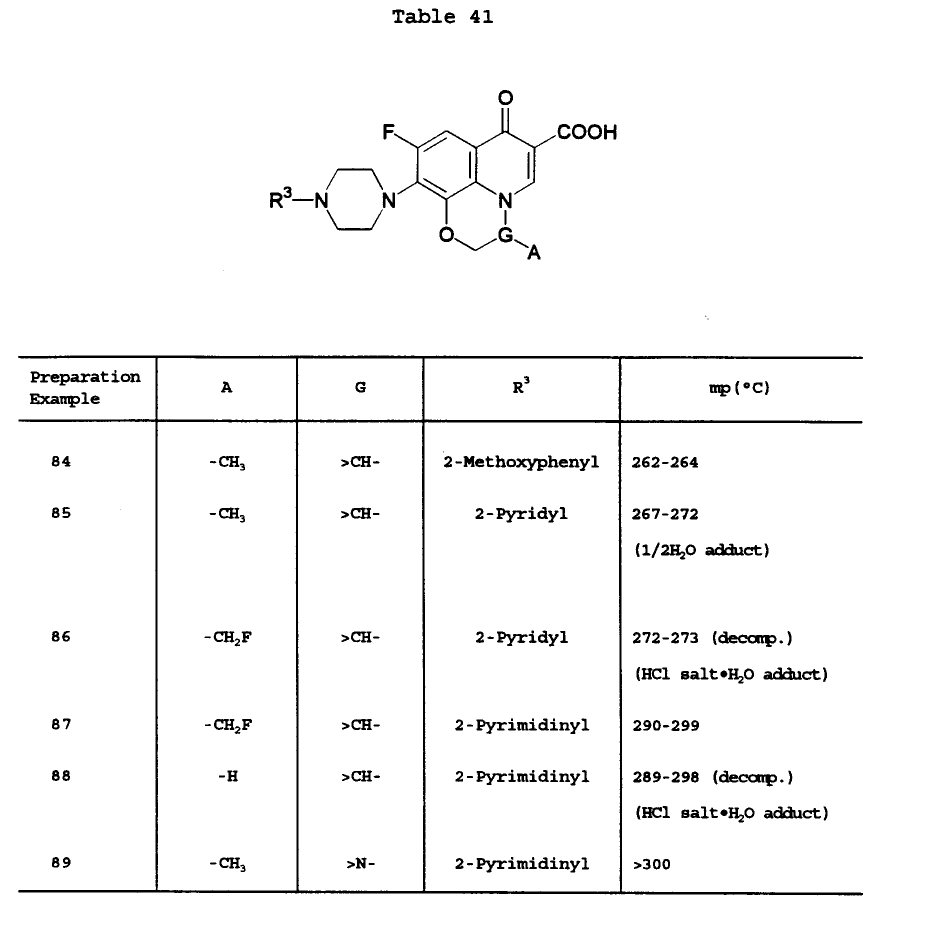

### (Preparation example 90)

### Synthesis of 6-fluoro-1-(4-fluorophenyl)-7-[4-(2-methoxyphenyl)piperazin-1-yl]-1,4-dihydro-4-oxo-1,8-naphthylidine-3-carboxylic acid

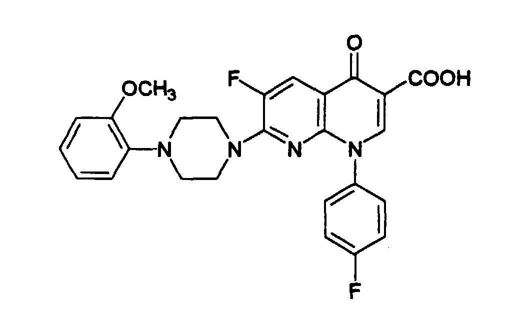

0.81 g (0.0042 mol) of 1-(2-methoxyphenyl)piperazine was dissolved in 40 ml of ethanol, and while the solution was stirred at 30°C, 1.02 g (0.0028 mol) of ethyl 7-chloro-6-fluoro-1-(4-fluorophenyl)-1,4-dihydro-4-oxo-1,8-naphthylidine-3-carboxylate was added to the solution by portions. After completion of the addition, the mixture was stirred at the same temperature for 4 hours. After cooling the reaction mixture precipitate was separated from the mixture by filtration, followed by washing with ethanol. 12 ml of a 6N aqueous hydrochloric acid solution was added to the precipitate and the mixture was refluxed for 6 hours. After cooling the reaction mixture the pH of the reaction mixture was adjusted to 8.5 with a 1N aqueous sodium hydroxide solution. Precipitate was separated from the mixture by filtration and subjected to silica gel column chromatography (eluting solvent: mixture of chloroform:methanol = 9.5:0.5) to obtain 0.87 g of the title compound as a slightly yellow powder.
m.p.: 272-273°C
NMR (DMSO-d₆, δ): 2.97 (4H, br.s), 3.71 (4H, br.s), 3.80 (3H, s), 6.86-7.70 (8H, m), 8.17-8.20 (1H, d, J=13.6Hz), 8.70 (1H, s), 15.13 (1H, s)
MS spectrum (CI): m/e 493 (M⁺ +1)

### (Preparation examples 91 to 93)

Compounds shown in Table 42 were prepared in similar procedures to that described in Preparation example 90.

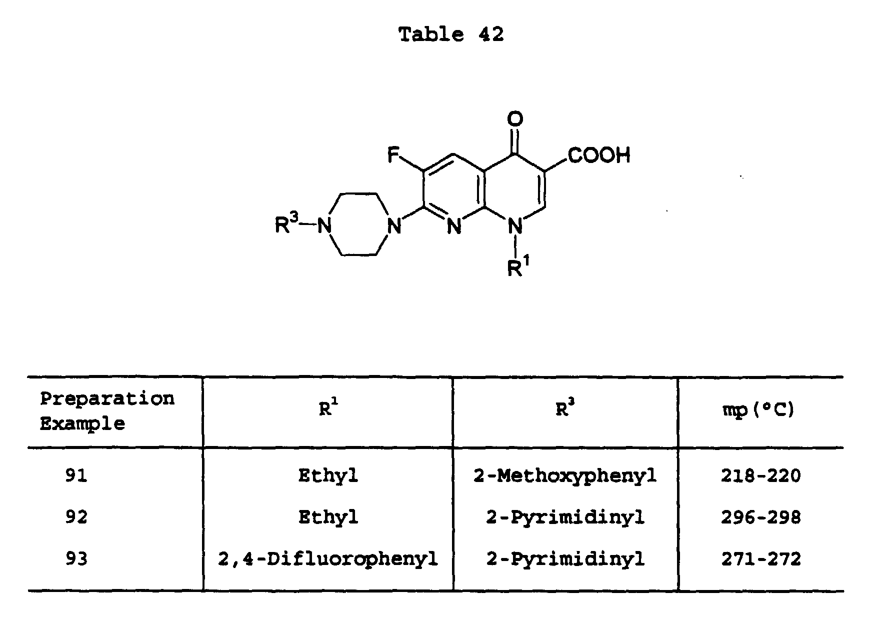

### (Preparation example 94)

### Synthesis of 1-ethyl-6-fluoro-8-difluoromethoxy-1-[3-methyl-4-(2-pyrimidinyl)piperazin-1-yl]-1,4-dihydro-4-oxoquinoline-3-carboxylic acid

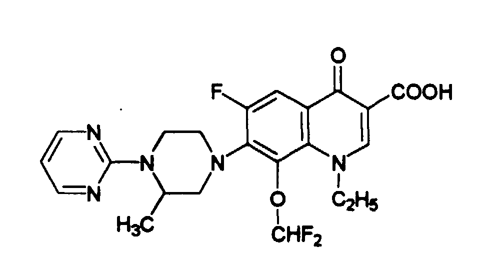

3.19 g (0.01 mol) of 1-ethyl-6,7-difluoro-8-difluoromethoxy-1,4-dihydro-4-oxoquinoline-3-carboxylic acid and 3.0 g (0.03 mol) of 2-methylpiperazine were dissolved in 60 ml of pyridine, and the solution was stirred at 105 to 110°C for 2 hours, followed by evaporation of the solvent under reduced pressure. Water was added to the residue and precipitate was separated from the mixture by filtration. The precipitate was washed with water and ethanol and followed by distilling off the solvent to obtain 3.23 g of 1-ethyl-6-fluoro-8-difluoromethoxy-7-(3-methylpiperazin-1-yl)-1,4-dihydro-4-oxoquinoline-3-carboxylic acid as a pale yellow powder.

1.6 g (0.004 mol) of this powder, 0.9 g (0.008 mol) of 2-chloropyrimidine and 0.81 g (0.008 mol) of triethylamine were added to 20 ml of N,N-dimethylformamide, and the mixture was stirred at 130°C for 15 hours, followed by evaporation of the solvent under reduced pressure. Ethanol was added to the residue and precipitate was separated from the mixture by filtration and subjected to silica gel coloum chromatography (eluting solvent: a mixture of chloroform:methanol = 9.5:0.5) to obtain 0.34 g of the title compound as a pale yellow powder.
m.p.: 219-221°C
MS spectrum (CI): m/e 478 (M⁺ +1)

| Elemental analysis (%); for C₂₂H₂₂F₃N₅O₄ | | | |
|---|---|---|---|
| Theoretical value; | C: 55.35, | H: 4.64, | N: 14.67 |
| Found value; | C: 55.41, | H: 4.56, | N: 14.65 |

### (Preparation examples 95 to 97)

Compounds shown in Table 43 were prepared in similar procedures to that described in Preparation example 94.

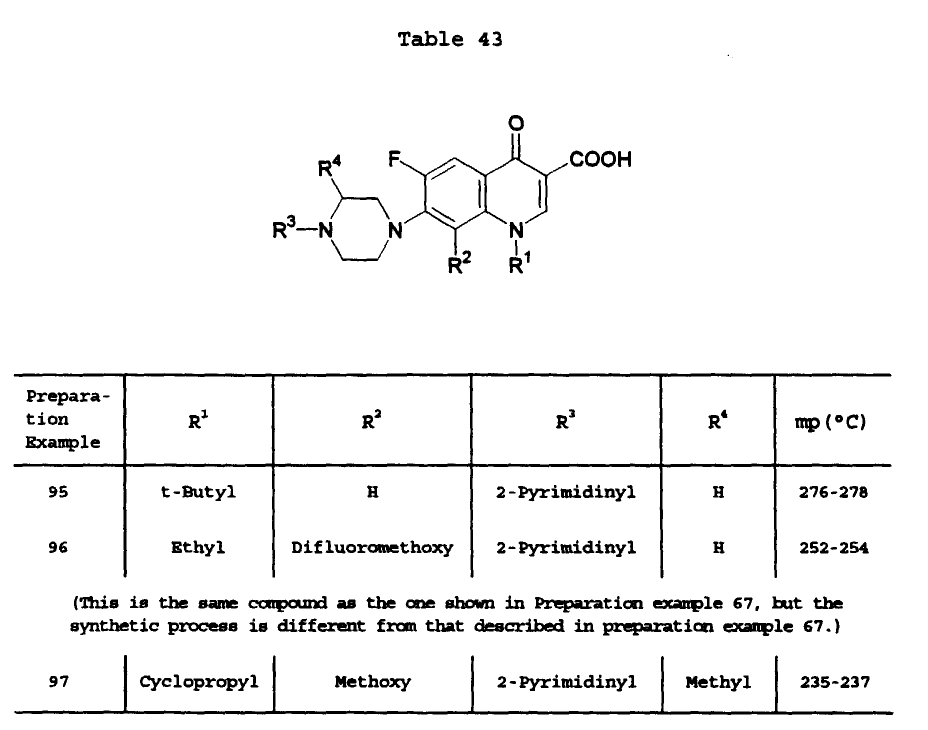

### (Preparation example 98)

### Synthesis of 1-ethyl-6-fluoro-8-difluoromethoxy-7-[4-(2-pyrimidinyl)piperazin-1-yl]-3-(5-tetrazolyl)-1,4-dihydro-4-oxoquinoline

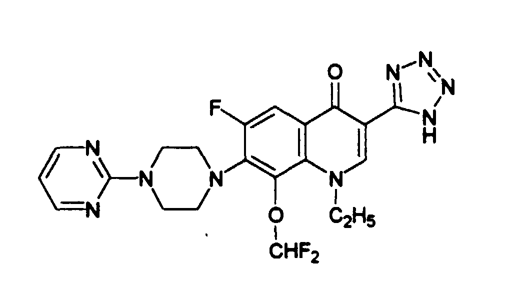

4.85 g of 3-cyano-1-ethyl-6,7-difluoro-8-difluoromethoxy-1,4-dihydro-4-oxoquinoline was reacted with 9.49 g of 1-(2-pyrimidinyl)piperazine in a similar manner to that described in Preparation example 1 to obtain 4.2 g of 3-cyano-1-ethyl-6-fluoro-8-difluoromethoxy-7-[4-(2-pyrimidinyl)piperazin-1-yl]-1,4-dihydro-4-oxoquinoline as a pale yellow powder (m.p.: 286 to 290°C).

0.5 g (0.0011 mol) of 3-cyano-1-ethyl-6-fluoro-8-difluoromethoxy-7-[4-(2-pyrimidinyl)piperazin-1-yl]-1,4-dihydro-4-oxoquinoline, 0.21 g (0.0033 mol) of sodium azide and 1.07 g (0.0033 mol) of tributyltin chloride were added to 25 ml of xylene, and the mixture was refluxed with stirring for 9 hours. After the reaction mixture was cooled to room temperature, 7 ml of a 1N aqueous hydrochloric acid solution was added to the reaction mixture. After stirring, precipitate was separated from the mixture by filtration and washed with ethanol and toluene, and then the precipitate was subjected to silica gel column chromatography (eluting solvent: mixture of chloroform:methanol = 9:1) to obtain 0.37 g of the title compound as a pale yellow powder.
m.p.: 265-268°C
MS spectrum (CI): m/e 488 (M⁺ +1)

| Elemental analysis (%); for C₂₁H₂₀F₃N₉O₂·1/2H₂O | | | |
|---|---|---|---|
| Theoretical value; | C: 50.81, | H: 4.06, | N: 25.39 |
| Found value; | C: 50.96, | H: 4.16, | N: 25.58 |

### (Preparation example 99)

### Synthesis of 1-ethyl-8-difluoromethoxy-7-[4-(2-pyrimidinyl)piperazin-1-yl]-1,4-dihydro-4-oxoquinoline-3-carboxylic acid

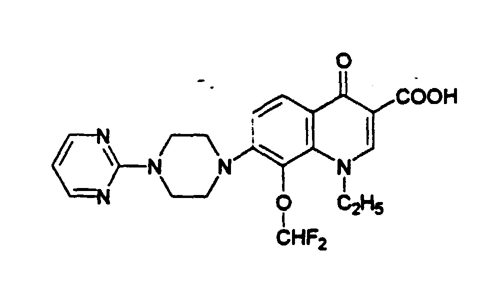

1-Ethyl-7-fluoro-8-difluoromethoxy-1,4-dihydro-4-oxoquinoline-3-carboxylic acid was reacted with 1-(2-pyrimidinyl)piperazine in a similar manner to that described in Preparation example 1 to obtain the title compound as a white powder.
m.p.: >300°C
MS spectrum (CI): m/e 446 (M⁺ +1)

| Elemental analysis (%); for C₂₁H₂₁F₂N₅O₄ | | | |
|---|---|---|---|
| Theoretical value; | C: 56.63, | H: 4.75, | N: 15.72 |
| Found value; | C: 56.70, | H: 4.62, | N: 15.34 |

### (Preparation example 100)

### Synthesis of 6-fluoro-1-methyl-4-oxo-7-[4-(2-pyrimidinyl)piperazin-1-yl]-1H,4H-[1,3]thiazeto[3,2-a]quinoline-3-carboxylic acid

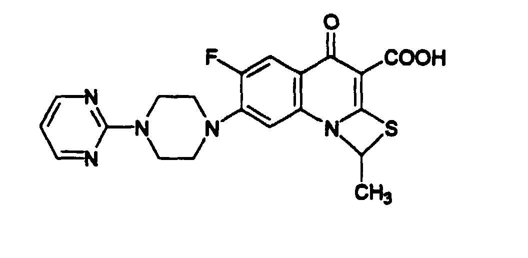

2.0 g (0.0064 mol) of ethyl 6,7-difluoro-1-methyl-4-oxo-1H,4H-[1,3]thiazeto[3,2-a]quinoline-3-carboxylate, 3.0 g (0.0129 mol) of 1-(2-pyrimidinyl)piperazine dihydrochloride and 3.9 g (0.0256 mol) of 1,8-diazabicyclo[5.4.0]-7-undecene were added to 16 ml of N, N-dimethylformamide, and the mixture was stirred at room temperature for 5 days. Water was added to the reaction mixture and precipitate was separated from the mixture by filtration. The precipitate was washed with water and followed by distilling off the solvent to obtain 2.8 g of ethyl 6-fluoro-1-methyl-4-oxo-7-[4-(2-pyrimidinyl)piperazin-1-yl]-1H,4H-[1,3]thiazeto[3,2-a]quinoline-3-carboxylate as a pale yellow powder.

5 ml of methanol, 1 ml of dioxane, 1 ml of water and 7 ml of 1N sodium hydroxide were added to 0.8 g (0.0018 mol) of this powder, and the mixture was stirred at room temperature for 3 days. A diluted aqueous acetic acid solution was added to the reaction mixture to adjust the pH of the reaction mixture to 7.2, and the precipitate was separated from the mixture by filtration and subjected to silica gel column chromatography (eluting solvent : mixture of chloroform:methanol = 20:1) to obtain 0.22 g of the title compound as a slightly yellow powder.
m.p.: 266-268°C (decomp.)
MS spectrum (CI): m/e 428 (M⁺ +1)

| Elemental analysis (%); for C₂₀H₁₈FN₅O₃S·1/2H₂O | | | |
|---|---|---|---|
| Theoretical value; | C: 55.04, | H: 4.39, | N: 16.05 |
| Found value; | C: 54.85, | H: 4.19, | N: 15.92 |

### (Preparation example 101)

### Synthesis of 1-ethyl-6-fluoro-8-difluoromethoxy-7-[(4-hydroxy-3-phenylamino)pyrrolidin-1-yl]-1,4-dihydro-4-oxoquinoline-3-carboxylic acid

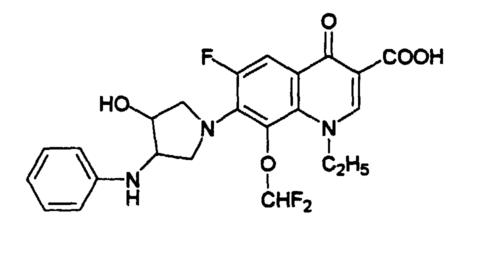

0.9 g (0.0028 mol) of 1-ethyl-6,7-difluoro-8-difluoromethoxy-1,4-dihydro-4-oxoquinoline-3-carboxylic acid, 1.52 g (0.0071 mol) of (4-hydroxy-3-phenylamino)pyrrolidine hydrochloride and 1.6 g (0.0142 mol) of triethylenediamine were added to 20 ml of pyridine, and the mixture was stirred at room temperature for 30 minutes and at 105 to 110°C for 3 hours, followed by evaporation of the solvent under reduced pressure. Water was added to the residue and precipitate was separated from the mixture by filtration and washed with ethanol. The precipitate was subjected to silica gel column chromatography (eluting solvent: chloroform:methanol = 9.5:0.5 mixed solution) to obtain 0.71 g of the title compound as a slightly yellowish white powder.
m.p.: 233-235°C
MS spectrum (CI): m/e 478 (M⁺ +1)

| Elemental analysis (%); for C₂₃H₂₂F₃N₃O₅ | | | |
|---|---|---|---|
| Theoretical value; | C: 57.86, | H: 4.64, | N: 8.80 |
| Found value; | C: 57.83, | H: 4.59, | N: 8.80 |

### (Preparation examples 102 and 103)

Compounds shown in Table 44 were prepared in a similar procedures to that described in Preparation example 101.

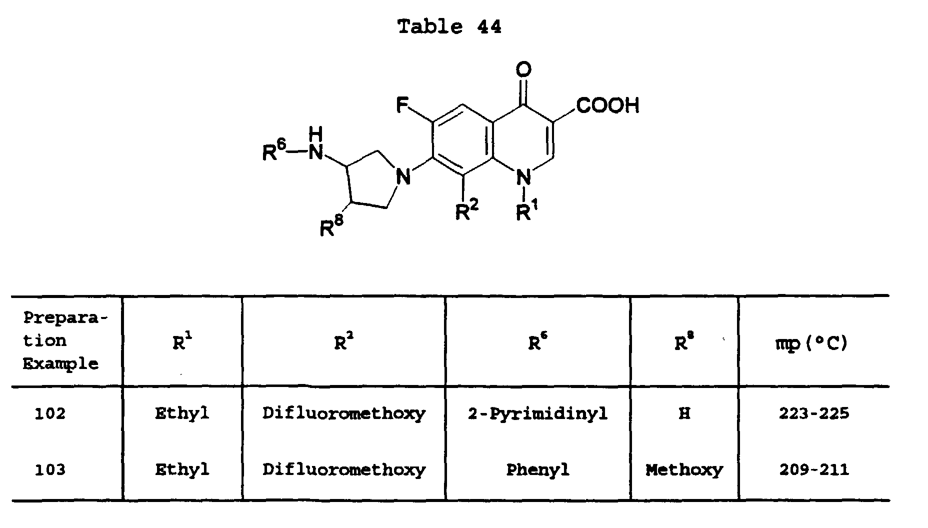

### (Preparation example 104)

### Synthesis of 1-ethyl-6-fluoro-8-difluoromethoxy-7-[4-(2-pyrimidinyl)piperazin-1-yl]-1,4-dihydro-4-oxoquinoline-3-carboxylic acid-2-morpholinoethyl ester

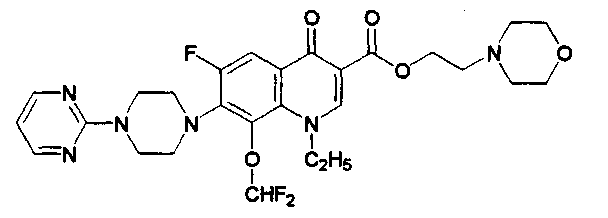

58.94 g (0.127 mol) of 1-ethyl-6-fluoro-8-difluoromethoxy-7-[4-(2-pyrimidinyl)piperazin-1-yl]-1,4-dihydro-4-oxoquinoline-3-carboxylic acid, 25.05 g (0.191 mol) of 4-(2-hydroxyethyl)morpholine, 23.3 g (0.191 mol) of 4-dimethylaminopyridine and 48.8 g (0.254 mol) of 1-ethyl-3-(3-dimethylaminopropyl)carbodiimide hydrochloride were added to 3 liters of methylene chloride, and the mixture was stirred at room temperature for 4 days, followed by evaporation of the solvent under reduced pressure. The residue was dissolved in chloroform, washed with a 1N aqueous hydrochloric acid solution and then with water, and dried, followed by evaporation of the solvent under reduced pressure. The residue was subjected to silica gel column chromatography (eluting solvent: mixture of chloroform:methanol:28% aqueous ammonia=40:9:1) to obtain 50.25 g of the title compound as a slightly yellowish white powder.
m.p.: 162-164°C
MS spectrum (CI): m/e 577 (M⁺ +1)

| Elemental analysis (%); for C₂₇H₃₁F₃N₆O₅·1/2H₂O | | | |
|---|---|---|---|
| Theoretical value; | C: 55.38, | H: 5.34, | N: 14.35 |
| Found value; | C: 55.06, | H: 5.20, | N: 14.26 |

### (Preparation examples 105 to 108)

Compounds shown in Table 45 were prepared in similar procedures to that described in Preparation example 104.

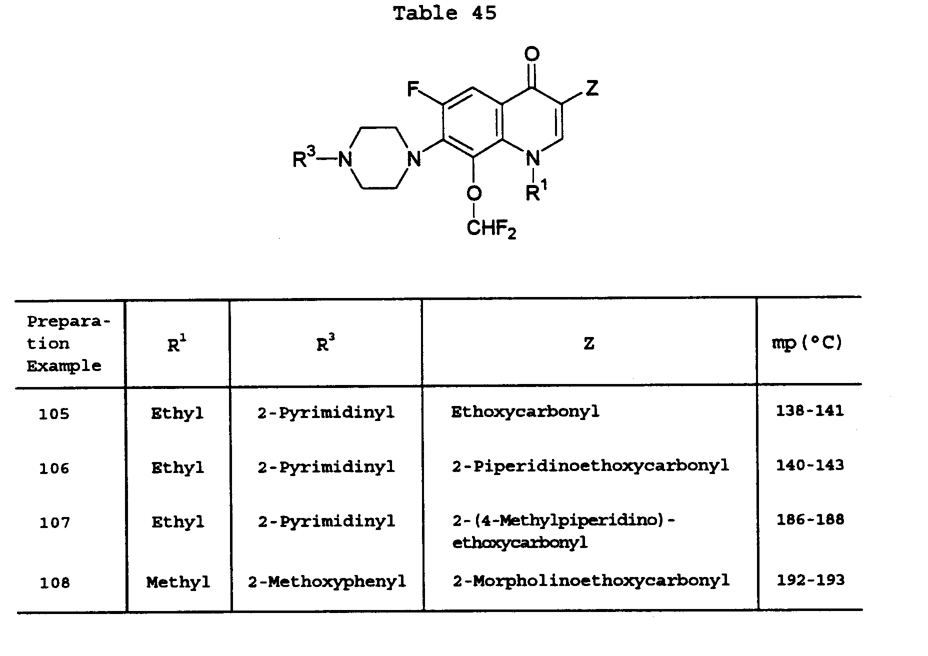

### (Preparation example 109)

### Synthesis of 9-ethyl-6-fluoro-8-difluoromethoxy-7-[4-(2-pyrimidinyl)piperazin-1-yl]-2,3,4,9-tetrahydroisothiazolo[5,4-b]quinoline-3,4-dione

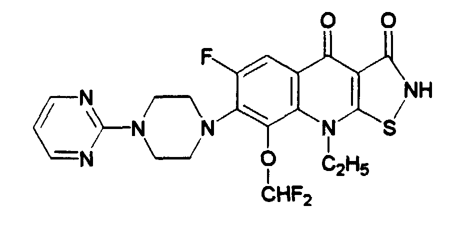

Reaction of 9-ethyl-6,7-difluoro-8-difluoromethoxy-2,3,4,9-tetrahydroisothiazolo[5,4-b]quinoline-3,4-dione (500 mg (1.44 mmol)) with 1-(2-pyrimidinyl)piperazine (1.9 g (11.5 mmol)) and purification of the reaction mixture were carried out in a similar manner to that described in Preparation example 1, to afford 10 mg of the title compound as a pale yellow powder.
m.p.: 259-262°C
MS spectrum (CI): m/e 493 (M⁺ +1)

| Elemental analysis (%); for C₂₁H₁₉F₃N₆O₃S·1/2H₂O | | | |
|---|---|---|---|
| Theoretical value; | C: 50.30, | H: 3.82, | N: 16.75 |
| Found value; | C: 50.56, | H: 3.51, | N: 17.03 |

### (Preparation example 110)

### Synthesis of 1-cyclopropyl-6-fluoro-8-trifluoromethyl-1,4-dihydro-4-oxo-7-[4-(2-pyrimidinyl)piperazin-1-yl]quinoline-3-carboxylic acid

1.0 g (0.003 mol) of 1-cyclopropyl-6,7-difluoro-8-trifluoromethyl-1,4-dihydro-4-oxoquinoline-3-carboxylic acid and 1.23 g (0.0075 mol) of 1-(2-pyrimidinyl)piperazine were dissolved in 20 ml of pyridine, and the solution was stirred at 105°C for 3 hours. The solvent was evaporated under reduced pressure and the residue was subjected to silica gel column chromatography (eluting solution: mixture of chloroform:methanol = 9.5:0.5) to obtain 0.68 g of 1-cyclopropyl-6-fluoro-8-trifluoromethyl-1,4-dihydro-4-oxo-7-[4-(2-pyrimidinyl)piperazin- 1-yl]quinoline-3-carboxylic acid as a yellow powder.
m.p.: 285-287°C
NMR (DMSO-d₆, δ): 0.91 (2H, m), 1.17-1.18 (2H, m), 3.49 (4H, br.s), 3.94 (4H, br.s), 4.07 (1H, m), 6.69-6.71 (1H, t, J=9.3Hz), 8.06-8.09 (1H, d, J=11.7Hz), 8.42-8.43 (2H, d, J=4.4Hz), 8.85 (1H, s), 14.58 (1H, s).
MS spectrum (CI): m/e 478 (M⁺ +1)

### (Preparation examples 111 to 140)

Compounds shown in Table 46 were prepared in similar procedures to that described in Preparation example 110.

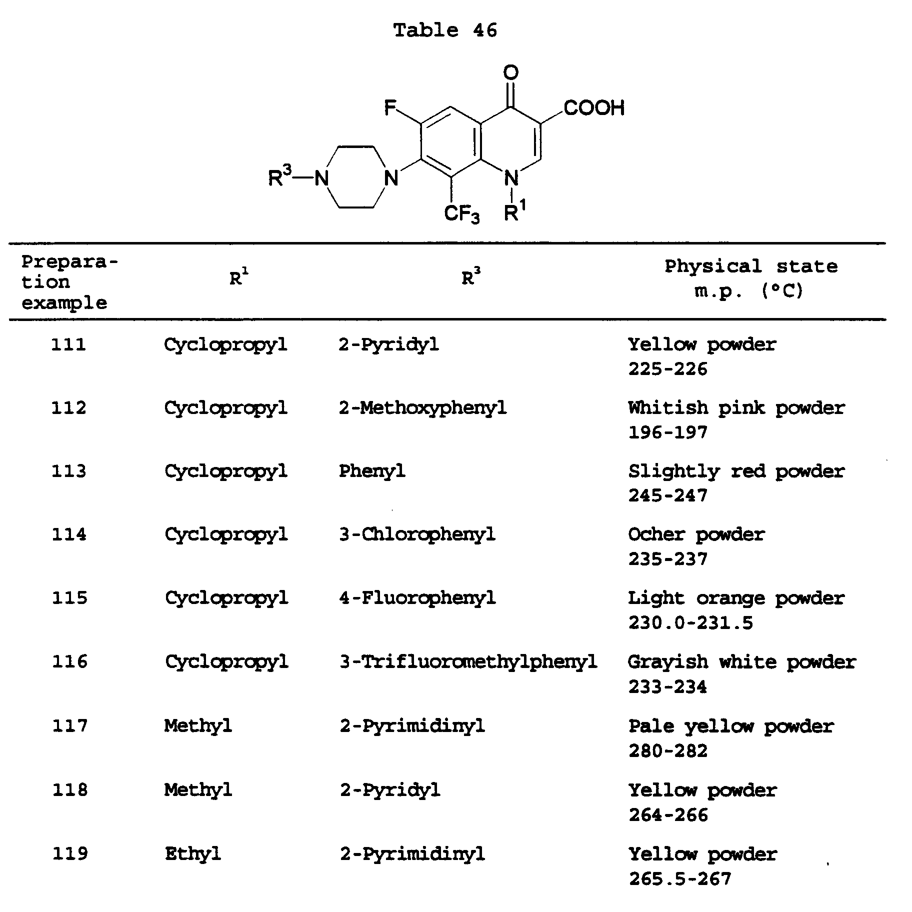

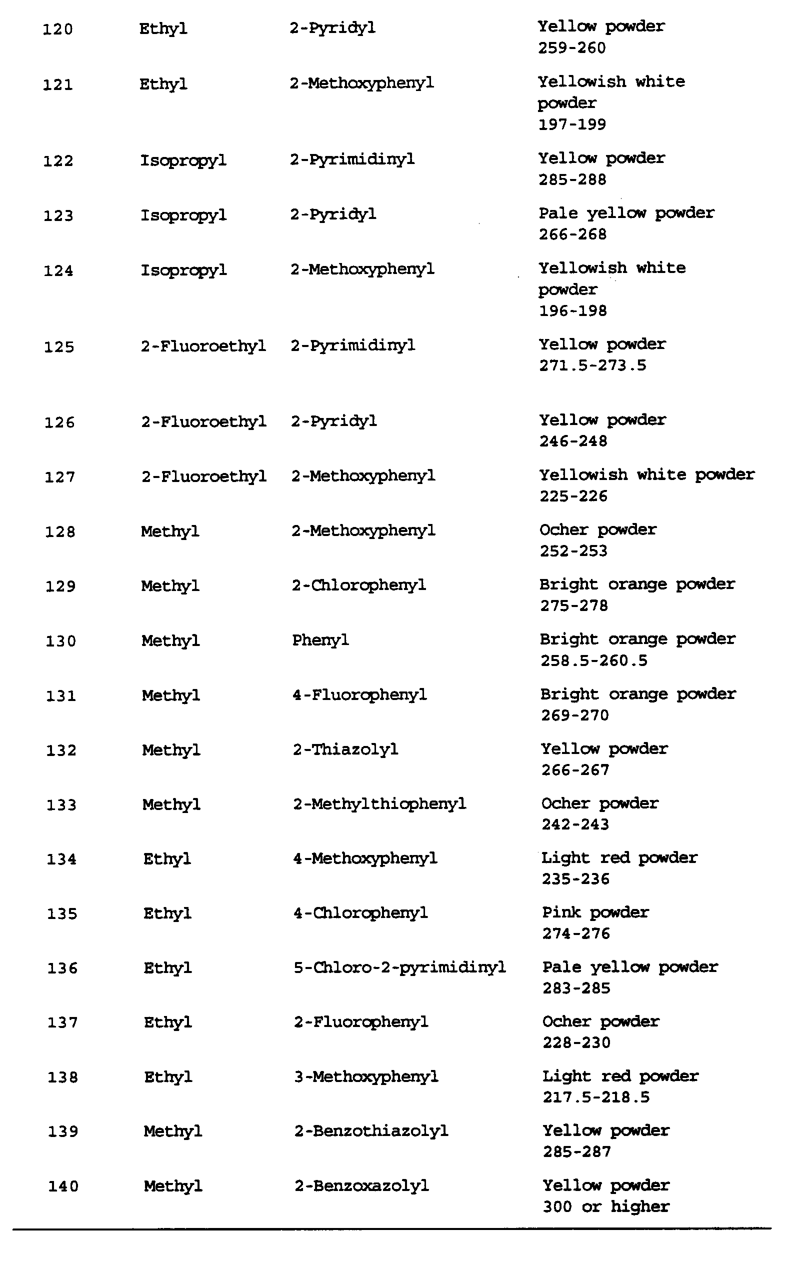

### (Preparation example 141)

### Synthesis of 1-cyclopropyl-6-fluoro-8-trifluoromethyl-1,4-dihydro-4-oxo-7-[4-(2-pyridyl)piperazin-1-yl]quinoline-3-carboxylic acid 2-morpholinoethyl ester

100 mg (0.21 mmol) of 1-cyclopropyl-6-fluoro-8-trifluoromethyl-1,4-dihydro-4-oxo-7-[4-(2-pyridyl)piperazin-1-yl]quinoline-3-carboxylic acid, 0.055 g (0.42 mmol) of 4-(2-hydroxyethyl)morpholine, 0.045 g (0.37 mmol) of 4-dimethylaminopyridine and 0.091 g (0.48 mmol) of 1-ethyl-3-(3-dimethylaminopropyl)carbodiimide hydrochloride were added to 5 ml of methylene chloride, and the mixture was allowed to stand at room temperature for 7 days, followed by evaporation of the solvent under reduced pressure. The residue was subjected to silica gel column chromatography (eluting solvent: mixture of chloroform:methanol:28% aqueous ammonia = 40:9:1) to obtain 60 mg of 1-cyclopropyl-6-fluoro-8-trifluoromethyl-1,4-dihydro-4-oxo-7-[4-(2-pyridyl)piperazin-1-yl]quinoline-3-carboxylic acid 2-morpholinoethyl ester as a pale yellow powder.
m.p.: 203-205°C
MS spectrum (CI): m/e 590 (M⁺ +1)

### (Preparation example 142)

### Synthesis of 6-fluoro-8-trifluoromethyl-1,4-dihydro-4-oxo-1-methyl-7-[3-methyl-4-(2-pyrimidinyl)piperazin-1-yl]quinoline-3-carboxylic acid

1.5 g (0.0049 mol) of 6,7-difluoro-8-trifluoromethyl-1,4-dihydro-1-methyl-4-oxoquinoline-3-carboxylic acid and 1.5 g (0.015 mol) of 2-methylpiperazine were dissolved in 30 ml of pyridine, and the solution was stirred at 105°C for 3 hours, followed by evaporation of the solvent under reduced pressure. Ethanol was added to the residue and precipitate was collected by filtration. The precipitate thus obtained was washed with ethanol and followed by distilling off the solvent to obtain 1.49 g of 6-fluoro-8-trifluoromethyl-1,4-dihydro-4-oxo-1-methyl-7-(3-methylpiperazin-1-yl)quinoline-3-carboxylic acid as a yellow powder.

1.49 g (0.0039 mol) of the 6-fluoro-8-trifluoromethyl-1,4-dihydro-4-oxo-1-methyl-7-(3-methylpiperazin-1-yl)quinoline-3-carboxylic acid thus obtained, 0.88 g (0.0077 mol) of 2-chloropyrimidine and 0.78 g (0.0077 mol) of triethylamine were added to 20 ml of N,N-dimethylformamide, and the mixture was stirred at 130°C for 10 hours, followed by evaporation of the solvent under reduced pressure.

The residue was subjected to silica gel column chromatography (eluting solution: mixture of chloroform:methanol = 9:1) to obtain 0.35 g of 6-fluoro-8-trifluoromethyl-1,4-dihydro-4-oxo-1-methyl-7-[3-methyl-4-(2-pyrimidinyl)piperazin-1-yl]quinoline-3-carboxylic acid as an ocher powder.
m.p.: 283-284.5°C
MS spectrum (CI): m/e 466 (M⁺ +1)

| Elemental analysis (%); for C₂₁H₁₉F₄N₅O₃·1/2H₂O | | | |
|---|---|---|---|
| Theoretical value; | C: 53.17, | H: 4.25, | N: 14.76 |
| Found value; | C: 53.47, | H: 4.07, | N: 14.95 |

### (Preparation example 143)

### Synthesis of 6-fluoro-8-trifluoromethyl-1,4-dihydro-4-oxo-1-methyl-7-[4-(2-pyrimidinyl)homopiperazin-1-yl]quinoline-3-carboxylic acid

0.8 g (0.0026 mol) of 6,7-difluoro-8-trifluoromethyl-1,4-dihydro-1-methyl-4-oxoquinoline-3-carboxylic acid and 2.1 g (0.0118 mol) of 1-(2-pyrimidinyl)homopiperazine were dissolved in 12 ml of pyridine, and the solution was stirred at 105°C for 3 hours, followed by evaporation of the solvent under reduced pressure. The residue was subjected to silica gel column chromatography (eluting solution: mixture of chloroform:methanol =9.5:0.5) to obtain 0.45 g of 6-fluoro-8-trifluoromethyl-1,4-dihydro-4-oxo-1-methyl-7-[4-(2-pyrimidinyl)homopiperazin-1- yl]quinoline-3-carboxylic acid as a yellow powder.
m.p.: 243-245°C
MS spectrum (CI): m/e 466 (M⁺ +1)

| Elemental analysis (%); for C₂₁H₁₉F₄N₅O₃ | | | |
|---|---|---|---|
| Theoretical value; | C: 54.20, | H: 4.11, | N: 15.05 |
| Found value; | C: 54.06, | H: 4.03, | N: 14.96 |

### (Preparation example 144)

### Synthesis of 1-ethyl-6-fluoro-8-difluoromethoxy-1,4-dihydro-4-oxo-7-[4-(2-benzothiazolyl)piperazin-1-yl]quinoline-3-carboxylic acid

Reaction of 1-ethyl-6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 246-247 (ocher powder)

### (Preparation example 145)

### Synthesis of 1-cyclopropyl-6-fluoro-8-methoxy-1,4-dihydro-4-oxo-7-[4-(2-benzothiazolyl)piperazin-1-yl]quinoline-3-carboxylic acid

Reaction of 1-cyclopropyl-6,7-difluoro-8-methoxy-1,4-dihydro-4-oxoquinoline-3-carboxylic acid with 1-(2-benzothiazolyl)piperazine and purification of the reaction mixture were carried out in a similar manner to that described in Preparation example 63 to afford the title compound.
m.p. (°C): 248.5-249.5 (white powder)

### (Preparation example 146)

### Synthesis of 1-benzyl-6-fluoro-8-difluoromethoxy-1,4-dihydro-4-oxo-7-[4-(2-benzothiazolyl)piperazin-1-yl]quinoline-3-carboxylic acid

Reaction of 1-benzyl-6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 283-285 (white powder)

### (Preparation example 147)

### Synthesis of 1-methyl-6-fluoro-8-difluoromethoxy-1,4-dihydro-4-oxo-7-[4-(2-benzothiazolyl)piperazin-1-yl]quinoline-3-carboxylic acid

Reaction of 1-methyl-6,7-difluoro-8-difluoromethoxy-1,4-dihydro-4-oxoyinoline-3-carboxylic acid with 1-(2-benzothiazolyl)piperazine and purification of the reaction mixture were carried out in a similar manner to that described in Preparation example 1 to afford the title compound.
m.p. (°C): 251-253 (light yellow powder)

### (Preparation example 148)

### Synthesis of 1-methyl-6-fluoro-8-difluoromethoxy-1,4-dihydro-4-oxo-7-[4-(6-chloro-2-benzothiazolyl)piperazin-1-yl]quinoline-3-carboxylic acid

Reaction of 1-methyl-6,7-difluoro-8-difluoromethoxy-1,4-dihydro-4-oxoquinoline-3-carboxylic acid with 1-(6-chloro-2-benzothiazolyl)piperazine and purification of the reaction mixture were carried out in a similar manner to that described in Preparation example 1 to afford the title compound.
m.p. (°C): 300 or higher (yellow crystal, 3/2 H₂O)

### (Preparation example 149)

### Synthesis of 1-methyl-6-fluoro-8-difluoromethoxy-1,4-dihydro-4-oxo-7-[4-(6-methoxy-2-benzothiazolyl)piperazin-1-yl]quinoline-3-carboxylic acid

Reaction of 1-methyl-6,7-difluoro-8-difluoromethoxy-1,4-dihydro-4-oxoquinoline-3-carboxylic acid with 1-(6-methoxy-2-benzothiazolyl)piperazine and purification of the reaction mixture were carried out in a similar manner to that described in Preparation example 1 to afford the title compound.
m.p. (°C): 244-245 (yellow crystal, 1/2 H₂O)

### (Preparation example 150)

### Synthesis of 5-amino-1-cyclopropyl-6-fluoro-8-difluoromethoxy-1,4-dihydro-4-oxo-7-[4-(2-benzothiazolyl)piperazin-1-yl]quinoline-3-carboxylic acid

Reaction of 5-amino-1-cyclopropyl-6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 300 or higher (yellow powder)

### (Preparation example 151)

### Synthesis of 1-(2-hydroxyethyl)-6-fluoro-8-difluoromethoxy-1,4-dihydro-4-oxo-7-[4-(2-benzothiazolyl)piperazin-1-yl]quinoline-3-carboxylic acid

Reaction of 1-(2-hydroxyethyl)-6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 254.5-256.5 (yellow powder)

### (Preparation example 152)

### Synthesis of 1-(2-methoxyethyl)-6-fluoro-8-difluoromethoxy-1,4-dihydro-4-oxo-7-[4-(2-benzothiazolyl)piperazin-1-yl]quinoline-3-carboxylic acid

Reaction of 1-(2-methoxyethyl)-6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 229.5-231.0 (white powder)

### (Preparation example 153)

### Synthesis of 6-fluoro-8-difluoromethoxy-1,4-dihydro-4-oxo-7-[4-(2-benzothiazolyl)piperazin-1-yl]quinoline-3-carboxylic acid

Reaction of 6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 263-265 (ocher powder)

### (Preparation example 154)

### Synthesis of 1-cyclopropyl-6-fluoro-8-methoxy-1,4-dihydro-4-oxo-7-[4-(2-benzoxazolyl)piperazin-1-yl]quinoline-3-carboxylic acid

Reaction of 6,7-difluoro-8-difluoromethoxy-1,4-dihydro-4-oxoquinoline-3-carboxylic acid with 1-(2-benzothiazolyl)piperazine and purification of the reaction mixture were carried out in a similar manner to that described in Preparation example 1 to afford the title compound.
m.p. (°C): 246-248 (slight yellowish brown powder)

### (Preparation example 155)

### Synthesis of 1-cyclopropyl-6-fluoro-8-difluoromethyl-1,4-dihydro-4-oxo-7-[4-(2-pyrimidinyl)piperazin-1-yl)quinoline-3-carboxylic acid

Reaction of 1-cyclopropyl-6,7-difluoro-8-difluoromethyl-1,4-dihydro-4-oxoquinoline-3-carboxylic acid with 1-(2-pyrimidinyl)piperazine and purification of the reaction mixture were carried out in a similar manner to that described in Preparation example 1 to afford the title compound.
m.p. (°C): 264-265 (yellow crystal)

### (Preparation example 156)

### Synthesis of 1-methylamino-6-fluoro-8-difluoromethoxy-1,4-dihydro-4-oxo-7-[4-(2-benzoxazolyl)piperazin-1-yl]quinoline-3-carboxylic acid

Reaction of 1-methylamino-6,7-difluoro-8-difluoromethoxy-1,4-dihydro-4-oxoquinoline-3-carboxylic acid with 1-(2-benzoxazolyl)piperazine and purification of the reaction mixture were carried out in a similar manner to that described in Preparation example 1 to afford the title compound.
m.p. (°C): 227-229 (slight yellowish white powder)

### (Preparation example 157)

### Synthesis of 9-fluoro-3-fluoromethyl-7-oxo-10-[4-(2-benzothiazolyl)piperazin-1-yl]-2,3-dihydro-7H-pyrido[1,2,3-de][1,4]benzoxazine-6-carboxylic acid

Reaction of 9,10-difluoro-3-fluoromethyl-7-oxo-2,3-dihydro-7H-pyrido[1,2,3-de][1,4]benzoxazine-6-carboxylic acid with 1-(2-benzothiazolyl)piperazine and purification of the reaction mixture were carried out in a similar manner to that described in Preparation example 63 to afford the title compound.
m.p. (°C): 280-282 (yellow powder)

### (Preparation example 158)

### Synthesis of 1-methyl-6-fluoro-8-trifluoromethyl-1,4-dihydro-4-oxo-7-[4-(2-pyrazinyl)piperazin-1-yl]quinoline-3-carboxylic acid

Reaction of 1-methyl-6,7-difluoro-8-trifluoromethyl-1,4-dihydro-4-oxoquinoline-3-carboxylic acid with 1-(2-pyrazinyl)piperazine and purification of the reaction mixture were carried out in a similar manner to that described in Preparation example 110 to afford the title compound.
m.p. (°C): 285-287 (yellow powder)

### (Preparation example 159)

### Synthesis of 1-methyl-6-fluoro-8-trifluoromethyl-1,4-dihydro-4-oxo-7-[4-(3-benzoisothiazolyl)piperazin-1-yl]quinoline-3-carboxylic acid

Reaction of 1-methyl-6,7-difluoro-8-trifluoromethyl-1,4-dihydro-4-oxoquinoline-3-carboxylic acid with 1-(3-benzoisothiazolyl)piperazine and purification of the reaction mixture were carried out in a similar manner to that described in Preparation example 1 to afford the title compound.
m.p. (°C): 296-298 (yellow powder)

### (Preparation example 160)

### Synthesis of 1-(3-aminopropyl)-6-fluoro-8-trifluoromethyl-7-[4-(2-pyrimidinyl)piperazin-1-yl]-1,4-dihydro-4-oxoquinoline-3-carboxylic acid

### 160-1)

7.56 g (0.0158 mol) of ethyl 1-(3-tert-butoxycarbonylaminopropyl)-6,7-difluoro-8-trifluoromethyl-1,4-dihydro-4-oxoquinoline-3-carboxylate, 9.1 g (0.0554 mol) of 1-(2-pyrimidinyl)piperazine and 12.3 g (0.095 mol) of N-ethyldiisopropylamine were dissolved in 50 ml of acetonitrile, and the solution was refluxed with stirring for 48 hours. The solvent was evaporated under reduced pressure and the residue was subjected to silica gel column chromatography (eluting solution: mixture of ethyl acetate:toluene = 7:3) to obtain 2.2 g of ethyl 1-(3-tert-butoxycarbonylaminopropyl)-6-fluoro-8-trifluoromethyl-7-[4-(2-pyrimidinyl)piperazin-1-yl]-1,4-dihydro-4-oxoquinoline-3-carboxylate as a yellow powder.
m.p. (°C): 188-190

### 160-2)

To a solution of ethyl 1-(3-tert-butoxycarbonylaminopropyl)-6-fluoro-8-trifluoromethyl-7-[4-(2-pyrimidinyl)piperazin-1-yl]-1,4-dihydro-4-oxoquinoline-3-carboxylate (2.2 g (0.0035 mol)) in acetonitrile (50 ml) was added 21.2 ml of a 1N aqueous sodium hydroxide solution, and the mixture was refluxed with stirring for 1 hour. After cooling the reaction mixture, 21.2 ml of a 1N aqueous hydrochloric acid solution was added thereto, followed by evaporation of the solvent under reduced pressure. The residue was subjected to silica gel column chromatography (eluting solution: mixture of chloroform:methanol = 9:1) to obtain 2.04 g of 1-(3-tert-butoxycarbonylaminopropyl)-6-fluoro-8-trifluoromethyl-7-[4-(2-pyrimidinyl)piperazin-1-yl]-1,4-dihydro-4-oxoquinoline-3-carboxylic acid as a yellow powder.
m.p. (°C): 246-248

### 160-3)

21.5 ml of trifluoroacetic acid was added to a solution of 1-(3-tert-butoxycarbonylaminopropyl)-6-fluoro-8-trifluoromethyl-7-[4-(2-pyrimidinyl)piperazin-1-yl]-1,4-dihydro-4-oxoquinoline-3-carboxylic acid (2.04 g (0.0034 mol)) in 50 ml of methylene chloride, and the mixture was stirred at room temperature for 1 hour. After the solvent was evaporated under reduced pressure, water was added to the residue, and the pH of the mixture was adjusted to 7.5 with a saturated aqueous sodium hydrogencarbonate solution. Precipitate was separated from the mixture by filtration and washed with water to obtain 1.36 g of 1-(3-aminopropyl)-6-fluoro-8-difluoromethyl-1,4-dihydro-4-oxo-7-[4-(2-pyrimidinyl)piperazin-1-yl]quinoline-3-carboxylic acid as a slightly yellow powder.
m.p. (°C): 243-245

### (Preparation example 161)

### Synthesis of 1-methyl-6-fluoro-8-trifluoromethyl-1,4-dihydro-4-oxo-7-[4-(2-benzoxazolyl)-3-methylpiperazin-1-yl]quinoline-3-carboxylic acid

Reaction of 1-methyl-6,7-difluoro-8-trifluoromethyl-1,4-dihydro-4-oxoquinoline-3-carboxylic acid with 2-methyl-1-(2-benzoxazolyl)piperazine and purification of the reaction mixture were carried out in a similar manner to that described in Preparation example 1 to afford the title compound.
m.p. (°C): 300 or higher (pale yellow powder)

### (Reference example 1)

### Synthesis of 6,7-difluoro-8-difluoromethoxy-1-(2-pyridylmethyl)-1,4-dihydro-4-oxoquinoline-3-carboxylic acid

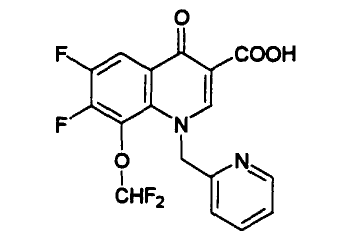

30 g (0.124 mol) of 2,4,5-trifluoro-3-difluoromethoxybenzoic acid was dissolved in 37 ml of toluene, and 35 ml of thionyl chloride and 0.5 ml of N,N-dimethylformamide were added thereto, and the resulting mixture was heated under reflux for 4 hours. After completion of the reaction, toluene and excess thionyl chloride were evaporated under reduced pressure to obtain 2,4,5-trifluoro-3-difluoromethoxybenzoic chloride.

Meanwhile, a suspension of ethoxy magnesium malonic acid diethyl ester in tetrahydrofuran was obtained by reflux 15.5 g (0.135 mol) of magnesium ethoxide and 20.9 g (0.130 mol) of diethyl malonate in 100 ml of anhydrous tetrahydrofuran with stirring for 2.5 hours. A solution of the above-mentioned acid chloride in 20 ml of tetrahydrofuran was added dropwise to the suspension with stirring at room temperature, and further the mixture was stirred at room temperature for 2 hours. 100 ml of 1N hydrochloric acid was added to the reaction mixture and the resulting mixture was vigorously stirred, followed by separation. After the organic layer was washed with water and dried over anhydrous sodium sulfate, the solvent was evaporated under reduced pressure to obtain 51.8 g of 2,4,5-trifluoro-3-difluoromethoxybenzoylmalonic acid diethyl ester as a pale red liquid.
- MS spectrum (CI): m/e: 385 (M⁺ + 1)
339 (M⁺ -OC₂H₅)

Then, the compound thus obtained was dissolved in 200 ml of dioxane, and 23.6 g (0.124 mol) of p-toluenesulfonic acid · mono hydrate was added thereto, and the resulting mixture was heated under reflux for 8.5 hours. The reaction mixture was concentrated under reduced pressure, water and 10.41 g (0.124 mol) of sodium hydrogencarbonate were added to the residue, and the resulting mixture was extracted with ethyl acetate. The ethyl acetate layer was washed with water and dried over anhydrous sodium sulfate and then concentrated under reduced pressure to obtain 32.0 g of 2,4,5-trifluoro-3-difluoromethoxybenzoylacetic acid ethyl ester as a yellow liquid.
- MS spectrum (CI): m/e: 313 (M⁺ + 1)
225 (M⁺ -CH₂COO₂H₅)

11 ml of acetic anhydride and 3.2 ml of ethyl orthoformate were added to 4.85 g (0.0155 mol) of the thus obtained 2,4,5-trifluoro-3-difluoromethoxybenzoylacetic acid ethyl ester, and the mixture was heated under reflux for 2 hours, followed by evaporation of excess acetic anhydride and ethyl orthoformate under reduced pressure. The residue was dissolved in 150 ml of dichloromethane, and 2.01 g (0.0186 mol) of 2-aminomethylpyridine was added dropwise thereto under ice-cooling and stirring, and the resulting mixture was stirred under ice-cooling for 1 hour. The reaction mixture was concentrated under reduced pressure and the residue was subjected to silica gel column chromatography (eluting solution: mixture of toluene:ethyl acetate = 9:1) to obtain 6.56 g of 2-(2,4,5-trifluoro-3-difluoromethoxybenzoyl)-3-(2-pyridylmethylamino)acrylic acid ethyl ester as an amber liquid. The compound thus obtained was recrystallized from n-hexane to obtain 3.6 g of white crystals.
MS spectrum (CI): m/e 431 (M⁺ + 1)

5.6 g (0.013 mol) of the thus obtained 2-(2,4,5-trifluoro-3-difluoromethoxybenzoyl)-3-(2-pyridylmethylamino)acrylic acid ethyl ester was dissolved in 50 ml of N,N-dimethylformamide, and 2.2 g (0.026 mol) of sodium hydrogencarbonate was added thereto, and the resulting mixture was stirred at 120°C for 30 minutes. The reaction mixture was poured to 200 ml of water to collect precipitate crystals by filtration. The precipitate was washed with water and ethanol and distilled off the solvent to obtain 2.6 g of 6,7-difluoro-8-difluoromethoxy-1-(2-pyridylmethyl)-1,4-dihydro-4-oxoquinoline-3-carboxylic acid ethyl ester as yellow crystals.
MS spectrum (CI): m/e 411 (M⁺ + 1)

Then, 1.7 g (0.0041 mol) of the ester was suspended in a mixture of 4.2 ml of acetic acid, 1.5 ml of water and 0.48 ml of conc. sulfuric acid, and the suspension was heated under reflux with stirring for 2 hours. The reaction mixture was cooled to room temperature, and water was added thereto to collect precipitate by filtration. The precipitate was washed with water and distilled off water to obtain 1.1 g of 6,7-difluoro-8-difluoromethoxy-1-(2-pyridylmethyl)-1,4-dihydro-4-oxoquinoline-3-carboxylic acid as white crystals.
m.p.: 207-213°C (decomp.)
MS spectrum (CI): m/e 383 (M⁺ + 1)

### (Reference examples 2 to 9)

Compounds shown in Table 47 were prepared in a similar manner to that described in Reference example 1.

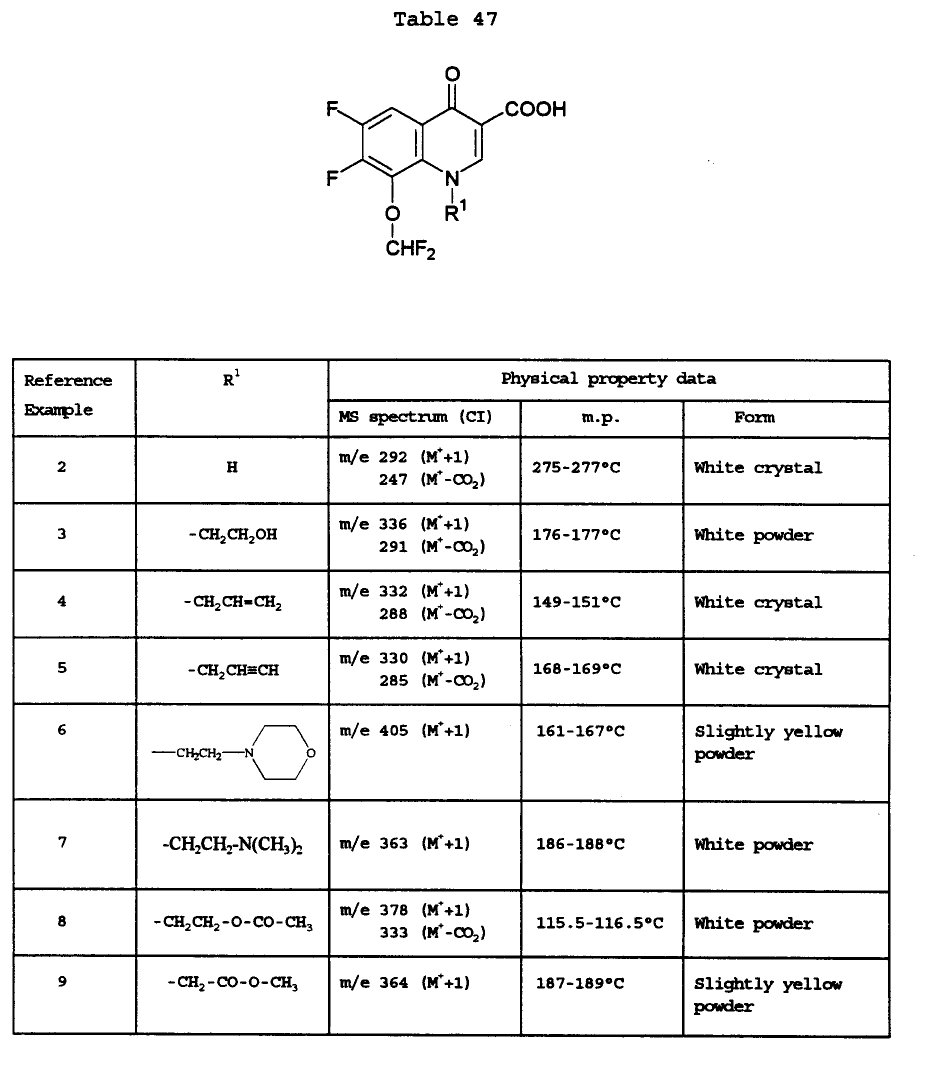

### (Reference example 10)

### Synthesis of 1-ethyl-7-fluoro-8-difluoromethoxy-1,4-dihydro-4-oxoquinoline-3-carboxylic acid

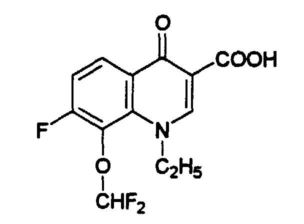

26.4 g (0.168 mol) of 2-fluoro-6-nitrophenol and 17.2 g (0.168 mol) of acetic anhydride were dissolved in 290 ml of acetic acid, and 3.0 g of 5% palladium/carbon was added thereto, and hydrogen gas was introduced to the resulting mixture while stirring at room temperature for 2 hours. The reaction mixture was filtered and the filtrate was concentrated under reduced pressure, followed by extraction of the residue with ethyl acetate. The ethyl acetate layer was washed with water and dried over anhydrous sodium sulfate. The solvent was evaporated under reduced pressure and the residue was subjected to silica gel column chromatography (eluting solution: mixture of ethyl acetate:toluene = 1:2) to obtain 25.9 g of 2-acetylamino-6-fluorophenol as brown crystals.
- MS spectrum (CI): m/e: 170 (M⁺ + 1)
127 (M⁺ + 1 -COOH₃)

25.9 g (0.153 mol) of the 2-acetylamino-6-fluorophenol thus obtained was dissolved in 110 ml of N,N-dimethylformamide, and 25.4 g (0.184 mol) of potassium carbonate and 33.1 g (0.383 mol) of chlorodifluoromethane were added thereto, and the resulting mixture was stirred at 100°C for 5 hours in an autoclave. After completion of the reaction, the reaction mixture was poured to 1 liter of water and extracted with ethyl acetate. The ethyl acetate layer was washed with water and dried over anhydrous sodium sulfate, followed by evaporation of the solvent under reduced pressure. The residue was subjected to silica gel column chromatography (eluting solution : mixture of ethyl acetate:toluene = 1:3) to obtain 30.4 g of 3-fluoro-2-difluoromethoxyacetoanilide as slightly brown crystals.
MS spectrum (CI): m/e 220 (M⁺ +1)

30.3 g (0.138 mol) of the thus obtained 3-fluoro-2-difluoromethoxyacetoanilide was dissolved in 180 ml of ethyl alcohol, and 50.3 ml of conc. hydrochloric acid was added thereto, followed by heating under reflux for 3 hours. After completion of the reaction, ethyl alcohol and conc. hydrochloric acid were evaporated under reduced pressure. 200 ml of water was added to the residue, and the mixture was neutralized with potassium carbonate and extracted with chloroform. The chloroform layer was washed with a saturated NaCl solution and dried over anhydrous sodium sulfate, followed by evaporation of the solvent under reduced pressure. The residue was subjected to silica gel column chromatography (eluting solvent: chloroform) to obtain 22.4 g of 3-fluoro-2-difluoromethoxyaniline as a red livid.

A mixture of 22.4 g (0.127 mol) of the 3-fluoro-2-difluoromethoxyaniline thus obtained and 27.4 g (0.127 mol) of diethyl ethoxymethylenemalonate was heated at 120°C for 5 hours. The mixture was cooled to room temperature, and n-hexane was added to the mixture, followed by filtration. The precipitate collected by filtration was washed with n-hexane and disilled off the solvent to obtain 37.5 g of N-(2,2-diethoxycarbonylvinyl)-3-fluoro-2-difluoromethoxyaniline as a white powder.

A mixture of 10.0 g (0.029 mol) of the thus obtained N-(2,2-diethoxycarbonylvinyl)-3-fluoro-2-difluoromethoxyaniline and 70 ml of diphenyl ether was heated under reflux for 30 minutes. After the mixture was cooled to room temperature, n-hexane was added to the mixture, and then precipitate was collected by filtration and distilled off the solvent to obtain 5.25 g of 7-fluoro-8-difluoromethoxy-4-hydroxyquinoline-3-carboxylic acid ethyl ester as a white powder.
m.p.: 218-219°C
MS spectrum (CI): m/e 302 (M⁺ +1)

3.0 g (0.01 mol) of the thus obtained 7-fluoro-8-difluoromethoxy-4-hydroxyquinoline-3-carboxylic acid ethyl ester was dissolved in 96 ml of N,N-dimethylformamide, and 6.9 g (0.05 mol) of potassium carbonate and 12.5 g (0.08 mol) of ethyl iodide were added thereto, followed by stirring of the mixture at 100°C for 14 hours. After completion of the reaction, the solvent was evaporated under reduced pressure, and water was added to the residue. Precipitate was collected by filtration and subjected to silica gel column chromatography (eluting solution: mixture of chloroform:methanol = 95:5) to obtain 1.1 g of 1-ethyl-7-fluoro-8-difluoromethoxy-1,4-dihydro-4-oxoquinoline-3-carboxylic acid ethyl ester as white crystals.
m.p.: 214.5-215.5°C
MS spectrum (CI): m/e 329 (M⁺ +1)

Then, 1.1 g (0.0033 mol) of the ester was suspended in a mixture of 9 ml of acetic acid, 6.6 ml of water and 1.2 ml of conc. sulfuric acid, and the suspension was heated under reflux with stirring for 2 hours. After the reaction mixture was cooled to room temperature, water was added to the mixture, and then precipitate was collected by filtration. The precipitate was washed with water and distilled off water to obtain 0.85 g of 1-ethyl-7-fluoro-8-difluoromethoxy-1,4-dihydro-4-oxoquinoline-3-carboxylic acid as white crystals.
m.p.: 169-170°C
MS spectrum (CI): m/e 302 (M⁺ +1)

### (Reference example 11)

### Synthesis of 3-cyano-1-ethyl-6,7-difluoro-8-difluoromethoxy-1,4-dihydro-4-oxoquinoline

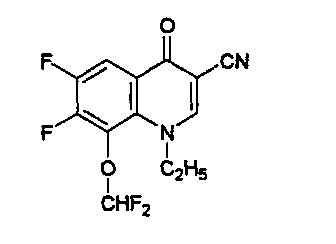

45 ml of toluene, 15 ml of thionyl chloride and 0.2 ml of N,N-dimethylformamide were added to 36.0 g (0.15 mol) of 2,4,5-trifluoro-3-difluoromethoxybenzoic acid, and the mixture was heated under reflux for 4 hours. After completion of the reaction, toluene and excess thionyl chloride were evaporated under reduced pressure to obtain 2,4,5-trifluoro-3-difluoromethoxybenzoic chloride.

15.2 g (0.158 mol) of 3-dimethylaminoacrylonitrile was dissolved in 75 ml of anhydrous tetrahydrofuran, and 16.7 g (0.165 mol) of triethylamine was added to the resulting solution. A solution of the above-mentioned acid chloride in 15 ml of anhydrous tetrahydrofuran was gradually added dropwise to the mixture at room temperature. After completion of the addition, the resulting mixture was heated under reflux for 1 hour and cooled to room temperature, followed by filtration. 9.7 ml of triethylamine and 14.7 g (0.18 mol) of ethylamine hydrochloride were added to the filtrate, and the mixture was stirred at 40°C for 2 hours. The mixture was cooled to room temperature and filtrated. The filtrate was concentrated under reduced pressure and the residue was subjected to silica gel column chromatography (eluting solution: mixture of ethyl acetate:toluene = 3:7) to obtain 39.3 g of 2-(2,4,5-trifluoro-3-difluoromethoxybenzoyl)-3-ethylaminoacrylonitrile as a red liquid. The red liquid was dissolved in 700 ml of a mixture of anhydrous diethyl ether and tetrahydrofuran, and 4.9 g (0.123 mol) of 60% sodium hydride-mineral oil was gradually added to the mixture under ice-cooling, followed by stirring of the mixture at the same temperature for 1 hour. 120 ml of 1N hydrochloric acid was added to the reaction mixture and the resulting mixture was vigorously stirred to acidify the whole reaction mixture. The precipitate was collected by filtration and washed with water and then with diethyl ether to obtain 17.2 g of 3-cyano-1-ethyl-6,7-difluoro-8-difluoromethoxy-1,4-dihydro-4-oxoquinoline as a white powder.
m.p.: 194-195°C
MS spectrum (CI): m/e 301 (M⁺ +1)

### (Reference example 12)

### Synthesis of 1-(4,6-dimethoxy-2-pyrimidinyl)piperazine

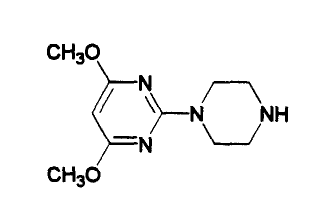

10 g (0.0567 mol) of 1-benzylpiperazine was dissolved in 100 ml of acetonitrile, and 11.7 g (0.085 mol) of potassium carbonate and 14.8 g (0.068 mol) of 4,6-dimethoxy-2-methylsulfonylpyrimidine were added thereto, followed by heating under reflux for 5 hours. The reaction mixture was cooled to room temperature and filtered. The filtrate was concentrated under reduced pressure and the residue was subjected to silica gel column chromatography (eluting solution: mixture of ethyl acetate:toluene = 1:9) to obtain 17.6 g of 4-(4,6-dimethoxy-2-pyrimidinyl)-1-benzylpiperazine as a colorless liquid.
MS spectrum (CI): m/e 315 (M⁺ +1)

3.9 g (0.0125 mol) of the thus obtained 4-(4,6-dimethoxy-2-pyrimidinyl)-1-benzylpiperazine was dissolved in 110 ml of ethanol, and 2.5 g of 5% palladium/carbon was added thereto. Hydrogen gas was introduced to the resulting mixture while heating for 6 hours under reflux. The reaction mixture was cooled to room temperature and filtered. The filtrate was concentrated under reduced pressure and the residue was subjected to silica gel column chromatography (eluting solution: mixture of chloroform:methanol = 9:1) to obtain 1.38 g of 1-(4,6-dimethoxy-2-pyrimidinyl)piperazine as pale red crystals.
MS spectrum (CI): m/e 225 (M⁺ +1)

### (Reference example 13)

### Synthesis of 1-(6-methoxy-2-pyridyl)piperazine

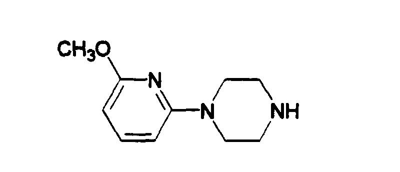

Reaction of piperazine(8.6 g) with 2-chloro-6-methoxypyridine (2.9 g, 0.02 mol)) was carried out at 150°C for 4 hours in a sealed tube. After completion of the reaction, the reaction mixture was subjected to silica gel column chromatography (eluting solution: mixture of chloroform:methanol:28% aqueous ammonia = 40:9:1) to obtain 2.4 g of 1-(6-methoxy-2-pyridyl)piperazine as a pale yellow liquid.
MS spectrum (CI): m/e 194 (M⁺ +1)

### (Reference example 14)

### Synthesis of 1-(2-thiazolyl)piperazine

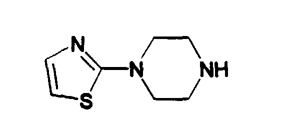

5.0 g (0.0305 mol) of 2-bromothiazole was dissolved in 50 ml of acetonitrile, and 13.1 g (0.153 mol) of piperazine, 8.4 g (0.061 mol) of potassium carbonate and a catalytic amount of potassium iodide were added to the resulting solution, followed by heating under reflux for 5 hours. The reaction mixture was cooled to room temperature and filtered. The filtrate was concentrated under reduced pressure and the residue was subjected to silica gel column chromatography (eluting solution: mixture of chloroform:methanol:28% aqueous ammonia = 40:9:1) to obtain 3.62 g of 1-(2-thiazolyl)piperazine as a colorless liquid.
MS spectrum (CI): m/e 170 (M⁺ +1)

### (Reference example 15)

### Synthesis of 1-(3-amino-2-pyridyl)piperazine

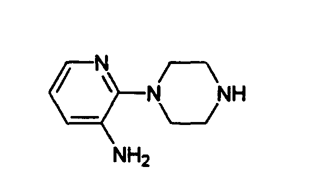

After 3.13 g (0.015 mol) of 1-(3-nitro-2-pyridyl)piperazine was dissolved in 30 ml of methanol, 2 g of 5% palladium/carbon was added to the solution, and hydrogen gas was introduced to the resulting mixture for 1 hour with stirring at room temperature. The reaction mixture was filtered and the filtrate was concentrated under reduced pressure to obtain 2.56 g of 1-(3-amino-2-pyridyl)piperazine as a pale yellow liquid.
MS spectrum (CI): m/e 179 (M⁺ +1)

### (Reference example 16)

### Synthesis of 1-(2-benzoxazolyl)piperazine

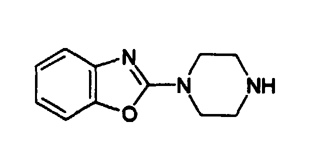

33.6 g (0.39 mol) of piperazine and 10.0 g (0.065 mol) of 2-chlorobenzoxazole were dissolved in 200 ml of acetonitrile, and 9.0 g (0.065 mol) of potassium carbonate and a catalytic amount of potassium iodide were added thereto, followed by heating under reflux with stirring for 11 hours. The reaction mixture was cooled to room temperature and filtered. The filtrate was concentrated under reduced pressure and the residue was subjected to silica gel column chromatography (eluting solution: mixture of chloroform:methanol = 9:1) to obtain 7.54 g of 1-(2-benzoxazolyl)piperazine as white crystals.
MS spectrum (CI): m/e 204 (M⁺ +1)

### (Reference example 17)

### Synthesis of 3-hydroxy-4-phenylaminopyrrolidine hydrochloride

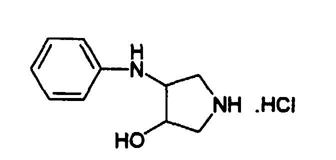

(1) 5 g (0.027 mol) of 1-t-butoxycarbonyl-3-epoxypyrrolidine was dissolved in 20 ml of ethyl alcohol, and 12.6 g (0.135 mol) of aniline was added thereto, followed by heating of the resulting mixture under reflux for 20 hours. The reaction mixture was cooled to room temperature and the solvent was evaporated under reduced pressure. The residue was subjected to silica gel column chromatography (eluting solution: mixture of ethyl acetate:toluene = 1:4) to obtain 4.85 g of 1-t-butoxycarbonyl-3-hydroxy-4-phenylaminopyrrolidine as yellow brown crystals.
   - MS spectrum (EI): m/e: 278 (M⁺)
   57 (C₄H₉⁺)
(2) 2.4 g (0.0086 mol) of the thus obtained 1-t-butoxycarbonyl-3-hydroxy-4-phenylaminopyrrolidine was dissolved in 100 ml of methanol, and 30 ml of 6N hydrochloric acid was added thereto, followed by heating of the resulting mixture under reflux for 2.5 hours. Then, the solvent was evaporated under reduced pressure. The residue was washed with ethanol and ether to obtain 1.52 g of 3-hydroxy-4-phenylaminopyrrolidine hydrochloride as a pale brown powder.
   MS spectrum (CI): m/e 179 (M⁺ +1)

### (Reference example 18)

### Synthesis of 3-methoxy-4-phenylaminopyrrolidine hydrochloride

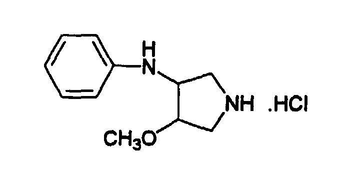

0.34 g (0.0085 mol) of 60% sodium hydride-mineral oil was added to 10 ml of anhydrous tetrahydrofuran, and 1.21 g (0.0085 mol) of methyl iodide was added thereto with stirring at 45 to 50°C. To the resulting mixture was added dropwise a solution of 2.37 g of 1-t-butoxycarbonyl-3-hydroxy-4-phenylaminopyrrolidine, which was obtained in (1) of Reference example 17, in 10 ml of anhydrous tetrahydrofuran, and the resulting mixture was further stirred at the same temperature for 1 hour. The reaction mixture was cooled to room temperature and the solvent was evaporated under reduced pressure. The residue was subjected to silica gel column chromatography (eluting solution: mixture of methyl acetate:toluene = 1:4) to obtain 1.98 g of 1-t-butoxycarbonyl-3-methoxy-4-phenylaminopyrrolidine as white crystals.
MS spectrum (EI): m/e 292 (M⁺)

1.98 g (0.0068 mol) of the thus obtained 1-t-butoxycarbonyl-3-methoxy-4-phenylaminopyrrolidine was dissolved in 100 ml of methanol, and 23.5 ml of 6N hydrochloric acid was added thereto. The resulting mixture was allowed to stand at room temperature overnight. The solvent was evaporated under reduced pressure and the residue was washed with ethanol to obtain 1.71 g of 3-methoxy-4-phenylaminopyrrolidine hydrochloride as a white powder.
MS spectrum (CI): m/e 193 (M⁺ +1)

### (Reference example 19)

### Synthesis of 1-(6-ethyl-4-pyrimidinyl)piperazine

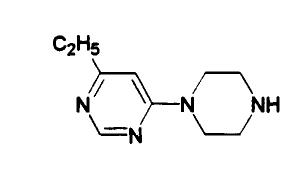

50 ml of 1,2-dichloroethane was added to 12.4 g (0.1 mol) of 6-ethyl-4-hydroxypyrimidine, and 18.4 g (0.12 mol) of phosphorus oxychloride was added thereto, and the resulting mixture was heated under reflux for 3 hours. After cooling, water was added to the reaction mixture, and the resulting mixture was extracted with chloroform. The chloroform layer was washed with water and dried over anhydrous sodium sulfate. The solvent was evaporated under reduced pressure to obtain 10.38 g of 4-chloro-6-ethylpyrimidine as a slightly red liquid.

Then, this was dissolved in 100 ml of acetonitrile, and 11.5 g (0.073 mol) of 1-ethoxycarbonylpiperazine, 20.2 g (0.146 mol) of potassium carbonate and a catalytic amount of potassium iodide were added thereto, followed by heating of the resulting mixture under reflux for 10 hours. The reaction mixture was cooled to room temperature and filtered. The filtrate was concentrated under reduced pressure and the residue was subjected to silica gel column chromatography (eluting solution: mixture of chloroform:methanol = 4:1) to obtain 20.69 g of 1-(6-ethyl-4-pyrimidinyl)-4-ethoxycarbonylpiperazine as an orange liquid.
MS spectrum (CI): m/e 265 (M⁺ +1)

To 20.69 g (0.78 mol) of the thus obtained 1-(6-ethyl-4-pyrimidinyl)-4-ethoxycarbonylpiperazine was added 200 ml of 6N hydrochloric acid, and the mixture was heated under reflux for 18 hours. Sodium hydroxide was added to the reaction mixture to adjust the pH thereof to 10 or higher, and the reaction mixture was extracted with diethyl ether. The organic layer was dried over anhydrous sodium sulfate and the solvent was evaporated under reduced pressure. The residue was subjected to silica gel column chromatography (eluting solution: mixture of chloroform:methanol = 4:1) to obtain 1-(6-ethyl-4-pyrimidinyl)piperazine as a colorless liquid.
MS spectrum (CI): m/e 193 (M⁺ +1)

### (Reference example 20)

### Synthesis of 9-ethyl-6,7-difluoro-8-difluoromethoxy-2,3,4,9-tetrahydrothiazolo[5,4-b]quinoline-3,4-dione

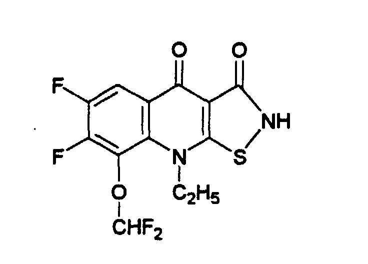

Reaction was carried out in a similar procedure to that described in Reference example 23 in Japanese Patent Application Kokai No. Hei 3-209367 to obtain the title compound as a pink powder.
m.p.: 211-213°C
MS spectrum (CI): m/e 349 (M⁺ +1)

### (Reference example 21)

### Synthesis of 1-cyclopropyl-6,7-difluoro-8-trifluoromethyl-1,4-dihydro-4-oxoquinoline-3-carboxylic acid

30 ml of benzene, 17 ml of thionyl chloride and a few drops of N,N-dimethylformamide were added to 8.5 g (0.0348 mol) of 2,4,5-trifluoro-3-trifluoromethylbenzoic acid, and the mixture was heated under reflux for 3 hours. After completion of the reaction, benzene and excess thionyl chloride were evaporated under reduced pressure to obtain 2,4,5-trifluoro-3-trifluoromethylbenzoic chloride.

After 5.47 g (0.0383 mol) of ethyl 3-dimethylaminoacrylate was dissolved in 30 ml of anhydrous tetrahydrofuran, 4.2 g (0.0415 mol) of triethylamine was added thereto, and a solution of the above-mentioned acid chloride in 7 ml of anhydrous tetrahydrofuran was gradually added dropwise to the resulting mixture at room temperature. After completion of the addition, the mixture was heated at 50°C for 3 hours and then cooled to room temperature, followed by filtration. 3.9 g (0.0417 mol) of cyclopropylamine hydrochloride was added to the filtrate, and the mixture was stirred at 40°C for 30 minutes. After the reaction mixture was cooled to room temperature, it was filtered, and the filtrate was concentrated under reduced pressure. The residue was subjected to silica gel column chromatography (eluting solution: mixture of ethyl acetate:toluene = 1:4) to obtain 10.63 g of ethyl 2-(2,4,5-trifluoro-3-trifluoromethylbenzoyl)-3-cyclopropylaminoacrylate as a pale yellow solid. This solid was dissolved in 100 ml of anhydrous diethyl ether, and 1.6 g (0.0416 mol) of 62.4% sodium hydride-mineral oil was gradually added thereto under ice-cooling, followed by stirring of the resulting mixture at room temperature for 1 hour. 41.7 ml of 1N hydrochloric acid was added to the reaction mixture and the resulting mixture was vigorously stirred to acidify the whole reaction mixture. The precipitate was collected by filtration and washed with water and then with diethyl ether to obtain 7.32 g of 1-cyclopropyl-6,7-difluoro-8-trifluoromethyl-1,4-dihydro-4-oxoquinoline-3-carboxylic acid ethyl ester as a white powder.
m.p.: 184-185°C
MS spectrum (CI): m/e 362 (M⁺ +1)

Then, 0.8 g (0.0022 mol) of this ester was suspended in a mixture of 5 ml of acetic acid, 3 ml of water and 0.3 ml of conc. sulfuric acid, and the suspension was heated under reflux with stirring for 2 hours. After the reaction mixture was cooled to room temperature, water was added to the mixture to collect precipitate by filtration. The precipitate was washed with water and distilled off water to obtain 0.7 g of 1-cyclopropyl-6,7-difluoro-8-trifluoromethyl-1,4-dihydro-4-oxoquinoline-3-carboxylic acid as white crystals.
m.p.: 210-212°C
MS spectrum (CI): m/e 334 (M⁺ +1)

### (Reference example 22)

### Synthesis of 1-ethyl-6,7-difluoro-8-trifluoromethyl-1,4-dihydro-4-oxoquinoline-3-carboxylic acid

Reaction was carried out in a similar manner to that described in Reference example 21 using ethylamine instead of cyclopropylamine hydrochloride to obtain 1-ethyl-6,7-difluoro-8-trifluoromethyl-1,4-dihydro-4-oxoquinoline-3-carboxylic acid as a white powder.
m.p.: 159-162°C
MS spectrum (CI): m/e 322 (M⁺ +1)

### (Reference example 23)

### Synthesis of 6,7-difluoro-8-trifluoromethyl-1,4-dihydro-1-methyl-4-oxoquinoline-3-carboxylic acid

Reaction was carried out in a similar manner to that described in Reference example 21 using methylamine hydrochloride instead of cyclopropylamine hydrochloride to obtain 6,7-difluoro-8-trifluoromethyl-1,4-dihydro-1-methyl-4-oxoquinoline-3-carboxylic acid as a white powder.
m.p.: 197.5-199°C
MS spectrum (CI): m/e 308 (M⁺ +1)

### (Reference example 24)

### Synthesis of 6,7-difluoro-8-trifluoromethyl-1,4-dihydro-1-isopropyl-4-oxoquinoline-3-carboxylic acid

Reaction was carried out in a same manner to that described in Reference example 21 using isopropylamine hydrochloride instead of cyclopropylamine hydrochloride to obtain 6,7-difluoro-8-trifluoromethyl-1,4-dihydro-1-isopropyl-4-oxoguinoline-3-carboxylic acid as a white powder.
m.p.: 197.5-200°C
MS spectrum (CI): m/e 336 (M⁺ +1)

### (Reference example 25)

### Synthesis of 6,7-difluoro-1-(2-fluoroethyl)-8-trifluoromethyl-1,4-dihydro-4-oxoquinoline-3-carboxylic acid

Reaction was carried out in a similar manner to that described in Reference example 21 using 2-fluoroethylamine hydrochloride instead of cyclopropylamine hydrochloride to obtain 6,7-difluoro-1-(2-fluoroethyl)-8-trifluoromethyl-1,4-dihydro-4-oxoquinoline-3-carboxylic acid as a white powder.
m.p.: 183-185°C
MS spectrum (CI): m/e 340 (M⁺ +1)

### (Reference example 26)

### Synthesis of 1-(2-pyrimidyl)homopiperazine

50 ml of acetonitrile was added to 10.0 g (0.1 mol) of homopiperazine, 2.9 g (0.025 mol) of 2-chloropyrimidine, 6.9 g (0.05 mol) of potassium carbonate and a catalytic amount of potassium iodide, and the mixture was heated under reflux for 11 hours. After the reaction mixture was cooled to room temperature, it was filtered, and the filtrate was concentrated under reduced pressure. The residue was subjected to silica gel column chromatography (eluting solution: mixture of chloroform:methanol = 8:2) to obtain 2.14 g of 1-(2-pyrimidyl)homopiperazine as a pale yellow liquid.
MS spectrum (CI): m/e 179 (M⁺ +1)

### (Reference example 27)

### Synthesis of 1-(2-methylthiophenyl)piperazine

40 ml of ethanol was added to 2.8 g (0.02 mol) of 2-methylthioaniline and 13.7 g (0.044 mol) of N-bis(2-bromoethyl) amine hydrobromide, and the mixture was heated under reflux for 10 hours. After the reaction mixture was cooled to room temperature, 10.2 g of potassium carbonate was added thereto, and the mixture was heated under reflux for 10 hours. After the reaction mixture was cooled to room temperature, it was filtered, and the filtrate was concentrated under reduced pressure. The residue was subjected to silica gel column chromatography (eluting solution: mixture of chloroform:methanol:28% aqueous ammonia = 40:9:1) to obtain 1.31 g of 1-(2-methylthiophenyl)piperazine as a pale yellow liquid.
MS spectrum (CI): m/e 209 (M⁺ +1)

### (Reference example 28)

### Synthesis of 1-(2-thiazolyl)piperazine

5.0 g (0.0305 mol) of 2-bromothiazole was dissolved in 50 ml of acetonitrile, and 13.1 g (0.153 mol) of piperazine, 8.4 g (0.061 mol) of potassium carbonate and a catalytic amount of potassium iodide were added thereto, followed by heating of the resulting mixture under reflux for 5 hours. After the reaction mixture was cooled to room temperature, it was filtered, and the filtrate was concentrated under reduced pressure. The residue was subjected to silica gel column chromatography (eluting solution: mixture of chloroform:methanol:28% aqueous ammonia = 40:9:1) to obtain 3.62 g of 1-(2-thiazolyl)piperazine as a colorless liquid.
MS spectrum (CI): m/e 170 (M⁺ +1)

### (Reference example 29)

### Synthesis of 1-bromo-3-difluoromethyl-2,4,5-trifluorobenzene

1.61 g (0.01 mol) of diethylamino sulfur trifluoride was added to 2.39 g (0.01 mol) of 1-bromo-2,4,5-trifluorobenzaldehyde which was cooled to 3°C. After the mixture was stirred at the same temperature for 30 minutes, the temperature of the mixture was carefully elevated to a temperature until exothermic reaction ceased.

When the exothermic reaction stopped, the temperature of the reaction mixture was elevated to 80°C and the mixture was stirred at the same temperature for 30 minutes. The reaction mixture was cooled to room temperature, and chloroform was added to the reaction mixture. The resulting mixture was washed with water and dried over anhydrous sodium sulfate, followed by evaporation under reduced pressure. The residue was subjected to silica gel column chromatography (eluting solvent: n-hexane) to obtain 1.47 g of 1-bromo-3-difluoromethyl-2,4,5-trifluorobenzene as a colorless liquid.
HS spectrum (CI): m/e 260 (M⁺ +1)

### (Reference example 30)

### Synthesis of 3-difluoromethyl-2,4,5-trifluorobenzoic acid

42.72 g (0.164 mol) of 1-bromo-3-difluoromethyl-2,4,5-trifluorobenzene was dissolved in 500 ml of anhydrous diethyl ether, and the mixture was cooled to -70°C. To this solution was added dropwise 108 ml (0.177 mol) of 1.63M n-butyl lithium n-hexane solution over 30 minutes, and the mixture was stirred at the same temperature for 5 minutes.

The above reaction mixture was added at once to a solution in which 1 kg of dry ice was added to 500 ml of anhydrous diethyl ether, and the mixture was stirred until the temperature of the mixture became room temperature. Then, 569 ml of 1N hydrochloric acid was added to the reaction mixture, and the ether layer was separated. The ether layer was extracted twice with 212 ml of 0.5N aqueous sodium hydroxide solution, and the pH of the aqueous layer was adjusted to 1 with 6N hydrochloric acid. The aquous layer was extracted twice with 500 ml of diethyl ether, the ether solution washed with a saturated aqueous NaCl solution and dried over anhydrous sodium sulfate, followed by evaporation under reduced pressure to obtain 34.0 g of 3-difluoromethyl-2,4,5-trifluorobenzoic acid as white crystals.
m.p.: 95-97°C
MS spectrum (CI): m/e 227 (M⁺ +1)

### (Reference example 31)

### Synthesis of 1-cyclopropyl-6,7-difluoro-8-difluoromethyl-1,4-dihydro-4-oxoquinoline-3-carboxylic acid

37.7 ml of benzene, 21.8 ml of thionyl chloride and a few drops of N,N-dimethylformamide were added to 10.0 g (0.0442 mol) of 2,4,5-trifluoro-3-difluoromethylbenzoic acid, and the mixture was heated under reflux for 3 hours. After completion of the reaction, benzene and excess thionyl chloride were evaporated under reduced pressure to obtain 2,4,5-trifluoro-3-difluoromethylbenzoic chloride.

6.95 g (0.0486 mol) of ethyl 3-dimethylaminoacrylate was dissolved in 38 ml of anhydrous tetrahydrofuran, and 5.37 g (0.0530 mol) of triethylamine was added to the solution, and to the resulting mixture was gradually added dropwise a solution of the above acid chloride in 8 ml of anhydrous tetrahydrofuran at room temperature. After completion of the addition, the mixture was heated at 50°C for 3 hours. After the reaction mixture was cooled to room temperature, it was filtered. 6.2 g (0.0663 mol) of cyclopropylamine hydrochloride was added to the filtrate, and the mixture was stirred at 40°C for 20 minutes. After the reaction mixture was cooled to room temperature, it was filtered, and the filtrate was concentrated under reduced pressure. The residue was subjected to silica gel column chromatography (eluting solution: mixture of ethyl acetate:toluene = 1:4) to obtain 14.27 g of ethyl 2-(2,4,5-trifluoro-3-difluoromethylbenzoyl)-3-cyclopropylaminoacrylate as a pale yellow solid. This solid was dissolved in 200 ml of anhydrous diethyl ether, and 2.36 g (0.059 mol) of 60.0% sodium hydride-mineral oil was gradually added to the solution under ice-cooling, followed by stirring of the resulting mixture at room temperature for 1 hour. 59 ml of 1N hydrochloric acid was added to the reaction mixture, and the mixture was vigorously stirred to acidify the whole reaction mixture. Precipitate was collected by filtration and washed with water and then with diethyl ether to obtain 11.40 g of 1-cyclopropyl-6,7-difluoro-8-difluoromethyl-1,4-dihydro-4-oxoquinoline-3-carboxylic acid ethyl ester as a white powder.
m.p.: 208-210°C
MS spectrum (CI): m/e 344 (M⁺ +1)

Then, 2.0 g (0.0058 mol) of this ester form was suspended in a mixture of 13.1 ml of acetic acid, 7.8 ml of water and 0.78 ml of conc. sulfuric acid, and the suspension was heated under reflux with stirring for 2 hours. After the reaction mixture was cooled to room temperature, water was added thereto to collect precipitate by filtration. The precipitate was washed with water and distilled off water to obtain 1.72 g of 1-cyclopropyl-6,7-difluoro-8-difluoromethyl-1,4-dihydro-4-oxoquinoline-3-carboxylic acid as white needle crystals.
m.p.: 190.5-192.5°C
MS spectrum (CI): m/e 315 (M⁺ +1)

### (Reference example 32)

### Synthesis of 1-(3-tert-butoxycarbonylaminopropyl)-6,7-difluoro-8-trifluoromethyl-1,4-dihydro-4-oxoquinoline-3-carboxylic acid ethyl ester

Reaction was carried out in a similar manner to that described in Reference example 1 using 2,4,5-trifluoro-3-trifluoromethylbenzoic acid and N-tert-butoxycarbonylpropylenediamine instead of 2,4,5-trifluoro-3-difluoromethoxybenzoic acid and 2-aminomethylpyridine, respectively, to obtain 1-(3-tert-butoxycarbonylaminopropyl)-6,7-difluoro-8-trifluoromethyl-1,4-dihydro-4-oxoquinoline-3-carboxylic acid ethyl ester as a white powder.
m.p.: 131-133°C

### Industrial applicability

By the combined use of the quinolone carboxylic acid derivative of the present invention and the transcriptase inhibitor, remarkable synergistic effects have been found with respect to the inhibition of proliferation of HIV. Therefore, the combination of the quinolone carboxylic acid derivative of the present invention and the transcriptase inhibitor is useful as an anit-HIV agent and a therapeutic agent of AIDS.

## Claims

1. The combined use of one kind or two or more kinds of a quinolone carboxylic acid having anti-HIV activity and one kind or two or more kinds of a reverse transcriptase inhibitor for the treatment or prevention of AIDS.

2. The combined use of one kind or two or more kinds of a quinolone carboxylic acid having anti-HIV activity and one kind or two or more kinds of an HIV protease inhibitor for the treatment or prevention of AIDS.

3. The combined use of one kind or two or more kinds of a quinolone carboxylic acid having anti-HIV activity, one kind or two or more kinds of a reverse transcriptase inhibitor and one kind or two or more kinds of an HIV protease inhibitor for the treatment or prevention of AIDS.

4. The combined use according to Claims 1 to 3 wherein the quinolone carboxylic acid having anti-HIV activity as an active ingredient is a quinolone carboxylic acid of formula (Ia), (Ib) or (Ic): 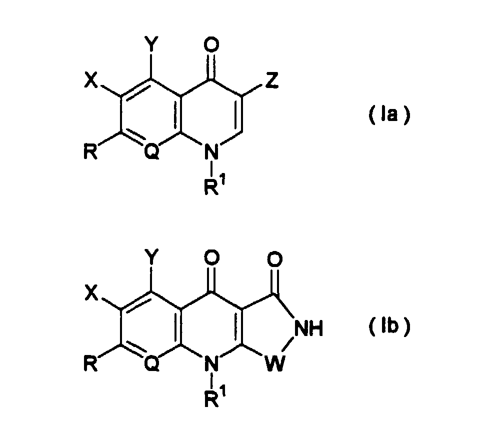 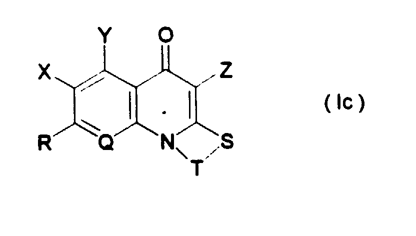 in the above formula (Ia), (Ib) or (Ic),
X represents a hydrogen atom or a halogen atom,
Y represents a hydrogen atom, a halogen atom, an alkyl group having from 1 to 4 carbon atoms, an amino group, a mono- or dialkylamino group which is substituted with one or two alkyl groups having from 1 to 4 carbon atoms, or a mono- or diaralkylamino group which is substituted with one or two aralkyl groups having from 7 to 14 carbon atoms,
Z represents an optionally protected carboxyl group or a 5-tetrazolyl group,
Q represents a nitrogen atom or a group of formula (d): 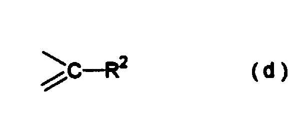 [wherein R² represents a hydrogen atom, a halogen atom, an alkyl group having from 1 to 4 carbon atoms which may be substituted with halogen, or an alkoxy group having from 1 to 4 carbon atoms which may be substituted with halogen],
W represents an oxygen atom or a sulfur atom,
T represents an alkylene group having from 1 to 4 carbon atoms which may be substituted with alkyl having from 1 to 4 carbon atoms or an alkenylene group having from 2 to 4 carbon atoms which may be substituted with alkyl having from 1 to 4 carbon atoms,
R¹ represents a hydrogen atom; an optionally substituted alkyl group having from 1 to 4 carbon atoms [the substituents are hydroxyl, carboxyl, halogen, alkoxy having from 1 to 4 carbon atoms, cycloalkyl having from 3 to 6 carbon atoms, alkanoyloxy having from 2 to 5 carbon atoms, a group of formula (e): 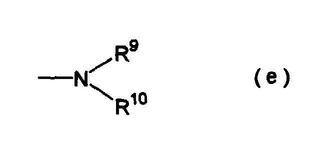 (wherein R⁹ and R¹⁰ each represents a hydrogen atom or an alkyl group having from 1 to 4 carbon atoms or R⁹ and R¹⁰ may together form a 3 to 7-membered saturated heteromonocyclic group with a nitrogen atom to which they bond, optionally containing a hetero atom selected from N, O and S), or an aryl group having from 6 to 10 carbon atoms which may be substituted with R⁰ as defined later, a 5- or 6-membered aromatic heteromonocyclic group containing one or two hetero atoms selected from N, O and S which may be substituted with R⁰ as defined later, or an aromatic heterocyclic fused-ring group in which a benzene ring is fused with the aromatic heteromonocyclic group which may be substituted with R⁰ as defined later]; an alkenyl group having from 2 to 5 carbon atoms which may be substituted with halogen; an alkynyl group having from 2 to 4 carbon atoms; an amino group; a mono- or dialkyl amino group substituted with one or two alkyl groups having from 1 to 4 carbon atoms; a cycloalkyl group having from 3 to 6 carbon atoms which may be substituted with halogen; an alkoxy group having from 1 to 4 carbon atoms; or an aryl group having from 6 to 10 carbon atoms which may be substituted with R⁰ as defined later, a 5- or 6-membered aromatic heteromonocyclic group containing one or two hetero atoms selected from N, O and S which may be substituted with R⁰ as defined later, or an aromatic heterocyclic fused-ring group in which a benzene ring is fused with the aromatic heteromonocyclic group which may be substituted with R⁰ as defined later, or
R¹ and R² in the formula (d) of Q together form a group of formula (f): 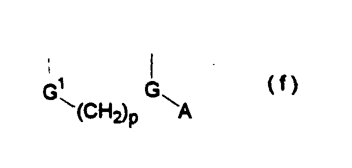 [wherein A represents a hydrogen atom or an alkyl group having from 1 to 4 carbon atoms which may be substituted with halogen, hydroxyl or alkoxy having from 1 to 4 carbon atoms, G represents a nitrogen atom or a group of formula (g): 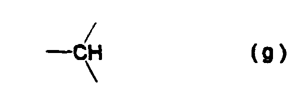 G¹ represents a methylene group, a carbonyl group, an oxygen atom, a sulfur atom or a group of -N(R¹¹)- (wherein R¹¹ represents a hydrogen atom or an alkyl group having from 1 to 4 carbon atoms) and p represents 0 or 1],
R represents a group of formula (h) or (i): 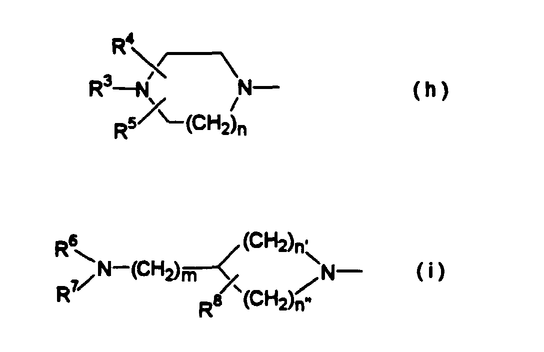 [wherein R³ and R⁶ each represents an aryl group having from 6 to 10 carbon atoms which may be substituted with R⁰ (R⁰ represents a group selected from halogen, nitro, hydroxy, alkyl having from 1 to 4 carbon atoms, alkyl having from 1 to 4 carbon atoms substituted with fluorine, alkoxy having from 1 to 4 carbon atoms, alkylthio having from 1 to 4 carbon atoms, amino and mono- or dialkylamino substituted with one or two alkyl groups having from 1 to 4 carbon atoms), a 5- or 6-membered aromatic heteromonocyclic group containing one or two hetero atoms selected from N, O and S which may be substituted with R⁰ as defined above, or an aromatic heterocyclic fused-ring group in which a benzene ring is fused with the aromatic heteromonocyclic group which may be substituted with R⁰ as defined above; R⁴, R⁵ and R⁷ each represent a hydrogen atom or an alkyl group having from 1 to 4 carbon atoms; R⁸ represents a hydrogen atom, a hydroxyl group, an alkyl group having from 1 to 4 carbon atoms or an alkoxy group having from 1 to 4 carbon atoms; n represents 1 or 2; m represents 0 or 1; n 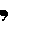 represents 1 or 2; and n 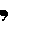 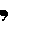 represents 1, 2, 3 or 4],
its pharmacologically acceptable salts or its esters.

5. The combined use according to Claims 1 to 3 wherein the quinolone carboxylic acid having anti-HIV activity as the active ingredient is the compound of the formula (Ia).

6. The combined use according to Claim 5 wherein the quinolone carboxylic acid having anti-HIV activity as the active ingredient is a compound of the formula (Ia)
wherein X is a fluorine atom,
Y is a hydrogen atom, a fluorine atom, an amino group, a methyl group or an ethyl group,
Z is an optionally protected carboxyl group,
Q is a group of the formula (d) and R² of the formula (d) is a methoxy, difluoromethoxy or trifluoromethyl group,
R¹ is a hydrogen atom; a methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group,
R is a 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group.

7. The combined use according to Claim 5 wherein the quinolone carboxylic acid having anti-HIV activity as the active ingredient is a compound of the formula (Ia)
wherein X is a fluorine atom,
Y is a hydrogen atom, a fluorine atom, an amino group, a methyl group or an ethyl group,
Z is an optionally protected carboxyl group,
Q is a group of the formula (d) and R² of the formula (d) is a methoxy, difluoromethoxy or trifluoromethyl group,
R¹ is a methyl, ethyl, 2-hydroxyethyl, 2-fluoroethyl, cyclopropyl or methylamino group,
R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group.

8. The combined use according to Claim 5 wherein the quinolone carboxylic acid having anti-HIV activity as the active ingredient is
1-cyclopropyl-6-fluoro-8-trifluoromethyl-1,4-dihydro-4-oxo-7-[4-(2-pyridyl)piperazin-1-yl]quinoline-3-carboxylic acid,
6-fluoro-8-trifluoromethyl-1,4-dihydro-4-oxo-1-methyl-7-[4-(2-pyrimidinyl)piperazin-1-yl]quinoline-3-carboxylic acid,
6-fluoro-8-trifluoromethyl-1,4-dihydro-4-oxo-1-methyl-7-[4-(2-pyridyl)piperazin-1-yl]quinoline-3-carboxylic acid,
6-fluoro-8-trifluoromethyl-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-ethyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(6-methoxy-2-benzothiazolyl)piperazin-1-yl]quinoline-3-carboxylic acid,
6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
6-fluoro-8-difluoromethoxy-1,4-dihydro-4-oxo-1-methylamino-7-[4-(2-benzoxazolyl)piperazin-1-yl]quinoline-3-carboxylic acid.

9. The combined use according to Claim 1 or 3 wherein the reverse transcriptase inhibitor as the active ingredient is ZDV, DDI, DDC, d4T, 3TC, FTC or nevirapine.

10. The combined use according to Claim 1 or 3 wherein the reverse transcriptase inhibitor as the active ingredient is ZDV, DDI and nevirapine.

11. The combined use according to Claim 1 or 3 wherein the reverse transcriptase inhibitor as the active ingredient is ZDV.

12. The combined use according to Claim 2 or 3 wherein the HIV protease inhibitor as the active ingredient is VX-478, KNI-272, AG-1343, saquinavir, ritonavir, indinavir and Compound A shown below:

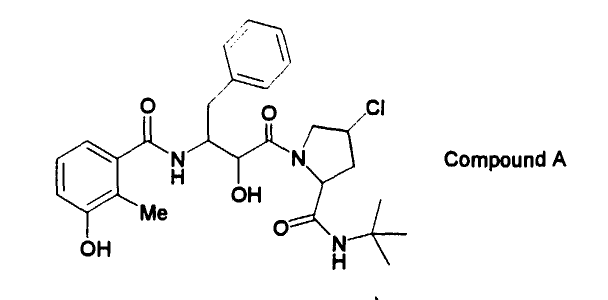

13. The combined use according to Claim 2 or 3 wherein the HIV protease inhibitor as the active ingredient is AG-1343, saquinavir, ritonavir or indinavir.

14. An AIDS therapeutic or preventive agent containing as its active ingredients one kind or two or more kinds of a quinolone carboxylic acid having anti-HIV activity and one kind or two or more kinds of a reverse transcriptase inhibitor.

15. An AIDS therapeutic or preventive agent containing as its active ingredients one kind or two or more kinds of a quinolone carboxylic acid having anti-HIV activity and one kind or two or more kinds of an HIV protease inhibitor.

16. An AIDS therapeutic or preventive agent containing as its active ingredients one kind or two or more kinds of a quinolone carboxylic acid having anti-HIV activity, one kind or two or more kinds of a reverse transcriptase inhibitor, and one kind or two or more kinds of an HIV protease inhibitor.

17. The AIDS therapeutic or preventive agent according to Claims 14 to 16 wherein the quinolone carboxylic acid having anti-HIV activity as an active ingredient is a quinolone carboxylic acid of formula (Ia), (Ib) or (Ic): 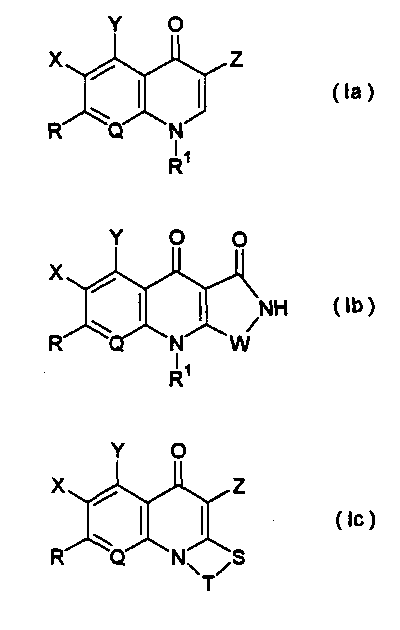 in the above formula (Ia), (Ib) or (Ic),
X represents a hydrogen atom or a halogen atom,
Y represents a hydrogen atom, a halogen atom, an alkyl group having from 1 to 4 carbon atoms, an amino group, a mono- or dialkylamino group which is substituted with one or two alkyl groups having from 1 to 4 carbon atoms, or a mono- or diaralkylamino group which is substituted with one or two aralkyl groups having from 7 to 14 carbon atoms,
Z represents an optionally protected carboxyl group or a 5-tetrazolyl group,
Q represents a nitrogen atom or a group of formula (d): 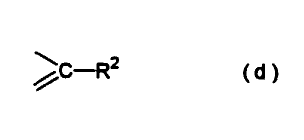 [wherein R² represents a hydrogen atom, a halogen atom, an alkyl group having from 1 to 4 carbon atoms which may be substituted with halogen, or an alkoxy group having from 1 to 4 carbon atoms which may be substituted with halogen],
W represents an oxygen atom or a sulfur atom,
T represents an alkylene group having from 1 to 4 carbon atoms which may be substituted with alkyl having from 1 to 4 carbon atoms or an alkenylene group having from 2 to 4 carbon atoms which may be substituted with alkyl having from 1 to 4 carbon atoms,
R¹ represents a hydrogen atom; an optionally substituted alkyl group having from 1 to 4 carbon atoms [the substituents are hydroxyl, carboxyl, halogen, alkoxy having from 1 to 4 carbon atoms, cycloalkyl having from 3 to 6 carbon atoms, alkanoyloxy having from 2 to 5 carbon atoms, a group of formula (e): 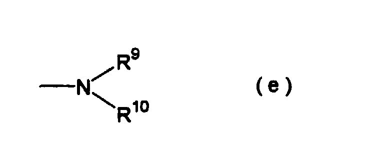 (wherein R⁹ and R¹⁰ each represents a hydrogen atom or an alkyl group having from 1 to 4 carbon atoms or R⁹ and R¹⁰ may together form a 3- to 7-membered saturated heteromonocyclic group with a nitrogen atom to which they bond, optionally containing a hetero atom selected from N, O and S), or an aryl group having from 6 to 10 carbon atoms which may be substituted with R⁰ as defined later, a 5- or 6-membered aromatic heteromonocyclic group containing one or two hetero atoms selected from N, O and S which may be substituted with R⁰ as defined later, or an aromatic heterocyclic fused-ring group in which a benzene ring is fused with the aromatic heteromonocyclic group which may be substituted with R⁰ as defined later; an alkenyl group having from 2 to 5 carbon atoms which may be substituted with halogen; an alkynyl group having from 2 to 4 carbon atoms; an amino group; a mono- or dialkylamino group substituted with one or two alkyl groups having from 1 to 4 carbon atoms; a cycloalkyl group having from 3 to 6 carbon atoms which may be substituted with halogen; an alkoxy group having from 1 to 4 carbon atoms; or an aryl group having from 6 to 10 carbon atoms which may be substituted with R⁰ as defined later, a 5- or 6-membered aromatic heteromonocyclic group containing one or two hetero atoms selected from N, O and S which may be substituted with R⁰ as defined later, or an aromatic heterocyclic fused-ring group in which a benzene ring is fused with the aromatic heteromonocyclic group which may be substituted with R⁰ as defined later, or
R¹ and R² in the formula (d) of Q together form a group of formula (f): 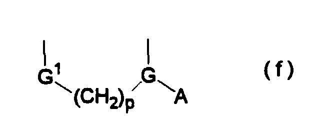 (wherein the formula (f), A represents a hydrogen atom or an alkyl group having from 1 to 4 carbon atoms which may be substituted with halogen, hydroxyl or alkoxy having from 1 to 4 carbon atoms, G represents a nitrogen atom or a group of formula (g): 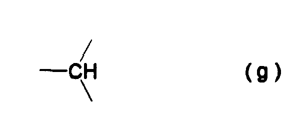 G¹ represents a methylene group, a carbonyl group, an oxygen atom, a sulfur atom or a group of formula -N(R¹¹)- (wherein R¹¹ represents a hydrogen atom or an alkyl group having from 1 to 4 carbon atoms) and p represents 0 or 1],
R represents a group of formula (h) or (i): 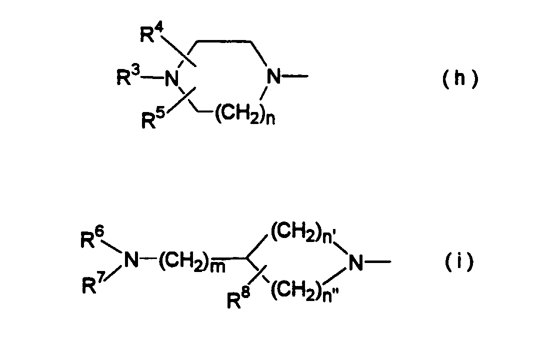 [wherein R³ and R⁶ each represents an aryl group having from 6 to 10 carbon atoms which may be substituted with R⁰ (R⁰ represents a group selected from halogen, nitro, hydroxy, alkyl having from 1 to 4 carbon atoms, alkyl having from 1 to 4 carbon atoms substituted with fluorine, alkoxy having 1 to 4 carbon atoms, alkylthio having from 1 to 4 carbon atoms, amino and mono- or dialkylamino substituted with one or two alkyl groups having from 1 to 4 carbon atoms), a 5- or 6-membered aromatic heteromonocyclic group containing one or two hetero atoms selected from N, O and S which may be substituted with R⁰ as defined above, or an aromatic heterocyclic fused-ring group in which a benzene ring is fused with the aromatic heterocyclic group which may be substituted with R⁰ as defined above; R⁴, R⁵ and R⁷ each represents a hydrogen atom or an alkyl group having from 1 to 4 carbon atoms; R⁸ represents a hydrogen atom, a hydroxyl group, an alkyl group having from 1 to 4 carbon atoms or an alkoxy group having from 1 to 4 carbon atoms; n represents 1 or 2; m represents 0 or 1; n 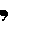 represents 1 or 2; and n 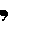 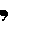 represents 1, 2, 3 or 4],
its pharmacologically acceptable salts or its esters.

18. The AIDS therapeutic or preventive agent according to Claims 14 to 16 wherein the quinolone carboxylic acid having anti-HIV activity as the active ingredient is the compound of the above-mentioned formula (Ia).

19. The AIDS therapeutic or preventive agent according to Claim 18 wherein the quinolone carboxylic acid having anti-HIV activity as the active ingredient is a compound of the formula (Ia)
wherein X is a fluorine atom,
Y is a hydrogen atom, a fluorine atom, an amino group, a methyl group or an ethyl group,
Z is an optionally protected carboxyl group,
Q is a group of the formula (d) and R² of the formula (d) is a methoxy, difluoromethoxy or trifluoromethyl group,
R¹ is a hydrogen atom; a methyl, ethyl, propyl, isopropyl; 2-hydroxyethyl; carboxymethyl; 2-fluoroethyl, 2-chloroethyl, 2,2,2-trifluoroethyl; 2-acetoxyethyl; phenylmethyl, phenylethyl; 2-pyridylmethyl; 2-dimethylaminoethyl, 2-morpholinoethyl; amino; methylamino; methoxy; cyclopropyl, cyclobutyl, cyclopentyl, 2-fluorocyclopropyl; phenyl, 2-fluorophenyl, 3-fluorophenyl, 4-fluorophenyl, 2,4-difluorophenyl; vinyl, 2-propenyl; or 2-propynyl group,
R is a 4-(2-pyrimidinyl)piperazin-1-yl, 4-(2-benzothiazolyl)piperazin-1-yl, 4-(2-benzoxazolyl)piperazin-1-yl, 4-(6-methoxy-2-benzoxazolyl)piperazin-1-yl, 4-(6-methoxy-2-benzothiazolyl)piperazin-1-yl or 4-(2-pyridyl)piperazin-1-yl group.

20. The AIDS therapeutic or preventive agent according to Claim 18 wherein the quinolone carboxylic acid having anti-HIV activity as the active ingredient is a compound of the formula (Ia)
wherein X is a fluorine atom,
Y is a hydrogen atom, a fluorine atom, an amino group, a methyl group or an ethyl group,
Z is an optionally protected carboxyl group,
Q is a group of the formula (d) and R² of the formula (d) is a methoxy, difluoromethoxy or trifluoromethyl group,
R¹ is a methyl, ethyl, 2-hydroxyethyl, 2-fluoroethyl, cyclopropyl or methylamino group,
R is a 4-(2-pyrimidinyl)piperazin-1-yl, 4-(2-benzothiazolyl)piperazin-1-yl, 4-(2-pyridyl)piperazin-1-yl, 4-(2-benzoxazolyl)piperazin-1-yl or 4-(6-methoxy-2-benzothiazolyl)piperazin-1-yl group.

21. The AIDS therapeutic agent or preventive agent according to Claim 18 wherein the quinolone carboxylic acid having anti-HIV activity as the active ingredient is
1-cyclopropyl-6-fluoro-8-trifluoromethyl-1,4-dihydro-4-oxo-7-[4-(2-pyridyl)piperazin-1-yl]quinoline-3-carboxylic acid,
6-fluoro-8-trifluoromethyl-1,4-dihydro-4-oxo-1-methyl-7-[4-(2-pyrimidinyl)piperazin-1-yl]quinoline-3-carboxylic acid,
6-fluoro-8-trifluoromethyl-1,4-dihydro-4-oxo-1-methyl-7-[4-(2-pyridyl)piperazin-1-yl]quinoline-3-carboxylic acid,
6-fluoro-8-trifluoromethyl-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-ethyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(2-benzothiazolyl)piperazin-1-yl]quinoline-3-carboxylic acid,
6-fluoro-8-difluoromethoxy-1,4-dihydro-4-oxo-1-methyl-7-[4-(6-methoxy-2-benzothiazolyl)piperazin-1-yl]quinoline-3-carboxylic acid,
6-fluoro-8-difluoromethoxy-1,4-dihydro-4-oxo-1-(2-hydroxyethyl)-7-[4-(2-benzothiazolyl)piperazin-1-yl]quinoline-3-carboxylic acid or
6-fluoro-8-difluoromethoxy-1,4-dihydro-4-oxo-1-methylamino-7-[4-(2-benzoxazolyl)piperazin-1-yl]quinoline-3-carboxylic acid.

22. The AIDS therapeutic or preventive agent according to Claim 14 or 16 wherein the reverse transcriptase inhibitor as the active ingredient is ZDV, DDI, DDC, d4T, 3TC, FTC or nevirapine.

23. The AIDS therapeutic or preventive agent according to Claim 14 or 16 wherein the reverse transcriptase inhibitor as the active ingredient is ZDV, DDI and nevirapine.

24. The AIDS therapeutic or preventive agent according to Claim 14 or 16 wherein the reverse transcriptase inhibitor as the active ingredient is ZDV.

25. The AIDS therapeutic or preventive agent according to Claim 15 or 16 wherein the HIV protease inhibitor as the active ingredient is VX-478, KNI-272, AG-1343, saquinavir, ritonavir, indinavir and Compound A shown below:

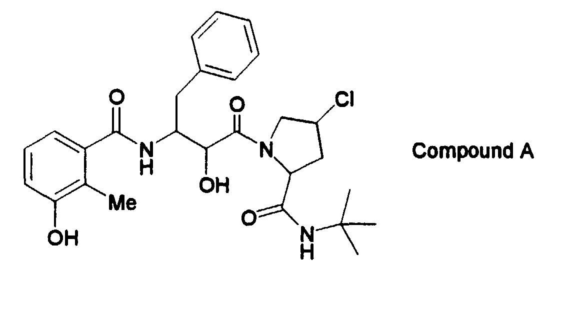

26. The AIDS therapeutic or preventive agent according to Claim 15 to 16 wherein the HIV protease inhibitor as the active ingredient is AG-1343, saquinavir, ritonavir or indinavir.
